(19) Europäisches Patentamt
European Patent Office
Office européen des brevets

(11) **EP 3 858 864 A1**

(12) # EUROPEAN PATENT APPLICATION

(43) Date of publication:
**04.08.2021 Bulletin 2021/31**

(21) Application number: **21159557.4**

(22) Date of filing: **06.01.2017**

(51) Int Cl.:
*C07K 16/28* (2006.01)
*C07K 14/705* (2006.01)
*C07K 14/725* (2006.01)
*A61K 39/395* (2006.01)
*C12N 5/22* (2006.01)
*C07K 19/00* (2006.01)
*A61P 35/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.01.2016 GB 201600328**

(62) Document number(s) of the earlier application(s) in accordance with Art. 76 EPC:
**17700624.4 / 3 400 244**

(71) Applicant: **Oslo Universitetssykehus HF**
**0424 Oslo (NO)**

(72) Inventors:
- **SMELAND, Erlend**
  **0778 Oslo (NO)**
- **WÄLCHLI, Sébastien**
  **0369 Oslo (NO)**
- **KVALHEIM, Gunnar**
  **0381 Oslo (NO)**
- **HOLTE, Harald**
  **0779 Oslo (NO)**
- **MYKLEBUST, June Helen**
  **1386 Asker (NO)**
- **FUNDERUD, Steinar**
  **0756 Oslo (NO)**
- **INDERBERG, Else Marit**
  **0276 Oslo (NO)**

(74) Representative: **Dehns**
**St. Bride's House**
**10 Salisbury Square**
**London EC4Y 8JD (GB)**

Remarks:
This application was filed on 26.02.2021 as a divisional application to the application mentioned under INID code 62.

(54) **ANTI-CD37 CHIMERIC ANTIGEN RECEPTORS AND IMMUNE CELLS EXPRESSING THEM**

(57) The present invention relates to nucleic acid molecules encoding chimeric antigen receptors (CARs) against the antigen CD37. The CARs disclosed herein have complementarity-determining regions (CDRs) derived from the potent monoclonal anti-CD37 antibody HH1, and may be used in immunotherapy to target cells expressing CD37. Such immunotherapy has a particular use in the treatment of B-cell cancers. The CARs of the present invention are highly functional in the redirection of immune cells to kill CD37+ cells, and include humanised CARs of particular use in medical therapy.

The present invention also includes vectors comprising the above-described nucleic acid molecules, immune effector cells expressing the aforementioned CARs and the use of such immune effector cells in therapy, particularly adoptive transfer therapy, for cancer, including B-cell malignancies.

Figure 1:

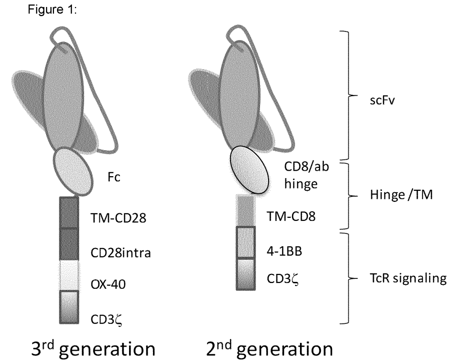


Figure 2:

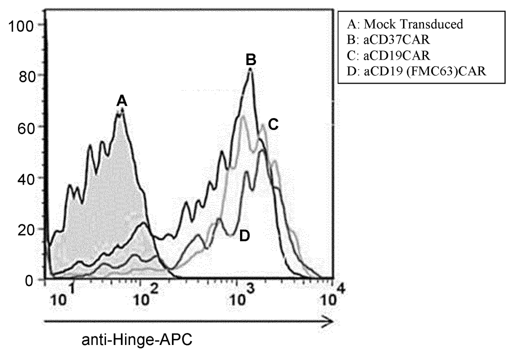


EP 3 858 864 A1

## Description

This invention relates to chimeric antigen receptors (CARs) against the antigen CD37 and their expression in immune effector cells to target cells expressing CD37, and particularly the use of such immune cells in treating B-cell cancers. The invention provides nucleic acid molecules encoding such CARs and vectors containing them which may be used to modify immune effector cells to express the CAR. In particular, the CARs of the invention comprise an antigen-binding domain derived from a particular antibody, the antibody HH1.

Immunotherapy using antibodies, particularly monoclonal antibodies, has emerged in recent years as a safe and selective method for treating cancer and other diseases. Various extracellular cancer antigens have been identified but antibodies developed against a number of antigens expressed on the surface of B-cells, e.g. CD19, CD20 and CD22, have particularly been successful in the treatment of B-cell malignancies. More recently the antigen CD37 has been identified as an attractive B-cell target antigen, including but not only in patients not responding to anti-CD20 (Rituximab) therapy. A very potent murine monoclonal anti-CD37 antibody has been isolated, antibody HH1 (Smeland et al., Scand. J. Immunol. 1985, 21(3), p205-14). A modified version of this antibody radiolabelled with Lu177 (Belutin™; see also WO 2011/092295) is presently undergoing phase 1 clinical trials for the treatment of non-Hodgkin lymphoma (NHL). Chimeric and humanized antibodies based on antibody HH1 are described in WO 2013/088363.

As well as antibody therapies, cell-based cancer therapies have also been developed using cytotoxic immune effector cells to target and kill cancer cells (adoptive cell transfer therapy, ACT). Whilst tumour-infiltrating $CD8^+$ T-lymphocytes (TILs) may be isolated from a patient, expanded and re-introduced into the patient to target and trigger an immune response against the tumour, it has been found that T-cell redirection, in which the patient's own T-cells are modified to express T-cell receptors (TcRs) against selected target cancer antigens (which may be identified from particularly effective TILs) is a more promising approach. However, the utility of this approach is limited by the need to match the TcR introduced into the T-cell to a patient's immune type, as well as by the availability of suitable TcRs.

Accordingly, as an alternative to the use of TcRs, therapies involving the expression of Chimeric Antigen Receptors (CARs) in T-cells or other immune effector cells, e.g. NK cells, have also been suggested and developed. CARs, now widely known and described in the art, are fusion proteins comprising an antigen-binding domain, typically but not always derived from an antibody, linked to the signalling domain of the TcR complex (or equivalent), and can be used to direct T-cells or other immune effector cells against a tumour if a suitable antigen-binding domain or antibody is selected. Unlike a TcR, a CAR does not need to be MHC-matched to the recipient.

Although CARs are now a well-known and practiced technology and the use of immune cells expressing CARs represents an attractive and promising approach to cancer therapy, the design of an appropriate CAR is not always straightforward. In particular, with regard to the antigen-binding (antigen recognition) domain of the CAR, it cannot be predicted that a particular domain shown to have antigen binding activity in one particular context (e.g. in an antibody) will be effective when used in the context of a CAR (e.g. will be able to bind to the target antigen). Furthermore, the antigen-binding domain of a CAR is typically based on an scFv (single chain variable fragment) and not all antibodies make effective scFvs. A CAR construct generally comprises an antigen-binding domain, a hinge domain, which functions as a spacer to extend the antigen-binding domain away from the plasma membrane of the immune effector cell on which it is expressed, a transmembrane (TM) domain, an intracellular signalling domain (e.g. the signalling domain from the zeta chain of the CD3 molecule (CD3ζ) of the TcR complex, or an equivalent) and optionally one or more co-stimulatory domains which may assist in signalling or functionality of the cell expressing the CAR. The different domains may be linked directly or by linkers. A variety of options are available for these different domains and linkers, and the selection of different domains, and/or the combination in which they are used, may affect the efficacy or functionality of the CAR when expressed on the surface of a cell, and its ability to bind to and/or be effective against (e.g. cytotoxic to) a target cell. Accordingly, not all CARs are effective, or equally effective, and the efficacy of a CAR directed against a particular antigen (e.g. comprising a particular antigen-binding domain, or derived from a particular antibody or scFv) may be dependent upon the precise domains, or combination of domains, used, or on the precise nature of the construct.

The present inventors have found that an effective CAR for use in adoptive cell transfer therapy against cells expressing CD37 may be based on the specific antibody HH1, and more particularly on the variable region ($V_L$ and $V_H$ chains) of this antibody and specifically on the hypervariable regions or CDRs (complementarity determining regions) thereof. As will be described in more detail below, in more particular embodiments the CAR may comprise an antigen-binding domain based on, or comprising, the $V_L$ and $V_H$ chains of the HH1 antibody, in combination with a particular "signalling tail" comprising specific combinations of hinge, transmembrane, co-stimulatory and intracellular signalling domains.

Accordingly, in a first aspect, the present invention provides a nucleic acid molecule encoding a chimeric antigen receptor (CAR) directed against the antigen CD37, wherein said CAR when expressed on the surface of an immune effector cell is capable of binding to the antigen CD37 expressed on a target cell surface and comprises an antigen-binding domain comprising a $V_L$ sequence and a $V_H$ sequence each comprising three CDR sequences, wherein

a) CDRs 1, 2 and 3 of the $V_L$ sequence have the sequences of SEQ ID NOs. 43, 44 and 35 respectively; and
b) CDRs 1, 2 and 3 of the $V_H$ sequence have the sequences of SEQ ID NOs. 45, 46 and 34 respectively, and

wherein one or more of said CDR sequences may optionally be modified by substitution, addition or deletion of 1 to 3 amino acids.

The CDR sequences are, or correspond to, the CDR sequences contained in the $V_L$ and $V_H$ sequences of SEQ ID NOs. 3 and 1 respectively. SEQ ID NOs. 3 and 1 represent the amino acid sequences of the $V_L$ and $V_H$ regions of antibody HH1, respectively (SEQ ID NOs. 4 and 2, respectively, represent the nucleotide sequences encoding said amino acid sequences). SEQ ID NOs. 43, 44 and 35 correspond respectively to CDRs 1, 2 and 3 lying at positions 27-32, 50-52 and 89-97 of SEQ ID NO. 3, and SEQ ID NOs. 45, 46 and 34 correspond respectively to CDRs 1, 2 and 3 lying at positions 26-33, 51-58 and 97-108 of SEQ ID NO. 1.

In a preferred embodiment CDR3 at least of the $V_L$ and $V_H$ sequences is unmodified and preferably all of the CDRs are unmodified (i.e. have the amino acid sequences of SEQ ID NOs. 43, 44 and 35 ($V_L$) and 45, 46 and 34 ($V_H$) respectively.

More particularly, the CAR when expressed on the surface of an immune effector cell is capable of directing the immune effector cell against a target cell expressing CD37. In other words, the immune cell is capable of directing its effect or function, e.g. its cytotoxic activity, against a said target cell, particularly a target cancer cell, e.g. a malignant B-cell.

As is known in the art, and is described further below, the $V_L$ and $V_H$ chains of an antibody each comprise 3 CDRs separated by framework regions which act as a scaffold for the CDRs. Thus, the $V_L$ and $V_H$ sequences of a CAR of the invention comprise the CDR sequences of the $V_L$ and $V_H$ sequences of the HH1 antibody separated by framework regions. The framework regions may be those of the $V_L$ and $V_H$ chains of the HH1 antibody, but need not be. Thus, the framework regions of the $V_L$ and $V_H$ chains of the HH1 antibody may be modified, which includes that they may be substituted (thus the amino acid sequence of the framework regions may be modified and/or substituted), e.g. they may be humanised, as described in more detail below. In one particular embodiment, the invention provides a nucleic acid molecule encoding a chimeric antigen receptor (CAR) directed against the antigen CD37, wherein said CAR when expressed on the surface of an immune effector cell is capable of binding to the antigen CD37 expressed on a target cell surface and comprises an antigen-binding domain comprising the $V_L$ sequence of SEQ ID NO. 3 or an amino acid sequence having at least 95 % sequence identity thereto, and the $V_H$ sequence of SEQ ID NO. 1 or an amino acid sequence having at least 95 % sequence identity thereto.

In other embodiments, the framework regions of the $V_L$ and $V_H$ sequences are modified, and the CAR may comprise an antigen-binding domain comprising a $V_L$ sequence having an amino acid sequence as shown in SEQ ID NO. 3, or an amino acid sequence having at least 60 % sequence identity thereto, and a $V_H$ sequence having an amino acid sequence as shown in SEQ ID NO.1, or an amino acid sequence having at least 60 % sequence identity thereto, preferably with the proviso that the CDR sequences of SEQ ID NOs.43, 44, 35, 45, 46 and 34 are retained (i.e. are not modified or altered).

It will be understood, therefore, that in such embodiments, the CDR sequences of the HH1 antibody are retained or substantially retained (i.e. they may optionally be modified within the constraints set out above, namely substitution, addition or deletion of 1 to 3 amino acids, such that the binding specificity of the HH1 antibody is retained (e.g. unaltered).

The antigen-binding domain is extracellular (i.e. when the CAR is expressed on an immune effector cell). The CAR thus comprises an extracellular domain comprising an antigen-binding domain comprising the HH1-based $V_L$ and $V_H$ sequences as defined above. As will be described in more detail below, the extracellular domain may also comprise a signal sequence, more particularly a plasma membrane targeting sequence, and especially a plasma membrane targeting sequence based on the L-chain and having or comprising SEQ ID NO. 6, or an amino acid sequence with at least 95 % sequence identity thereto.

The nucleic acid molecule of the invention may be used to prepare immune effector cells (more particularly modified immune effector cells) directed against cells expressing CD37. Such (modified) immune effector cells express the CAR on their cell surface and are capable of recognising, or binding to, a target cell expressing CD37, e.g. a B-cell and in particular a cancerous or malignant B-cell or B-cell tumour. Accordingly, the nucleic acid molecule is such that an immune effector cell expressing said CAR (i.e. the CAR encoded by the nucleic acid molecule) is capable of effector activity (e.g. cytotoxic activity) against (e.g. killing) a target cell expressing CD37. A modified immune effector cell is accordingly a genetically modified or engineered immune effector cell, or alternatively expressed an immune effector cell which has been transduced with a nucleic acid molecule of the invention.

The nucleic acid molecule may be introduced into an immune effector cell as mRNA or as DNA for expression in the cell. Vectors may be used to transfer the nucleic acid molecule into the cell or to produce the nucleic acid for transfer (e.g. to produce mRNA for transfer, or to produce a nucleic acid molecule for preparation of an expression vector for transfer into a cell).

Accordingly, a further aspect of the invention provides a vector comprising the nucleic acid molecule of the invention as defined herein.

The vector may for example be an mRNA expression vector, a cloning vector or an expression vector for transfer into an immune cell e.g. a viral vector.

Another aspect of the invention provides an immune effector cell comprising a nucleic acid molecule or vector of the invention as defined herein.

In preferred embodiments the immune effector cell may be a T-cell or an NK cell.

Also provided is a method of generating a CD37-specific immune effector cell, said method comprising introducing a nucleic acid molecule or vector of the invention as defined herein into an immune effector cell.

Such a method may comprise stimulating the cell and inducing it to proliferate before and/or after introducing the nucleic acid molecule or vector.

As noted above, immune effector cells of the invention have a utility in therapy. Accordingly, further aspects of the invention include:

a composition, particularly a therapeutic or pharmaceutical composition, comprising the immune effector cell of the invention as defined herein and at least one physiologically acceptable carrier or excipient;

an immune effector cell or a composition of the invention as defined herein for use in therapy, particularly adoptive cell transfer therapy;

an immune effector cell or a composition of the invention as defined herein for use in the treatment of cancer, particularly for the treatment of a B-cell malignancy;

a method of treating cancer, particularly a B-cell malignancy, said method comprising administering to a subject in need thereof an immune effector cell or a composition of the invention as defined herein, particularly an effective amount of said cell or composition; and

use of the immune effector cell of the invention as defined herein for the manufacture of a medicament (or composition) for use in cancer therapy, particularly for treating a B-cell malignancy.

In the method of generating a CD37-specific immune effector cell, the immune effector cell which is modified by introduction of the nucleic acid molecule of the invention may be obtained from a subject to be treated (e.g. a subject with a B-cell malignancy). After modification of the immune effector cell, and optionally *in vitro* expansion, the modified immune effector cells expressing the CAR may be re-introduced (i.e. administered) to the subject. Thus, autologous immune effector cells may be used in the therapeutic methods and uses of the invention. Alternatively, heterologous (i.e. donor or allogeneic, or syngeneic or xenogeneic) immune effector cells may be used.

An immune effector cell may be any immune cell capable of an immune response against a target cell expressing CD37. More particularly, the immune effector cell is capable of abrogating, damaging or deleting a target cell, i.e. of reducing, or inhibiting, the viability of a target cell, preferably killing a target cell (in other words rendering a target cell less or non-viable). The immune effector cell is thus preferably a cytotoxic immune effector cell.

The term "cytotoxic" is synonymous with "cytolytic" and is used herein to refer to a cell capable of inducing cell death by lysis or apoptosis in a target cell.

The term "immune effector cell" as used herein includes not only mature or fully differentiated immune effector cells but also precursor (or progenitor) cells therefor, including stem cells (more particularly haemopoietic stem cells, HSC), or cells derived from HSC. An immune effector cell may accordingly be a T-cell, NK cell, NKT cell, neutrophil, macrophage, or a cell derived from HSCs contained within the $CD34^+$ population of cells derived from a haemopoietic tissue, e.g. from bone marrow, cord blood, or blood e.g. mobilised peripheral blood, which upon administration to a subject differentiate into mature immune effector cells. As will be described in more detail below, in preferred embodiments, the immune effector cell is a T-cell or an NK cell. Primary cells, e.g. cells isolated from a subject to be treated or from a donor subject may be used, optionally with an intervening cell culture step (e.g. to expand the cells) or other cultured cells or cell lines (e.g. NK cell lines such as the NK92 cell line).

The term "directed against the antigen CD37" is synonymous with "specific for CD37" or "anti-CD37", that is it means simply that the CAR is capable of binding specifically to CD37. In particular, the antigen-binding domain of the CAR is capable of binding specifically to CD37 (more particularly when the CAR is expressed on the surface of an immune effector cell). Specific binding may be distinguished from nonspecific binding to a non-target antigen (in this case an antigen other than CD37). Thus, an immune effector cell expressing the CAR according to the present invention is redirected to bind specifically to and exhibit cytotoxicity to (e.g. kill) a CD37-expressing target cell. Alternatively expressed, the immune effector cell is modified to redirect cytotoxicity towards target cells expressing CD37.

In an embodiment, specific binding to CD37 may mean that the antigen-binding domain (or CAR comprising the antigen-binding domain) binds to or associates with CD37 (or more particularly a target cell expressing CD37 on its cell surface) with an affinity or $K_a$ (i.e. equilibrium association constant) of greater than or equal to about $10^5$ $M^{-1}$, e.g. at least $10^6$ $M^{-1}$, $10^7$ $M^{-1}$, or $10^8$ $M^{-1}$.

The binding of the antigen-binding domain of the CAR to its target antigen on the surface of the target cell delivers an activation stimulus to the CAR-containing cell, resulting in induction of effector cell signalling pathways. Binding to target antigen may thereby trigger proliferation, cytokine production, phagocytosis, lytic activity and/or production of molecules that can mediate cell death of the target cell in an MHC-independent manner. Although CARs comprising an intracellular domain comprising solely a signalling domain from CD3ζ or FcRγ may deliver a potent signal for immune cell activation and effector function they may not be sufficient to elicit signals that promote immune effector cell survival and expansion in the absence of a concomitant co-stimulatory signal. Accordingly, it may be preferred for the CAR to contain one or more co-stimulatory signalling domains.

A CAR of the invention thus generally comprises 4, or preferably 5, domains as follows:

(1) an antigen-binding domain, capable of binding specifically to CD37, that comprises VL and VH sequences based or derived from SEQ ID NOs. 3 and 1 as defined above;
(2) a hinge domain that extends the antigen-binding domain away from the surface of the immune effector cell;
(3) a transmembrane domain that anchors the CAR to the effector cell and links the extracellular domain comprising the antigen-binding domain to the intracellular signalling domain;
(4) an intracellular domain comprising a signalling domain; and optionally or preferably;
(5) one or more co-stimulatory signalling domains.

The CAR may further comprise (6) a signal sequence (i.e. a targeting domain), and in particular a sequence which targets the CAR to the plasma membrane of the immune effector cell. This will generally be positioned next to or close to the antigen-binding domain, generally upstream of the antigen-binding domain, at the end of the CAR molecule/construct

It can thus be seen that the CAR may comprise an extracellular domain comprising the antigen-binding domain and signal sequence, if present, linked via a hinge domain and transmembrane domain to an intracellular domain which comprises one or more signalling domains. In one aspect, the intracellular domain, or the hinge, transmembrane and intracellular domains, may be viewed as a "signalling tail" in the CAR construct. The order of the domains in the CAR construct is thus, N-terminal to C-terminal: extracellular domain - hinge domain - transmembrane domain - intracellular domain. Within the extracellular and intracellular domains the separate domains may be arranged in any order. Preferably however the order is signal sequence - antigen-binding domain in the extracellular domain. In one embodiment, in the intracellular domain the order may be co-stimulatory domain(s) - intracellular signalling domain(s). In another embodiment, the order may be intracellular signalling domain(s) - co-stimulatory domain(s).

In the CAR of the present invention the "antigen-binding domain", which is derived from the variable region sequences of the antibody HH1, may be provided in various formats, as long as it comprises the $V_L$ and $V_H$ sequences as defined above. It may accordingly be, or may correspond to, a natural or synthetic antibody sequence. Accordingly, the nucleotide sequence encoding the antigen-binding domain in the nucleic acid molecules of the invention may be derived from, or may correspond to, a natural sequence or may encode a genetically engineered product. Thus the antigen-binding domain may be (or more precisely may correspond to) a fragment of antibody HH1 comprising the variable region (the antibody light chain and heavy chain variable regions; the $V_L$ and $V_H$ regions), e.g. a Fv or Fab or $F(ab')_2$ or the light and heavy chain variable regions can be joined together in a single chain and in either orientation (e.g. $V_L$-$V_H$ or $V_H$-$V_L$). The $V_L$ and/or $V_H$ sequences may be modified, as discussed above. In particular the framework regions may be modified (e.g. substituted, for example to humanise the antigen-binding domain).

In a preferred embodiment, the binding domain is a single chain antibody (scFv) derived from antibody HH1. The single chain antibody may be cloned using known and readily available techniques from the V region genes of the hybridoma producing antibody HH1. The hybridoma is described in Smeland *et al.* 1985 (supra). As mentioned above the $V_L$ and/or $V_H$ sequences of the scFv may be modified.

"Single-chain Fv antibody" or "scFv" refers to an engineered antibody consisting of a light chain variable region ($V_L$) and a heavy chain variable region ($V_H$) connected to one another directly or via a peptide linker sequence.

"Heavy chain variable region" or "$V_H$" refers to the fragment of the heavy chain of an antibody that contains three CDRs (complementarity determining regions) interposed between flanking stretches known as framework regions, which are more highly conserved than the CDRs and form a scaffold to support the CDRs.

"Light chain variable region" or "$V_L$" refers to the fragment of the light chain of an antibody that contains three CDRs interposed between framework regions.

"Fv" refers to the smallest fragment of an antibody to bear the complete antigen-binding site. An Fv fragment consists of the variable region of a single light chain bound to the variable region of a single heavy chain.

In one preferred embodiment the $V_L$ and $V_H$ are linked together by a linker sequence. More precisely this may be referred to as a "variable region linker sequence", which is an amino acid sequence that connects a heavy chain variable region to a light chain variable region and provides a spacer function compatible with interaction of the two sub-binding domains so that the resulting polypeptide retains a specific binding affinity to the same target molecule as an antibody that comprises the same light and heavy chain variable regions. The linker sequence may be used to provide for appropriate spacing and conformation of the molecule.

Thus, in one embodiment the scFv comprises the $V_L$ sequence of SEQ ID NO. 3 or an amino acid sequence having at least 95 % sequence identity thereto linked to the $V_H$ sequence of SEQ ID NO. 1 or an amino acid sequence having at least 95 % sequence identity thereto, preferably in the order $V_L$-$V_H$.

In another embodiment the scFv comprises the $V_L$ sequence of SEQ ID NO. 3 or an amino acid sequence having at least 60 % sequence identity thereto linked to the $V_H$ sequence of SEQ ID NO.1 or an amino acid sequence having at least 60 % sequence identity thereto, preferably in the order $V_L$-$V_H$. As noted above, this is subject to the proviso that the CDR sequences remain as defined above, and preferably to the proviso that the CDR sequences are unaltered.

More preferably, the $V_L$ sequence is linked to $V_H$ by a linker sequence. The linker sequence may be between 1-30, more preferably 1-25, 1-22 or 1-20, amino acids long. The linker may be a flexible linker. Suitable linkers can be readily selected and can be of any of a suitable length, such as from 1 amino acid (e.g. Gly) to 20 amino acids, from 2 amino acids to 15 amino acids, from 3 amino acids to 12 amino acids, including 4 amino acids to 10 amino acids, 5 amino acids to 9 amino acids, 6 amino acids to 8 amino acids, or 7 amino acids to 8 amino acids, and may be 1, 2, 3, 4, 5, 6, or 7 amino acids or longer.

Exemplary flexible linkers include glycine polymers $(G)_n$, glycine-serine polymers, where n is an integer of at least one, glycine-alanine polymers, alanine-serine polymers, and other flexible linkers known in the art. Glycine and glycine-serine polymers are relatively unstructured, and therefore may be able to serve as a neutral tether between domains of fusion proteins such as the CARs described herein.

In a representative embodiment the linker sequence may be $(G4S)_4$ (SEQ ID NO. 5). Thus in a representative embodiment the nucleic acid molecule of the invention may comprise a nucleotide sequence encoding the amino acid sequence of SEQ ID NO. 16, comprising in order the $V_L$ of SEQ ID NO. 3, the linker of SEQ ID NO. 5 and the $V_H$ of SEQ ID NO. 1, or a sequence having at least 95 % sequence identity thereto.

The $V_L$ and $V_H$ regions may be encoded by nucleotide sequences comprising the nucleotide sequences of SEQ ID NOs. 4 and 2 respectively, nucleotide sequences having at least 95 % nucleotide sequence identity thereto, or nucleotide sequences degenerate with SEQ ID NOs. 4 and 2.

In another embodiment The $V_L$ and $V_H$ regions may be encoded by nucleotide sequences comprising the nucleotide sequences of SEQ ID NOs. 4 and 2 respectively, or nucleotide sequences having at least 60 % nucleotide sequence identity thereto. As above, this is subject this is subject to the proviso that the CDR sequences encoded by the nucleotide sequences remain as defined above, and preferably to the proviso that the CDR sequences are unaltered.

The $V_L$ and $V_H$ sequences may, if desired, be humanised by modifying one or more of the framework regions to correspond to at least one human framework region. A "human framework region" refers to a wild type (i.e. naturally occurring) framework region of a human immunoglobulin variable region, an altered framework region of a human immunoglobulin variable region with less than about 50 % (e.g. preferably less than about 45 %, 40 %, 30 %, 25 %, 20 %, 15 %, 10 %, 5 %, or 1 %) of the amino acids in the region deleted or substituted (e.g. with one or more amino acid residues of a nonhuman immunoglobulin framework region at corresponding positions), or an altered framework region of a nonhuman immunoglobulin variable region with less than about 50 % (e.g. less than 45 %, 40 %, 30 %, 25 %, 20 %, 15 %, 10 %, or 5 %) of the amino acids in the region deleted or substituted (e.g. at positions of exposed residues and/or with one or more amino acid residues of a human immunoglobulin framework region at corresponding positions) so that, in one aspect, immunogenicity is reduced.

Thus, in a particular embodiment, the framework regions of the $V_L$ and $V_H$ sequences of SEQ ID NOs. 3 and 1 respectively may be modified (more specifically the amino acid sequences of the framework regions may be modified), whilst retaining, or substantially retaining, the amino acid sequences of the CDRs.

Accordingly, in another embodiment, the framework regions of the $V_L$ and $V_H$ sequences in the CAR have at least 60 % amino acid sequence identity to the framework regions of SEQ ID NOs. 3 and 1 respectively.

The framework regions (FRs) of the $V_L$ sequence of SEQ ID NO. 3 lie at the following amino acid positions: FR1 at positions 1-26; FR2 at positions 33-49, FR3 at positions 53-98 and FR4 at positions 98-107.

The framework regions of the $V_H$ sequence of SEQ ID NO. 1 lie at the following amino acid positions: FR1 at positions 1-25; FR2 at positions 34-50, FR3 at positions 59-96 and FR4 at positions 109-119.

SEQ ID NO. 47 shows the amino acid sequence of a modified $V_L$ sequence having human framework regions and CDRs 1, 2 and 3 of SEQ ID NO. 3. SEQ ID NO. 48 shows the amino acid sequence of a modified $V_H$ sequence having human framework regions and CDRs 1, 2 and 3 of SEQ ID NO. 1. The corresponding nucleotide sequences encoding SEQ ID NOs. 47 and 48 are shown in SEQ ID NOs. 49 and 50.

Amino acid sequences SEQ ID NOs. 47 and/or 48, or nucleotide sequences SEQ ID NOs. 49 and/or 50, or sequences having at least 95 % sequence identity thereto, or nucleotide sequences degenerate with SEQ ID NOs. 49 and/or 50, may be used (i.e. contained or comprised) in the CARs or nucleic acid molecules of the invention.

As noted above, the CAR, and more particularly the extracellular domain thereof, may also comprise a signal sequence (or targeting domain). Such a sequence will generally be provided at the N-terminal end of the molecule (construct) and may function to, co-translationally or post-translationally, direct transfer of the molecule. In particular, the signal sequence may be a sequence which targets the CAR to the plasma membrane of the immune effector cell. This may be linked directly or indirectly (e.g. via a linker sequence) to the antigen-binding domain, generally upstream of the antigen-binding domain, at the N-terminal end of the CAR molecule/construct. The linker sequence may be a linker as described in connection with the variable region linker above. In one embodiment the signal sequence is linked directly to the N-terminal end of the antigen-binding domain, e.g. to the N-terminal end of the $V_L$ sequence (for example SEQ ID NO. 3). In one preferred embodiment the signal sequence is, or is derived from, the L-chain having the sequence of SEQ ID NO. 6 or an amino acid sequence having at least 95 % sequence identity thereto.

Accordingly, in a representative embodiment, the nucleic acid molecule encodes (or comprises a nucleotide sequence encoding) a CAR comprising an extracellular domain which comprises or has the sequence of SEQ ID NO. 7 or an amino acid sequence with at least 95 % sequence identity thereto, wherein SEQ ID NO. 7 comprises in order the L-chain of SEQ ID NO. 6, the $V_L$ of SEQ ID NO. 3, the linker of SEQ ID NO. 5 and the $V_H$ of SEQ ID NO. 1.

An extracellular domain (or a scFv domain) having or comprising an amino acid sequence as set out in SEQ ID NO. 7, or an amino acid sequence having at least 95 % sequence identity thereto, may be encoded by a nucleic acid molecule having or comprising a nucleotide sequence as set out in SEQ ID NO. 31 or 38, or a nucleotide sequence having at least 95 % sequence identity thereto, or a nucleotide sequence degenerate with SEQ ID NO. 31 or 38.

In a representative embodiment in which the $V_L$ and $V_H$ sequences are humanised, the nucleic acid molecule encodes (or comprises a nucleotide sequence encoding) a CAR comprising an extracellular domain (or a scFv domain) which comprises or has the sequence of SEQ ID NO. 53 or an amino acid sequence with at least 95 % sequence identity thereto, wherein SEQ ID NO. 53 comprises in order the L-chain of SEQ ID NO. 6, the $V_L$ of SEQ ID NO. 47, the linker of SEQ ID NO. 5 and the $V_H$ of SEQ ID NO. 48. An extracellular domain having or comprising an amino acid sequence as set out in SEQ ID NO. 53, or an amino acid sequence having at least 95 % sequence identity thereto, may be encoded by a nucleic acid molecule having or comprising a nucleotide sequence as set out in SEQ ID NO. 54, or a nucleotide sequence having at least 95 % sequence identity thereto, or a nucleotide sequence degenerate with SEQ ID NO. 54.

The antigen-binding domain of the CAR is generally followed by a hinge domain. The hinge region in a CAR is generally between the transmembrane domain and the antigen-binding domain. In certain embodiments, a hinge region is an immunoglobulin hinge region and may be a wild type immunoglobulin hinge region or an altered wild type immunoglobulin hinge region, for example a truncated hinge region. Other exemplary hinge regions which may be used include the hinge region derived from the extracellular regions of type 1 membrane proteins such as CD8$\alpha$, CD4, CD28 and CD7, which may be wild-type hinge regions from these molecules or may be altered. Preferably the hinge region is, or is derived from, the hinge region of human CD8$\alpha$, CD4, CD28 or CD7.

An "altered wild type hinge region" or "altered hinge region" refers to (a) a wild type hinge region with up to 30 % amino acid changes (e.g. up to 25 %, 20 %, 15 %, 10 %, or 5 % amino acid changes e.g. substitutions or deletions), (b) a portion of a wild type hinge region that is at least 10 amino acids (e.g. at least 12, 13, 14 or 15 amino acids) in length with up to 30 % amino acid changes (e.g. up to 25 %, 20 %, 15 %, 10 %, or 5 % amino acid changes, e.g. substitutions or deletions), or (c) a portion of a wild type hinge region that comprises the core hinge region (which may be 4, 5, 6, 7, 8, 9, 10, 11, 12, 13, 14, or 15, or at least 4, 5, 6, 7, 8, 9, 10, 11, 12, 13, 14, or 15 amino acids in length). When an altered wild type hinge region is interposed between and connecting the CD37-specific binding domain and another region (e.g. a transmembrane domain) in the chimeric antigen receptors described herein, it allows the chimeric fusion protein to maintain specific binding to CD37.

In certain embodiments, one or more cysteine residues in a wild type immunoglobulin hinge region may be substituted by one or more other amino acid residues (e.g. one or more serine residues). An altered immunoglobulin hinge region may alternatively or additionally have a proline residue of a wild type immunoglobulin hinge region substituted by another amino acid residue (e.g. a serine residue).

Hinge regions comprising the CH2 and CH3 constant region domains are described in the art for use in CARs (for example the CH2CH3 hinge, referred to as an "Fc hinge" or "IgG hinge", as shown in SEQ ID NO. 40). However, it is preferred that when the hinge domain is based on or derived from an immunoglobulin it does not comprise a CH3 domain, e.g. it may comprise or consist of the CH2 domain or a fragment or part thereof, without including CH3.

In one preferred embodiment the hinge domain has or comprises the amino acid sequence of SEQ ID NO. 9 (which represents the hinge domain of CD8$\alpha$) or an amino acid sequence having at least 95 % sequence identity thereto.

In another preferred embodiment the hinge domain has or comprises the amino acid sequence of SEQ ID NO. 10 (which represents a shortened IgG hinge) or an amino acid sequence having at least 95 % sequence identity thereto.

The hinge domain may be attached to the transmembrane domain by a linker sequence, which may be a linker sequence as defined above. An exemplary linker sequence is KDPK (SEQ ID NO. 11). A shortened IgG hinge with linker sequence is shown in SEQ ID NO. 21. Such a sequence, or a sequence having at least 95 % sequence identity thereto, may be included in a CAR of the present invention. More particularly such a sequence may be included between the extracellular domain (e.g. the scFv part) and the transmembrane domain.

The transmembrane domain may be based on or derived from the transmembrane domain of any transmembrane protein. Typically it may be, or may be derived from, a transmembrane domain from CD8α, CD28, CD4, CD3ζ CD45, CD9, CD16, CD22, CD33, CD64, CD80, CD86, CD134, CD137, and CD154, preferably from a human said protein. In one embodiment, the transmembrane domain may be, or may be derived from, a transmembrane domain from CD8α, CD28, CD4, or CD3ζ, preferably from human CD28, CD4, or CD3ζ. In another embodiment the transmembrane domain may be synthetic in which case it would comprise predominantly hydrophobic residues such as leucine and valine.

In a preferred embodiment the transmembrane domain is the CD8α transmembrane domain having the amino acid sequence of SEQ ID NO. 12 or an amino acid sequence having at least 95 % sequence identity thereto.

In another embodiment the transmembrane domain may be the transmembrane domain of human CD28 having the amino acid sequence of SEQ ID NO. 17 or an amino acid sequence having at least 95 % sequence identity thereto.

The "intracellular signalling domain" refers to the part of the CAR protein that participates in transducing the message of effective CAR binding to a target antigen into the interior of the immune effector cell to elicit effector cell function, e.g. activation, cytokine production, proliferation and cytotoxic activity, including the release of cytotoxic factors to the CAR-bound target cell, or other cellular responses elicited with antigen binding to the extracellular CAR domain. The term "effector function" refers to a specialized function of the cell. Effector function of the T-cell, for example, may be cytolytic activity or help or activity including the secretion of a cytokine. Thus, the term "intracellular signalling domain" refers to the portion of a protein which transduces the effector function signal and that directs the cell to perform a specialized function. While the entire intracellular signalling domain can be employed, in many cases it is not necessary to use the entire domain. To the extent that a truncated portion of an intracellular signalling domain is used, such truncated portion may be used in place of the entire domain as long as it transduces the effector function signal. The term intracellular signalling domain is meant to include any truncated portion of the intracellular signalling domain sufficient to transduce effector function signal. The intracellular signalling domain is also known as the, "signal transduction domain," and is typically derived from portions of the human CD3ζ or FcRy chains.

Additionally, to allow or to augment full activation of the immune effector cell the CAR may be provided with a secondary, or co-stimulatory domain. Thus, the intracellular signalling domain may initiate antigen dependent primary activation (i.e. may be a primary cytoplasmic signalling sequence) and the co-stimulatory domain may act in an antigen-independent manner to provide a secondary or co-stimulatory signal (secondary cytoplasmic signalling sequence(s)). Primary cytoplasmic signalling sequences may regulate primary activation, including in an inhibitory way. Primary cytoplasmic signalling sequences that act in a co-stimulatory manner may contain signalling motifs which are known as immunoreceptor tyrosine-based activation motifs or ITAMs.

Examples of ITAM-containing primary cytoplasmic signalling sequences that may be used in the invention include those derived from TCRζ, FcRy, FcRβ, CD3γ, CD3δ, CD3ε, CD5, CD22, CD79a, CD79b and CD66d. In certain particular embodiments, the intracellular signalling domain is derived from CD3ζ or FcRy, preferably human CD3ζ or FcRy.

In a preferred representative embodiment the intracellular signalling domain is preferably a human CD3ζ domain, more preferably a human CD3ζ domain having the amino acid sequence of SEQ ID NO. 8 or an amino acid sequence having at least 95% sequence identity thereto.

The term "co-stimulatory signalling domain" or "co-stimulatory domain", refers to the portion of the CAR comprising the intracellular domain of a co-stimulatory molecule. Co-stimulatory molecules are cell surface molecules other than antigen receptors or Fc receptors that provide a second signal required for efficient activation and function of an immune effector cell (e.g. a T-cell) upon binding to antigen. Examples of such co-stimulatory molecules include CD27, CD28, 4- IBB (CD137), OX40 (CD134), CD30, CD40, PD-1, ICOS (CD278), LFA-1 , CD2, CD7, LIGHT, NKD2C, B7-H2 and a ligand that specifically binds CD83, more particularly the intracellular domains of such molecules. Preferably the molecules are human. Accordingly, while exemplary or preferred co-stimulatory domains are derived from 4-1BB, CD28 or OX40 (CD134), other co-stimulatory domains are contemplated for use with the CARs described herein. The co-stimulatory domains may be used singly or in combination (i.e. one or more co-stimulatory domains may be included. The inclusion of one or more co-stimulatory signalling domains may enhance the efficacy and expansion of immune effector cells expressing the CARs.

The intracellular signalling and co-stimulatory signalling domains may be linked in any order in tandem to the carboxyl terminus of the transmembrane domain.

In a preferred embodiment the co-stimulatory domain is the intracellular domain of 4-1BB having the amino acid sequence of SEQ ID NO. 13 or an amino acid sequence having at least 95 % sequence identity thereto.

In another embodiment the co-stimulatory domain may be, or may include, the intracellular domain of human CD28 having the amino acid sequence of SEQ ID NO. 18 or an amino acid sequence having at least 95 % sequence identity thereto and/or an OX40 (CD134) co-stimulatory domain having the amino acid sequence of SEQ ID NO. 19 or an amino acid sequence having at least 95 % sequence identity thereto.

In a preferred embodiment of the invention, the CAR (or more particularly the "signalling tail" thereof) comprises a hinge domain from CD8α or a truncated IgG hinge domain not including the CH3 domain, a CD8α transmembrane domain, a 4-1BB co-stimulatory domain and a CD3ζ intracellular signalling domain.

In other embodiments the CAR (or the "signalling tail" thereof) comprises a hinge domain from CD8α or a truncated IgG hinge domain not including the CH3 domain, a CD28 transmembrane domain, a CD28 intracellular domain and/or OX40 co-stimulatory domain and a CD3ζ intracellular signalling domain.

More particularly, the CAR comprises, preferably in the following order:

(i) a hinge domain being (a) the hinge domain of CD8α having the amino acid sequence of SEQ ID NO. 9 or an amino acid sequence having at least 95 % sequence identity thereto; or (b) an IgG hinge domain having the amino acid sequence of SEQ ID NO. 10 or an amino acid sequence having at least 95 % sequence identity thereto;
(ii) a CD8α transmembrane domain having the amino acid sequence of SEQ ID NO. 12 or an amino acid sequence having at least 95 % sequence identity thereto;
(iii) a co-stimulatory domain being the intracellular domain of 4-1BB having the amino acid sequence of SEQ ID NO. 13 or an amino acid sequence having at least 95 % sequence identity thereto; and
(iv) a human CD3ζ domain having the amino acid sequence of SEQ ID NO. 8 or an amino acid sequence having at least 95 % sequence identity thereto.

Further, in the CAR the hinge domain may be attached to the transmembrane domain by means of a linker having the sequence KDPK (SEQ ID NO. 11). In particular embodiments the CAR may comprise the hinge-linker sequence of SEQ ID NO. 21 or an amino acid sequence having at least 95 % sequence identity thereto.

In an alternative embodiment, the CAR (or the signalling tail thereof) may comprise, preferably in the following order:

(i) a hinge domain being (a) the hinge domain of CD8α having the amino acid sequence of SEQ ID NO. 9 or an amino acid sequence having at least 95 % sequence identity thereto; or (b) an IgG hinge domain having the amino acid sequence of SEQ ID NO. 10 or an amino acid sequence having at least 95 % sequence identity thereto;
(ii) a CD28 transmembrane domain having the amino acid sequence of SEQ ID NO. 17 or an amino acid sequence having at least 95 % sequence identity thereto;
(iii) a co-stimulatory domain being the intracellular domain of CD28 (CD28 intra) having the amino acid sequence of SEQ ID NO. 18 or an amino acid sequence having at least 95 % sequence identity thereto and/or a co-stimulatory domain being an OX40 co-stimulatory domain having the amino acid sequence of SEQ ID NO. 19 or an amino acid sequence having at least 95 % sequence identity thereto;
(iv) a human CD3ζ domain having the amino acid sequence of SEQ ID NO. 8 or an amino acid sequence having at least 95 % sequence identity thereto.

Further in the CAR the hinge domain may be attached to the transmembrane domain by means of a linker having the sequence KDPK (SEQ ID NO. 11). In particular embodiments the CAR may comprise the hinge-linker sequence of SEQ ID NO. 21 or an amino acid sequence having at least 95 % sequence identity thereto. Where both CD28intra and OX40 co-stimulatory domains are present, they may be present in either order, but in one preferred embodiment they are present in the order CD28intra - OX40.

Such a CAR according to the invention may include a scFv antigen-binding domain as defined above and may further comprises a plasma membrane targeting sequence having the sequence set out in SEQ ID NO. 6 or an amino acid sequence having at least 95 % sequence identity thereto positioned upstream of the scFv.

Thus the CAR of the invention may in certain representative embodiments comprise, in addition to a signalling tail as defined above, an extracellular domain having the sequence of SEQ ID NO. 16 or 7 or a sequence having at least 95 % sequence identity thereto.

A representative CAR according to the present invention may thus have or comprise the amino acid sequence of SEQ ID NO. 32 or 33, or an amino acid having at least 95 % sequence identity thereto.

A nucleic acid molecule of the invention may comprise the nucleotide sequence of SEQ ID NO. 14 or SEQ ID NO. 15 or a nucleotide sequence having at least 95 % sequence identity thereto, or a nucleotide sequence degenerate with SEQ ID NO. 14 or SEQ ID NO. 15.

The present disclosure provides CAR polypeptides, and fragments thereof. The terms "polypeptide" and "protein" are used interchangeably and mean a polymer of amino acids not limited to any particular length. The term does not exclude modifications such as myristylation, sulfation, glycosylation, phosphorylation and addition or deletion of signal sequences. The terms "polypeptide" or "protein" or "peptide" mean one or more chains of amino acids, wherein each chain comprises amino acids covalently linked by peptide bonds, and wherein said polypeptide or protein can comprise a plurality of chains non-covalently and/or covalently linked together by peptide bonds, having the sequence of native proteins, that is, proteins produced by naturally-occurring and specifically non-recombinant cells, or genetically-engineered or recombinant cells, and comprise molecules having the amino acid sequence of the native protein, or molecules having deletions from, additions to, and/or substitutions of one or more amino acids of the native sequence. The terms "polypeptide" and "protein" specifically encompass the CARs of the present disclosure, or sequences that have deletions from, additions to, and/or substitutions of one or more amino acid of a CAR as disclosed herein.

As is clear from the above, the various domains of the CAR may comprise one or more amino acid sequence modifications with respect to the native sequences of the molecules from which they are derived. For example, it may be desirable to improve the binding affinity and/or other biological properties of the CAR. For example, amino acid sequence variants of a CAR, or binding domain, or a stimulatory signalling domain thereof, may be prepared by introducing appropriate nucleotide changes into a polynucleotide that encodes the CAR, or a domain thereof. Such modifications include, for example, deletions from, and/or insertions into and/or substitutions of, residues within the amino acid sequences of the CAR. Any combination of deletion, insertion, and substitution may be made to arrive at the final CAR, provided that the final construct possesses the desired characteristics, such as specific binding to CD37 by the binding domain, or increased signalling by the intracellular signalling domain and/or co-stimulatory domain. The amino acid changes also may alter post-translational processes of the CAR, such as changing the number or position of glycosylation sites. Any of the variations and modifications described above may be included in the CARs of the present invention.

In particular embodiments, the various domains of a CAR (other than the $V_L$ and $V_H$ sequences) may have an amino acid sequence that is at least 80 % identical, at least 85 %, at least 90 %, at least 95 % identical, or at least 98 % or 99 % identical, to the native sequence of the domains of the proteins from which they are derived. Thus, in particular embodiments the domains may have an amino acid sequence that has at least 80, 85, 90, 95, 98 or 99 % sequence identity to any of SEQ ID NOs. 5, 8, 9, 10, 11, 12, 13, 17, 18, 19 or 21.

Alternatively or additionally, the $V_L$ and $V_H$ sequences of a CAR, or the framework regions thereof, may have an amino acid sequence that is at least 60, 65 or 70 % identical to SEQ ID NOs. 3 and 1 respectively, or to the framework regions thereof, particularly with respect to SEQ ID NO. 3. In other embodiments the % sequence identity may be higher, e.g. at least 75, 80, 85, 90, 95, 98 or 99 % sequence identity to SEQ ID NOs. 3 or 1, or to the framework regions thereof.

The nucleic acid molecule of the invention may be an isolated nucleic acid molecule and may further include DNA or RNA or chemical derivatives of DNA or RNA, including molecules having a radioactive isotope or a chemical adduct such as a fluorophore, chromophore or biotin ("label"). Thus the nucleic acid may comprise modified nucleotides. Said modifications include base modifications such as bromouridine, ribose modifications such as arabinoside and 2',3'-dideoxyribose and internucleotide linkage modifications such as phosphorothioate, phosphorodithioate, phosphoroselenoate, phosphorodiselenoate, phosphoroanilothioate, phoshoraniladate and phosphoroamidate. The term "nucleic acid molecule" specifically includes single and double stranded forms of DNA and RNA.

In particular embodiments, nucleotide sequences in the nucleic acid molecule encoding the various domains of a CAR (other than the $V_L$ and $V_H$ sequences) may have a nucleotide sequence having at least 75 %, 80 %, 85 %, 90 %, 95 %, 96 %, 97 %, 98 %, or 99 % or higher, sequence identity, to the native sequence of the nucleic acids from which they are derived or to a reference polynucleotide sequence such as a sequence encoding a said domain as described herein.

Alternatively or additionally, the nucleotide sequences encoding the $V_L$ and $V_H$ sequences of a CAR, or the framework regions thereof, may have a nucleic acid sequence that is at least 60, 65 or 70 % identical to SEQ ID NOs. 4 and 2 respectively, or to the framework regions thereof, particularly with respect to SEQ ID NO. 4. In other embodiments the % sequence identity may be higher, e.g. at least 75, 80, 85, 90, 95, 98 or 99 % sequence identity to SEQ ID NOs. 4 or 2, or to the framework regions thereof.

Methods for determining sequence identity are well known in the art and any convenient or available method may be used, e.g. BLAST analysis, e.g. using standard or default parameters.

When comparing polynucleotide sequences, two sequences are said to be "identical" if the sequence of nucleotides in the two sequences is the same when aligned for maximum correspondence. Comparisons between two sequences are typically performed by comparing the sequences over a comparison window to identify and compare local regions of sequence similarity. A "comparison window" refers to a segment of at least about 20 contiguous positions, usually 30 to about 75, 40 to about 50, or more in which a sequence may be compared to a reference sequence of the same number of contiguous positions after the two sequences are optimally aligned. In particular, where % sequence identity is given herein with respect to a particular sequence, the % sequence identity is determined over the whole length of the specified sequence.

Optimal alignment of sequences for comparison may be conducted using the Megalign program in the Lasergene suite of bioinformatics software (DNASTAR, Inc., Madison, WI), using default parameters. Alternatively, optimal alignment of sequences for comparison may be conducted by the local identity algorithm of Smith and Waterman, Add. APL. Math 2:482 (1981), by the identity alignment algorithm of Needleman and Wunsch, J. Mol. Biol. 48:443 (1970), by the search for similarity methods of Pearson and Lipman, Proc. Natl. Acad. Sci. USA 85: 2444 (1988), by computerized implementations of these algorithms (GAP, BESTFIT, BLAST, FASTA, and TFASTA in the Wisconsin Genetics Software Package, Genetics Computer Group (GCG), 575 Science Dr., Madison, WI), or by inspection.

One representative example of algorithms that are suitable for determining percent sequence identity and sequence similarity are the BLAST and BLAST 2.0 algorithms, which are described in Altschul et al., Nucl. Acids Res. 25:3389-3402 (1977), and Altschul et al, J. Mol. Biol. 215:403-410 (1990), respectively. BLAST and BLAST 2.0 can be used, for example with the default parameters, to determine percent sequence identity among two or more nucleic acid molecules. Software for performing BLAST analyses is publicly available through the National Center for Biotechnology Information.

It will be appreciated by those of ordinary skill in the art that, as a result of the degeneracy of the genetic code, there are many nucleotide sequences that may encode a CAR as described herein. By degenerate nucleotide sequences is meant two (or more) nucleotide sequences which encode the same protein (or protein sequence), specifically in the open reading frame of the reference nucleotide sequence which begins at position 1 (i.e. in which codon 1 of the encoding sequence corresponds to positions 1-3 of the reference nucleotide sequence). Thus for example a nucleotide sequence degenerate with SEQ ID NO. 2 is a nucleotide sequence which is different to SEQ ID NO.2 but which, due to the degeneracy of the genetic code, encodes the same protein sequence as SEQ ID NO. 2, i.e. the protein sequence of SEQ ID NO. 1.

Methods for modifying nucleotide sequences to introduce changes to the amino acid sequences of the various domains are well known in the art e.g. methods of mutagenesis, such as site-specific mutagenesis, may be employed.

Likewise methods for preparing a nucleic acid molecule encoding the CAR are also well known e.g. conventional polymerase chain reaction (PCR) cloning techniques can be used to construct the nucleic acid molecule. The nucleic acid molecule can be cloned into a general purpose cloning vector such as pENT (Gateway), pUC19, pBR322, pBluescript vectors (Stratagene Inc.) or pCR TOPO® from Invitrogen Inc. The resultant nucleic acid construct (recombinant vector) carrying the nucleic acid molecule encoding the CAR can then be sub-cloned into expression vectors or viral vectors for protein expression, e.g. in mammalian cells. This may be for preparation of the CAR protein, or for expression in immune effector cells, e.g. in human T-cells or in NK cells or cell lines. Further the nucleic acid may be introduced into mRNA expression vectors for production of mRNA encoding the CAR. The mRNA may then be transferred into immune effector cells.

Thus, the nucleic acid molecule may be introduced into a cell in a vector or as a nucleic acid molecule or recombinant construct. Methods of heterologous gene expression are known in the art, both in terms of construct/vector preparation and in terms of introducing the nucleic acid molecule (vector or construct) into the cell. Thus, promoters and/or other expression control sequences suitable for use with mammalian cells, in particular immune effector cells (e.g. lymphoid cells or NK cells), and appropriate vectors etc. (e.g. viral vectors) are well known in the art.

Vectors or constructs (nucleic acid molecules) may be introduced into a cell of the invention by a variety of means, including chemical transfection agents (such as calcium phosphate, branched organic compounds, liposomes or cationic polymers), electroporation, cell squeezing, sonoporation, optical transfection, hydrodynamic delivery, or viral transduction. In a preferred embodiment, a vector or construct is introduced by viral transduction. This may allow for more persistent expression of the CAR. However, in some situations, e.g. in clinical trials, or in some clinical situations, it may be desirable to have a more transient period of expression of CAR protein. In such a situation it may be desirable to deliver the nucleic acid molecule to the immune effector cell as mRNA. mRNA expression vectors for production of mRNA may be prepared according to methods known in the art (e.g. using Gateway Technology) and are known in the art (e.g. pClpA102, Sæbøe-Larssen et al, 2002, J. Immunol. Methods 259, p 191-203 and pClpA120-G, Wälchli et al, 2011, PLoS ONE 6 (11) e27930).

The mRNA can be produced *in vitro* by e.g. *in vitro* transcription. The mRNA may then be introduced into the immune effector cells, e.g. as naked mRNA, e.g. by electroporation (as described for example in Almasbak et al., Cytotherapy 2011, 13, 629-640, Rabinovich et al., Hum. Gene Ther., 2009, 20, 51-60 and Beatty et al., Cancer Immunol. Res. 2014, 2, 112-120. Alternatively, mRNA may be introduced by other means such as by liposomes or cationic molecules etc. Heterologous nucleic acid molecules introduced into a cell may be expressed episomally, or may be integrated into the genome of the cell at a suitable locus.

Thus the nucleic acid molecule may be introduced or inserted into a vector. The term "vector" as used herein refers to a vehicle into which the nucleic acid molecule may be introduced (e.g. be covalently inserted) so as to bring about the expression of the CAR protein or mRNA and/or the cloning of the nucleic acid molecule. The vector may accordingly be a cloning vector or an expression vectors.

The nucleic acid molecule may be inserted into a vector using any suitable methods known in the art, for example, without limitation, the vector may be digested using appropriate restriction enzymes and then may be ligated with the nucleic acid molecule having matching restriction ends.

Expression vectors can contain a variety of control sequences, which refer to nucleic acid sequences necessary for the transcription and possibly translation of an operatively linked coding sequence in a particular host cell. In addition to control sequences that govern transcription and translation, vectors may contain additional nucleic acid sequences that serve other functions, including for example for replication, selectable markers etc.

The expression vector should have the necessary 5' upstream and 3' downstream regulatory elements such as promoter sequences such as CMV, PGK and EF1 a promoters, ribosome recognition and binding TATA box, and 3 ' UTR AATAAA (SEQ ID NO. 20) transcription termination sequence for the efficient gene transcription and translation in its respective host cell. Other suitable promoters include the constitutive promoter of simian virus 40 (SV40) early promoter, mouse mammary tumour virus (MMTV), HIV LTR promoter, MoMuLV promoter, avian leukaemia virus promoter, EBV immediate early promoter, and Rous sarcoma virus promoter. Human gene promoters may also be used, including, but not limited to the actin promoter, the myosin promoter, the haemoglobin promoter, and the creatine kinase promoter. In certain embodiments inducible promoters are also contemplated as part of the vectors expressing the CAR. This provides a molecular switch capable of turning expression of the nucleic acid molecule on or off. Examples of inducible promoters include, but are not limited to a metallothionine promoter, a glucocorticoid promoter, a progesterone promoter, or a tetracycline promoter.

Further, the expression vector may contain 5' and 3' untranslated regulatory sequences that may function as enhancer sequences, and/or terminator sequences that can facilitate or enhance efficient transcription of the nucleic acid molecule.

Examples of vectors are plasmid, autonomously replicating sequences, and transposable elements. Additional exemplary vectors include, without limitation, plasmids, phagemids, cosmids, artificial chromosomes such as yeast artificial chromosome (YAC), bacterial artificial chromosome (BAC), or PI - derived artificial chromosome (PAC), bacteriophages such as lambda phage or MI 3 phage, and animal viruses. Examples of categories of animal viruses useful as vectors include, without limitation, retrovirus (including lentivirus), adenovirus, adeno-associated virus, herpesvirus (e.g. herpes simplex virus), poxvirus, baculovirus, papillomavirus, and papovavirus (e.g. SV40). Examples of expression vectors are pCIneo vectors (Promega) for expression in mammalian cells; pLenti4/V5-DEST™ and pLenti6/V5-DEST™ for lentivirus-mediated gene transfer and expression in mammalian cells.

In certain embodiments viral vectors are preferred. A viral vector can be derived from retrovirus, lentivirus, or foamy virus. As used herein, the term, "viral vector," refers to a nucleic acid vector construct that includes at least one element of viral origin and has the capacity to be packaged into a viral vector particle. The viral vector can contain the nucleic acid molecule of the invention in place of nonessential viral genes. The vector and/or particle can be utilized for the purpose of transferring DNA, RNA or other nucleic acids into cells either *in vitro* or *in vivo.*

Accordingly, a further aspect of the invention includes a viral particle comprising a nucleic acid molecule as defined and described herein, or a preparation or composition comprising such viral particles. Such a composition may also contain at least one physiologically acceptable carrier.

Numerous forms of viral vectors are known in the art. In certain embodiments, the viral vector is a retroviral vector or a lentiviral vector. The vector may be a self-inactivating vector in which the right (3') LTR enhancer-promoter region, known as the U3 region, has been modified (e.g. by deletion or substitution) to prevent viral transcription beyond the first round of viral replication. Consequently, the vectors are capable of infecting and then integrating into the host genome only once, and cannot be passed further.

The retroviral vectors for use herein can be derived from any known retrovirus, e.g. type c retroviruses, such as Moloney murine sarcoma virus (MoMSV), Harvey murine sarcoma virus (HaMuSV), murine mammary tumour virus (MuMTV), gibbon ape leukaemia virus (GaLV), feline leukaemia virus (FLV), spumavirus, Friend, Murine Stem Cell Virus (MSCV) and Rous Sarcoma Virus (RSV), human T-cell leukaemia viruses, HTLV-1 and HTLV-2, and the lentiviral family of retroviruses, such as Human Immunodeficiency Viruses, HIV-1, HIV-2, simian immunodeficiency virus (SIV), feline immunodeficiency virus (FIV), equine immunodeficiency virus (EIV), and other classes of retroviruses.

A lentiviral vector is derived from a lentivirus, a group (or genus) of retroviruses that give rise to slowly developing disease. Viruses included within this group include HIV (human immunodeficiency virus; including HIV type 1, and HIV type 2).

A retroviral packaging cell line (typically a mammalian cell line) may be used to produce viral particles, which may then be used for transduction of the immune effector cells.

Illustrative viral vectors are described in WO2002087341, WO2002083080, WO2002082908, WO2004000220 and WO2004054512.

An exemplary retroviral vector as used in the Examples herein is pMP71 as described in Wälchli *et al* 2011. Other suitable vectors include pBABE, pWZL, pMCs-CAG, pMXs-CMV, pMXs-EF1$\alpha$, pMXs-IRES, pMXs-SR$\alpha$ and pMYs-IRES.

It is within the scope of the invention to include gene segments that cause the immune effector cells of the invention, e.g. T-cells, to be susceptible to negative selection *in vivo.* By "negative selection" is meant that the infused cell can be eliminated as a result of a change in the *in vivo* condition of the individual. The negative selectable phenotype may result from the insertion of a gene that confers sensitivity to an administered agent, for example, a compound. Negative selectable genes are known in the art, and include, *inter alia,* the following: the Herpes simplex virus type I thymidine kinase (HSV-I TK) gene (Wigler et al., Cell 11 (1):223-232, 1977) which confers ganciclovir sensitivity; the cellular hypoxanthinephosphribosyltransferase (HPRT) gene, the cellular adenine phosphoribosyltransferase (APRT) gene, bacterial cytosine deaminase, (Mullen et al., Proc. Natl. Acad. Sci. USA. 89:33-37 (1992)).

In some embodiments it may be useful to include in the genetically modified immune effector cells, such as T-cells, a positive marker that enables the selection of cells of the negative selectable phenotype *in vitro.* The positive selectable marker may be a gene which, upon being introduced into the host cell expresses a dominant phenotype permitting positive selection of cells carrying the gene. Genes of this type are known in the art, and include, *inter alia,* hygromycin-B phosphotransferase gene (hph) which confers resistance to hygromycin B, the amino glycoside phosphotransferase gene (neo or aph) from Tn5 which codes for resistance to the antibiotic G418, the dihydrofolate reductase (DHFR) gene, the adenosine daminase gene (ADA), and the multi-drug resistance (MDR) gene. Preferably, the positive selectable marker and the negative selectable element are linked such that loss of the negative selectable element necessarily also is accompanied by loss of the positive selectable marker. Even more preferably, the positive and negative selectable markers are fused so that loss of one obligatorily leads to loss of the other. An example of a fused polynucleotide that yields as an expression product a polypeptide that confers both the desired positive and negative selection features described above is a hygromycin phosphotransferase thymidine kinase fusion gene (HyTK). Expression of this gene yields a polypeptide that confers hygromycin B resistance for positive selection *in vitro,* and ganciclovir sensitivity for negative selection *in vivo.* See Lupton S. D., et al, Mol. and Cell. Biology 11:3374-3378, 1991.

For cloning of the nucleic acid molecule the vector may be introduced into a host cell (e.g. an isolated host cell) and such "production host cells" containing a cloning vector of the invention may form a further aspect of the invention. Suitable host cells can include, without limitation, prokaryotic cells, fungal cells, yeast cells, or higher eukaryotic cells such as mammalian cells. Suitable prokaryotic cells for this purpose include, without limitation, eubacteria, such as Gram-negative or Gram-positive organisms, for example, Enterobactehaceae such as *Escherichia,* e.g. *E. coli, Enterobacter, Erwinia, Klebsiella, Proteus, Salmonella,* e.g. *Salmonella typhimurium, Serratia,* e.g. *Serratia marcescans,* and *Shigella,* as well as *Bacilli* such as B. *subtilis* and B. *licheniformis, Pseudomonas* such as P. *aeruginosa,* and *Streptomyces.* A production host cell may alternatively contain an mRNA expression vector comprising the nucleic acid molecule.

The nucleic acid molecules or vectors are introduced into a host cell (e.g. a production host cell or an immune effector cell) using transfection and/or transduction techniques known in the art. As used herein, the terms, "transfection," and, "transduction," refer to the processes by which an exogenous nucleic acid sequence is introduced into a host cell. The nucleic acid may be integrated into the host cell DNA or may be maintained extra-chromosomally. The nucleic acid may be maintained transiently or a may be a stable introduction. Transfection may be accomplished by a variety of means known in the art including but not limited to calcium phosphate-DNA co-precipitation, DEAE-dextran-mediated transfection, polybrene-mediated transfection, electroporation, microinjection, liposome fusion, lipofection, protoplast fusion, retroviral infection, and biolistics. Transduction refers to the delivery of a gene(s) using a viral or retroviral vector by means of viral infection rather than by transfection. In certain embodiments, retroviral vectors are transduced by packaging the vectors into viral particles or virions prior to contact with a cell.

An "immune effector cell," is any cell of the immune system that has one or more effector functions (e.g. cytotoxic cell killing activity, secretion of cytokines, induction of ADCC and/or CDC). Representative immune effector cells thus include T-lymphocytes, in particular cytotoxic T-cells (CTLs; CD8+ T-cells) and helper T-cells (HTLs; CD4+ T-cells). Other populations of T-cells are also useful herein, for example naive T-cells and memory T-cells. Other immune effector cells include NK cells, NKT cells, neutrophils, and macrophages. As noted above, immune effector cells also include progenitors of effector cells, wherein such progenitor cells can be induced to differentiate into an immune effector cells *in vivo* or *in vitro.*

T-cells, particularly CD8+ T-cells, and NK cells represent preferred immune effector cells according to the invention.

The term "NK cell" refers to a large granular lymphocyte, being a cytotoxic lymphocyte derived from the common lymphoid progenitor which does not naturally comprise an antigen-specific receptor (e.g. a T-cell receptor or a B-cell receptor). NK cells may be differentiated by their CD3-, CD56+ phenotype. The term as used herein thus includes any known NK cell or any NK-like cell or any cell having the characteristics of an NK cell. Thus primary NK cells may be used or in an alternative embodiment, a NK cell known in the art that has previously been isolated and cultured may be used. Thus a NK cell-line may be used. A number of different NK cells are known and reported in the literature and any of these could be used, or a cell-line may be prepared from a primary NK cell, for example by viral transformation (Vogel et al. 2014, Leukemia 28:192-195). Suitable NK cells include (but are by no means limited to), in addition to NK-92, the NK-YS, NK-YT, MOTN-1, NKL, KHYG-1, HANK-1, or NKG cell lines. In a preferred embodiment, the cell is an NK-92 cell (Gong et al. 1994, Leukemia 8:652-658), or a variant thereof. A number of different variants of the original NK-92 cells have been prepared and are described or available, including NK-92 variants which are non-immunogenic. Any such variants can be used and are included in the term "NK-92". Variants of other cell lines may also be used.

Where the immune effector cell is a non-autologous cell for therapeutic use (i.e. is a donor cell) it is preferred that it is non-immunogenic, such that it does not, when administered to a subject, generate an immune response which affects, interferes with, or prevents the use of the cells in therapy.

NK cells may be naturally non-immunogenic, but NK cells or other immune effector cells may be modified to be non-immunogenic. Naturally non-immunogenic NK cells will not express the MHC molecule or only weakly express the MHC molecule, or may express a non-functional MHC molecule which does not stimulate an immunological response.

Immune effector cells which would be immunogenic may be modified to eliminate expression of the MHC molecule, or to only weakly express the MHC molecule at their surface. Alternatively, such cells may be modified to express a non-functional MHC molecule.

Any means by which the expression of a functional MHC molecule is disrupted is encompassed. Hence, this may include knocking out or knocking down a molecule of the MHC complex, and/or it may include a modification which prevents appropriate transport to and/or correct expression of an MHC molecule, or of the whole complex, at the cell surface.

In particular, the expression of one or more functional MHC class-I proteins at the surface of a cell of the invention may be disrupted. In one embodiment the cells may be human cells which are HLA-negative and accordingly cells in which the expression of one or more HLA molecules is disrupted (e.g. knocked out), e.g. molecules of the HLA MHC class I complex.

In a preferred embodiment, disruption of MHC class-I may be performed by knocking out the gene encoding $\beta_2$-microglobulin, a component of the mature MHC class-I complex. Expression of $\beta_2$m may be eliminated through targeted disruption of the $\beta_2$-microglobulin gene ($\beta_2$m), for instance by site-directed mutagenesis of the $\beta_2$m promoter (to inactivate the promoter), or within the gene encoding the $\beta_2$m protein to introduce an inactivating mutation that prevents expression of the $\beta_2$m protein, e.g. a frame-shift mutation or premature 'STOP' codon within the gene. Alternatively, site-directed mutagenesis may be used to generate non-functional $\beta_2$m protein that is not capable of forming an active MHC protein at the cell surface. In this manner the $\beta_2$m protein or MHC may be retained intracellularly, or may be present but non-functional at the cell surface.

Immune effector cells may alternatively be irradiated prior to being administered to a subject. Without wishing to be bound by theory, it is thought that the irradiation of cells results in the cells only being transiently present in a subject, thus reducing the time available for a subject's immune system to mount an immunological response against the cells. Whilst such cells may express a functional MHC molecule at their cell surface, they may also be considered to be non-immunogenic. Radiation may be from any source of $\alpha$, $\beta$ or $\gamma$ radiation, or may be X-ray radiation or ultraviolet light. A radiation dose of 5-10 Gy may be sufficient to abrogate proliferation, however other suitable radiation doses may be 1-10, 2-10, 3-10, 4-10, 6-10, 7-10, 8-10 or 9-10 Gy, or higher doses such as 11, 12, 13, 14, 15 or 20 Gy. Alternatively, the cells may be modified to express a 'suicide gene', which allows the cells to be inducibly killed or prevented from replicating in response to an external stimulus.

Thus, an immune effector cell according to the invention may be modified to be non-immunogenic by reducing its ability, or capacity, to proliferate, that is by reducing its proliferative capacity.

The modified immune effector cells of the invention may also be subject to modification in other ways, for example to alter or modify other aspects of cell function or behaviour, and/or to express other proteins. For instance, the cells may be modified to express a homing receptor, or localisation receptor, which acts to target or improve the localisation of the cells to a particular tissue or location in the body.

The present invention provides methods for making the immune effector cells which express the CAR as described herein. In one embodiment, the method comprises transfecting or transducing immune effector cells isolated from subject such that the immune effector cells express one or more CAR as described herein. In certain embodiments, the immune effector cells are isolated from a subject and modified by introduction of the nucleic acid molecule without further manipulation *in vitro.* Such cells can then be directly re-administered into the subject. In further embodiments, the immune effector cells are first activated and stimulated to proliferate *in vitro* prior to being modified to express a CAR. In this regard, the immune effector cells may be cultured before or after being genetically modified (i.e. transduced or transfected to express a CAR as described herein).

T-cells can be obtained from a number of sources, including peripheral blood mononuclear cells, bone marrow, lymph nodes tissue, cord blood, thymus issue, tissue from a site of infection, ascites, pleural effusion, spleen tissue, and tumours. In certain embodiments, T-cells can be obtained from a unit of blood collected from the subject using any number of techniques known to the skilled person, such as FICOLL™ separation. In one embodiment, cells from the circulating blood of a subject are obtained by apheresis. The apheresis product typically contains lymphocytes, including T-cells, monocytes, granulocytes, B-cells, other nucleated white blood cells, red blood cells, and platelets. In one embodiment, the cells collected by apheresis may be washed to remove the plasma fraction and to place the cells in an appropriate buffer or media for subsequent processing. In one embodiment of the invention, the cells are washed with PBS. In an alternative embodiment, the washed solution lacks calcium and/or magnesium or may lack many if not all divalent cations. As would be appreciated by those of ordinary skill in the art, a washing step may be accomplished by methods known to those in the art, such as by using a semi-automated flow-through centrifuge. For example, the Cobe 2991 cell processor, the Baxter CytoMate, or the like. After washing, the cells may be resuspended in a variety of biocompatible buffers or other saline solution with or without buffer. In certain embodiments, the undesirable components of the apheresis sample may be removed in the cell directly resuspended culture media.

In certain embodiments, T-cells are isolated from peripheral blood mononuclear cells (PBMCs) by lysing the red blood cells and depleting the monocytes, for example, by centrifugation through a PERCOLL™ gradient. A specific subpopulation of T-cells, such as CD28+, CD4+, CD8+, CD45RA+, and CD45RO+ T-cells, can be further isolated by positive or negative selection techniques. For example, enrichment of a T-cell population by negative selection can be accomplished with a combination of antibodies directed to surface markers unique to the negatively selected cells. One method for use herein is cell sorting and/or selection via negative magnetic immunoadherence or flow cytometry that uses a cocktail of monoclonal antibodies directed to cell surface markers present on the cells negatively selected. For example, to enrich for CD4+ cells by negative selection, a monoclonal antibody cocktail typically includes antibodies to CD14, CD20, CDIIb, CD16, HLA-DR, and CD8. Flow cytometry and cell sorting may also be used to isolate cell populations of interest for use in the present invention.

PBMC may be used directly for genetic modification using methods as described herein. In certain embodiments, after isolation of PBMC, T-lymphocytes are further isolated and in certain embodiments, both cytotoxic and helper T-lymphocytes can be sorted into naive, memory, and effector T-cell subpopulations either before or after genetic modification and/or expansion. CD8+ cells can be obtained by using standard methods. In some embodiments, CD8+ cells are further sorted into naive, central memory, and effector cells by identifying cell surface antigens that are associated with each of those types of CD8+ cells. In embodiments, memory T-cells are present in both CD62L+ and CD62L- subsets of CD8+ peripheral blood lymphocytes. PBMC are sorted into CD62L-/CD8+ and CD62L+/CD8+ fractions after staining with anti-CD8 and anti-CD62L antibodies. In some embodiments, the expression of phenotypic markers of central memory TCM include CD45RO, CD62L, CCR7, CD28, CD3, and CD127 and are negative for granzyme B. In some embodiments, central memory T-cells are CD45RO+, CD62L+, CD8+ T-cells. In some embodiments, effector T-cells are negative for CD62L, CCR7, CD28, and CD127, and positive for granzyme B and perforin. In some embodiments, naive CD8+ T-lymphocytes are characterized by the expression of phenotypic markers of naive T-cells including CD62L, CCR7, CD28, CD3, CD127, and CD45RA.

The immune effector cells, such as T-cells, can be modified following isolation, or the immune effector cells can be activated and expanded (or differentiated in the case of progenitors) *in vitro* prior to being modified. In another embodiment, the immune effector cells, such as T-cells, are modified by introduction of the nucleic acid molecules and then are activated and expanded *in vitro.* Methods for activating and expanding T-cells are known in the art and are described, for example, in US6905874; US6867041; US6797514; WO2012079000. Generally, such methods include contacting PBMC or isolated T-cells with a stimulatory agent and co-stimulatory agent, such as anti-CD3 and anti-CD28 antibodies, generally attached to a bead or other surface, in a culture medium with appropriate cytokines, such as IL-2. Anti-CD3 and anti-CD28 antibodies attached to the same bead serve as a "surrogate" antigen presenting cell (APC). In other embodiments, the T-cells may be activated and stimulated to proliferate with feeder cells and appropriate antibodies and cytokines using methods such as those described in US6040177; US5827642; and WO2012129514.

In one embodiment, CD34+ cells are transduced or transfected with a nucleic acid molecule in accordance with the invention. In certain embodiments, the modified (e.g. transfected or transduced) CD34+ cells differentiate into mature immune effector cells *in vivo* following administration into a subject, generally the subject from whom the cells were originally isolated. In another embodiment, $CD34^+$ cells may be stimulated *in vitro* prior to or after introduction of the nucleic acid molecule, with one or more of the following cytokines: Flt-3 ligand (FL), stem cell factor (SF), megakaryocyte growth and differentiation factor (TPO), IL-3 and IL-6 according to the methods known in the art.

The invention provides a modified immune effector cell for use in the treatment of cancer, the modified immune effector cell expressing a CAR as disclosed herein. For example, the modified immune effector cells may be prepared from peripheral blood mononuclear cells (PBMCs) obtained from a patient diagnosed with B-cell malignancy.

Standard procedures may be used for storage, e.g. cryopreservation, of the modified immune effector cells and/or preparation for use in a human or other subject.

The CAR-expressing immune effector cells can be utilized in methods and compositions for adoptive immunotherapy in accordance with known techniques In some embodiments, the cells are formulated by first harvesting them from their culture medium, and then washing and concentrating the cells in a medium and container system suitable for administration (a "pharmaceutically acceptable" carrier) in a treatment-effective amount. Suitable infusion medium can be any isotonic medium formulation, typically normal saline, Normosol R (Abbott) or Plasma-Lyte A (Baxter), but also 5 % dextrose in water or Ringer's lactate can be utilized. The infusion medium can be supplemented with human serum albumin.

A treatment-effective amount of cells in the composition is at least 2 cells (for example, at least 1 CD8+ central memory T-cell and at least 1 CD4+ helper T-cell subset) or is more typically greater than $10^2$ cells, and up to $10^6$, up to and including $10^8$ or $10^9$ cells and can be more than $10^{10}$ cells. The number of cells will depend upon the ultimate use for which the composition is intended as will the type of cells included therein. For uses provided herein, the cells are generally in a volume of a litre or less, 500 ml or less, even 250 ml or 100 ml or less. Hence the density of the desired cells is typically greater than $10^6$ cells/ml and generally is greater than $10^7$ cells/ml, generally $10^8$ cells/ml or greater. The clinically relevant number of immune cells can be apportioned into multiple infusions that cumulatively equal or exceed $10^5$, $10^6$, $10^7$, $10^8$, $10^9$, $10^{10}$, $10^{11}$, or $10^{12}$ cells. For example, 2, 3, 4, 5, 6 or more separate infusions may be administered to a patient, at intervals of 24 or 48 hours, or every 3, 4, 5, 6 or 7 days. Infusions may also be spaced at weekly, fortnightly or monthly intervals, or intervals of 6 weeks or 2, 3, 4, 5, or 6 months. It is also possible that yearly infusions may be administered. In some aspects of the present invention, since all the infused cells are redirected to a particular target antigen (namely CD37), lower numbers of cells, in the range of $10^6$-$10^8$/kilogram ($10^6$-$10^{11}$ per patient) may be administered. The cell compositions may be administered multiple times at dosages within these ranges. If desired, the treatment may also include administration of mitogens (e.g. PHA) or lymphokines, cytokines, and/or chemokines (e.g. IFN-$\gamma$, IL-2, IL-12, TNF-alpha, IL-18, and TNF-beta, GM-CSF, IL-4, IL-13, Flt3-L, RANTES, M$\Gamma$PT$\alpha$, etc.) to enhance induction of the immune response.

The CAR expressing immune effector cells of the present invention may be administered either alone, or as a pharmaceutical composition in combination with diluents and/or with other components such as IL-2 or other cytokines or cell populations. Briefly, pharmaceutical compositions of the present invention may comprise a CAR-expressing immune effector cell population, such as T-cells, as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g. aluminium hydroxide); and preservatives. Compositions of the present invention are preferably formulated for intravenous administration.

As noted elsewhere with regard to *in vivo* selectable markers for use in the vectors encoding the CAR, adverse events may be minimized by transducing the immune effector cells containing CAR with a suicide gene, such as inducible caspase 9 or a thymidine kinase, before, after or at the same time, as the cells are modified with nucleic acid molecule of the present invention.

The liquid pharmaceutical compositions, whether they be solutions, suspensions or other like form, may include one or more of the following: sterile diluents such as water for injection, saline solution, preferably physiological saline, Ringer's solution, isotonic sodium chloride, fixed oils such as synthetic mono- or diglycerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. An injectable pharmaceutical composition is preferably sterile.

Pharmaceutical compositions of the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

The immune response induced in a subject by administering CAR expressing immune effector cells described herein may include cellular immune responses mediated by cytotoxic T-cells capable of killing infected cells, regulatory T-cells, and helper T-cell responses. Humoral immune responses, mediated primarily by helper T-cells capable of activating B-cells thus leading to antibody production, may also be induced.

When an "effective amount" is indicated, the precise amount of the compositions to be administered can be determined by a physician with consideration of individual differences in age, weight, extent of malignancy, and general condition of the patient (subject). The optimal dosage and treatment regime for a particular patient can readily be determined by one skilled in the art of medicine by monitoring the subject for signs of disease and adjusting the treatment accordingly.

Thus the present invention provides for the treatment of a subject diagnosed with or suspected of having, or at risk of developing, a CD37-expressing cancer. In particular the cancer is a B-cell malignancy, or a haematological malignancy, such as a leukaemia or lymphoma, e.g. chronic lymophocytic leukaemia (CLL), acute lymphoblastic leukaemia (ALL), hairy cell leukaemia (HCL), B-cell non-Hodgkin lymphoma (B cell NHL), lymphoplasmacytic lymphoma, multiple myeloma or other similar malignancies, or any other CD37-expressing cancer, such as ovarian cancer or urothelial cancer.

The CAR-expressing immune effector cells may be administered in combination with one or more other therapeutic agents, which may include any other known cancer treatments, such as radiation therapy, chemotherapy, transplantation, immunotherapy, hormone therapy, photodynamic therapy, etc. The compositions may also be administered in combination with antibiotics or other therapeutic agents, including e.g. cytokines (e.g. IL-1, IL-2, IL-3, IL-4, IL-5, IL-6, IL-7, IL-8, IL-9, IL-10, IL-11, IL-12, IL-13, IL-15 and IL-17), growth factors, steroids, NSAIDs, DMARDs, antiinflammatories, analgesics, chemotherapeutics (e.g. monomethyl auristatin E, fludarabine, gemcitabine, capecitabine, methotrexate, taxol, taxotere, mercaptopurine, thioguanine, hydroxyurea, cytarabine, cyclophosphamide, ifosfamide, nitrosoureas, cisplatin, carboplatin, oxaliplatin, mitomycin, dacarbazine, procarbazine, etoposide, teniposide, campathecins, bleomycin, doxorubicin, idarubicin, daunorubicin, dactinomycin, plicamycin, mitoxantrone, L-asparaginase, 5-fluorouracil), radiotherapeutics, immune checkpoint inhibitors (e.g. Tremelimumab, Ipilimumab, Nivolumab, MK-3475, Urelumab, Bavituximab, MPDL3280A, MEDI4736), small molecule inhibitors or other active and ancillary agents.

The term 'target cell' refers to any cell which is to be killed or abrogated by the modified immune effector cells of the invention. As noted above, it will be generally be a CD37-expressing cancer cell, preferably a malignant B-cell or a cell associated with a B-cell malignancy. The immune effector cells of the invention may accordingly be used in B-cell depletion therapies.

Cancer is defined broadly herein to include any neoplastic condition, whether malignant, pre-malignant or non-malignant. Generally, however, it may be a malignant condition. Both solid and non-solid tumours are included and the term "cancer cell" may be taken as synonymous with "tumour cell".

In one embodiment of the present invention the cells may be administered to a subject directly intravenously. In an alternative embodiment the cells may administered directly into a tumour via intratumoural injection.

The subject to be treated using the methods and cells of the present invention may be any species of mammal. For instance, the subject may be any species of domestic pet, such as a mouse, rat, gerbil, rabbit, guinea pig, hamster, cat or dog, or livestock, such as a goat, sheep, pig, cow or horse. In a further preferred embodiment of the invention the subject may be a primate, such as a monkey, gibbon, gorilla, orang-utang, chimpanzee or bonobo. However, in a preferred embodiment of the invention the subject is a human.

It is contemplated that immune effector cells for use in the present invention may be obtained from any species of mammal, however, in a preferred embodiment the immune effector cells will be from the same species of mammal as the subject to be treated.

Embodiments of the invention include:

1. A nucleic acid molecule encoding a chimeric antigen receptor (CAR) directed against the antigen CD37, wherein said CAR when expressed on the surface of an immune effector cell is capable of binding to the antigen CD37 expressed on a target cell surface and comprises an antigen-binding domain comprising a $V_L$ sequence and a $V_H$ sequence each comprising three CDR sequences, wherein

    a) CDRs 1, 2 and 3 of the $V_L$ sequence have the sequences of SEQ ID NOs. 43, 44 and 35 respectively; and
    b) CDRs 1, 2 and 3 of the $V_H$ sequence have the sequences of SEQ ID NOs: 45, 46 and 34 respectively, and

    wherein one or more of said CDR sequences may optionally be modified by substitution, addition or deletion of 1 to 3 amino acids.

2. The nucleic acid molecule of embodiment 1, wherein the amino acid sequences of at least CDR3 of said $V_L$ and $V_H$ sequences are unmodified, preferably wherein the amino acid sequences of all of the CDRs are unmodified.

3. The nucleic acid molecule of embodiment 1 or 2, wherein the $V_L$ sequence has an amino acid sequence as shown in SEQ ID NO. 3 or an amino acid sequence having at least 60 % sequence identity thereto.

4. The nucleic acid molecule of any one of embodiments 1 to 3 wherein the $V_H$ sequence has an amino acid sequence as shown in SEQ ID NO. 1 or an amino acid sequence having at least 60 % sequence identity thereto.

5. The nucleic acid molecule of any one of embodiments 1 to 4, wherein the framework regions of said $V_L$ and $V_H$ sequences have at least 60 % amino acid sequence identity to the framework regions of SEQ ID NOS. 3 and 1 respectively.

6. The nucleic acid molecule of any one of embodiments 1 to 5, wherein the $V_L$ sequence has an amino acid sequence as shown in SEQ ID NO. 3 or an amino acid sequence having at least 95 % sequence identity thereto, and the $V_H$ sequence has an amino acid sequence as shown in SEQ ID NO. 1 or an amino acid sequence having at least 95 % sequence identity thereto.

7. The nucleic acid molecule of any one of embodiments 1 to 5, wherein the framework regions of said $V_L$ and $V_H$ sequences are humanised.

8. The nucleic acid molecule of embodiment 7, wherein the CDR sequences are unmodified.

9. The nucleic acid molecule of any one of embodiments 1 to 8, wherein the $V_L$ sequence has an amino acid sequence as shown in SEQ ID NO. 47 or an amino acid sequence having at least 95 % sequence identity thereto, preferably wherein the CDR sequences are unmodified.

10. The nucleic acid molecule of any one of embodiments 1 to 9, wherein the $V_H$ sequence has an amino acid sequence as shown in SEQ ID NO. 48 or an amino acid sequence having at least 95% sequence identity thereto, preferably wherein the CDR sequences are unmodified.

11. The nucleic acid molecule of any one of embodiments 1 to 9, wherein the antigen-binding domain is a scFv comprising the $V_L$ and $V_H$ sequences.

12. The nucleic acid molecule of any one of embodiments 1 to 10, wherein the antigen-binding domain or scFV comprises, in the following order, the $V_L$ sequence, a linker sequence, and the $V_H$ sequence.

13. The nucleic acid molecule of embodiment 12, wherein the linker sequence is $(G4S)_4$ (SEQ ID NO. 5).

14. The nucleic acid molecule of any one of embodiments 1 to 13, wherein the CAR comprises a plasma membrane targeting sequence.

15. The nucleic acid molecule of embodiment 14, wherein said plasma membrane targeting sequence is positioned upstream of the antigen-binding domain or scFv.

16. The nucleic acid molecule of embodiment 14 or 15, wherein the plasma membrane targeting sequence is the L-chain having the sequence set out in SEQ ID NO. 6 or an amino acid sequence having at least 95 % sequence identity thereto.

17. The nucleic acid molecule of any one of embodiments 1 to 16, wherein the nucleic acid molecule comprises the nucleotide sequence of SEQ ID NO. 4 encoding the $V_L$ sequence of SEQ ID NO. 3 or a nucleotide sequence having at least 60 % sequence identity thereto or a nucleotide sequence degenerate thereto, and the nucleotide sequence of SEQ ID NO. 2 encoding the $V_H$ sequence of SEQ ID NO. 1 or a nucleotide sequence having at least 60 % sequence identity thereto or a nucleotide sequence degenerate thereto.

18. The nucleic acid molecule of any one of embodiments 1 to 17, wherein the nucleic acid molecule comprises a $V_L$-encoding sequence having the nucleotide sequence of SEQ ID NO. 49 or a nucleotide sequence having at least 95 % sequence identity thereto or a nucleotide sequence degenerate thereto, and/or a $V_H$-encoding sequence having the nucleotide sequence of SEQ ID NO. 50 or a nucleotide sequence having at least 95 % sequence identity thereto or a nucleotide sequence degenerate thereto.

19. The nucleic acid molecule of any one of embodiments 1 to 18, wherein the CAR comprises, downstream of an extracellular domain comprising the antigen-binding domain, a hinge domain, a transmembrane domain, an intracellular signalling domain, and optionally one or more co-stimulatory signalling domains.

20. The nucleic acid molecule of any one of embodiments 1 to 19, wherein the intracellular signalling domain of the CAR is a CD3$\zeta$ or FcR$\gamma$ intracellular signalling domain, preferably a human CD3$\zeta$ domain, more preferably a human CD3$\zeta$ domain having the amino acid sequence of SEQ ID NO.8 or an amino acid sequence having at least 95 % sequence identity thereto.

21. The nucleic acid molecule of embodiment 19 or 20, wherein the hinge domain is derived from CD8$\alpha$, CD4, CD28, or CD7, preferably human CD8$\alpha$, CD4, CD28, or CD7, or from the Fc of an immunoglobulin, for example IgG, preferably wherein the Fc-derived hinge domain does not comprise a CH3 domain, e.g. comprises or consists of the CH2 domain or a part thereof.

22. The nucleic acid molecule of embodiment 21, wherein the hinge domain is:

(i) the hinge domain of CD8$\alpha$ having the amino acid sequence of SEQ ID NO. 9 or an amino acid sequence having at least 95 % sequence identity thereto; or
(ii) an IgG hinge domain having the amino acid sequence of SEQ ID NO. 10 or an amino acid sequence having at least 95 % sequence identity thereto.

23. The nucleic acid molecule of any one of embodiments 19 to 22, wherein the hinge domain is attached to the transmembrane domain by means of a linker, preferably wherein the linker has the sequence KDPK (SEQ ID NO. 11).

24. The nucleic acid molecule of any one of embodiments 1 to 23, wherein the transmembrane domain of the CAR is a transmembrane domain derived from CD8α, CD3ζ, CD28, CD4, CD45, CD9, CD16, CD22, CD33, CD64, CD80, CD86, CD134, CD137, or CD154, preferably human CD8α, CD3ζ, CD28, or CD4.

25. The nucleic acid molecule of embodiment 24, wherein the transmembrane domain is the CD8α transmembrane domain having the amino acid sequence of SEQ ID NO. 12 or an amino acid sequence having at least 95 % sequence identity thereto.

26. The nucleic acid molecule of any one of embodiments 19 to 25, wherein the co-stimulatory domain is derived from any one or more of 4-1BB (DAP10/CD137), CD28, OX-40 (CD134), CD278 (ICOS), CD27, CD30, CD40, PD-1, LFA-1, CD2, CD7, LIGHT, NKD2C, BH-H2 and a ligand which specifically binds CD83.

27. The nucleic acid molecule of embodiment 26, wherein the co-stimulatory domain is the intracellular domain of 4-1BB having the amino acid sequence of SEQ ID NO. 13 or an amino acid sequence having at least 95 % sequence identity thereto.

28. The nucleic acid molecule of any one of embodiments 1 to 27, wherein the CAR comprises, preferably in the following order:

(i) a hinge domain being (a) the hinge domain of CD8α having the amino acid sequence of SEQ ID NO. 9 or an amino acid sequence having at least 95 % sequence identity thereto; or (b) an IgG hinge domain having the amino acid sequence of SEQ ID NO. 10 or an amino acid sequence having at least 95 % sequence identity thereto;
(ii) a CD8α transmembrane domain having the amino acid sequence of SEQ ID NO. 12 or an amino acid sequence having at least 95 % sequence identity thereto;
(iii) a co-stimulatory domain being the intracellular domain of 4-1BB having the amino acid sequence of SEQ ID NO. 13 or an amino acid sequence having at least 95 % sequence identity thereto; and
(iv) a human CD3ζ domain having the amino acid sequence of SEQ ID NO. 8 or an amino acid sequence having at least 95 % sequence identity thereto.

29. The nucleic acid molecule of embodiment 28, wherein the hinge domain is attached to the transmembrane domain by means of a linker having the sequence KDPK (SEQ ID NO. 11) and/or wherein the CAR further comprises a plasma membrane targeting sequence having the sequence set out in SEQ ID NO. 6 or an amino acid sequence having at least 95 % sequence identity thereto positioned upstream of the antigen-binding domain or scFv.

30. The nucleic acid molecule of any one of embodiments 1 to 29, wherein said molecule comprises the nucleotide sequence of SEQ ID NO. 14 or SEQ ID NO. 15, or a nucleotide sequence having at least 95 % sequence identity thereto or a nucleotide sequence degenerate thereto.

31. The nucleic acid molecule of any one of embodiments 1 to 30, wherein said nucleic acid molecule is RNA.

32. A vector comprising the nucleic acid molecule of any one of embodiments 1 to 31.

33. The vector of embodiment 32, wherein said vector is an expression vector or a cloning vector.

34. The vector of embodiment 32 or 33 wherein said vector is an mRNA expression vector or a viral vector.

35. The vector of any one of embodiments 32 to 34 wherein the vector is a retroviral or lentiviral vector.

36. An immune effector cell comprising the nucleic acid molecule of any one of embodiments 1 to 31 or the vector of any one of embodiments 32 to 35.

37. The immune effector cell of embodiment 36, wherein the cell is a T-cell or an NK cell.

38. A composition comprising the immune effector cell of embodiment 36 or 37 and at least one physiologically acceptable carrier or excipient.

39. An immune effector cell as defined in embodiment 36 or 37, or a composition as defined in embodiment 38, for use in therapy.

40. An immune effector cell as defined in embodiment 36 or 37, or a composition as defined in embodiment 38 for use in adoptive cell transfer therapy.

41. The immune effector cell or composition for use according to embodiment 39, for use in the treatment of cancer.

42. The immune effector cell or composition for use according embodiment 41, wherein the cancer is a B-cell malignancy.

43. A method of treating cancer, particularly a B-cell malignancy, said method comprising administering to a subject in need thereof a composition as defined in embodiment 38.

44. A method of generating a CD37-specific immune effector cell, said method comprising introducing a nucleic acid molecule of any one of embodiments 1 to 31 or a vector of any one of embodiments 32 to 35 into an immune effector cell.

45. The method of embodiment 44, wherein said method further comprises stimulating the cells and inducing them to proliferate before and/or after introducing the nucleic acid molecule or vector.

46. The method of embodiment 44 or 45 wherein the cell is a T-cell or NK cell.

47. Use of the immune effector cell of embodiment 36 or 37 for the manufacture of a medicament for use in cancer therapy, particularly for treating a B-cell malignancy.

The present invention may be more fully understood from the Examples below and in reference to the drawings, in which:

Figure 1 shows an overview of two different CAR constructs made and tested in Example 1. On the left is an initial 3rd generation CAR construct: the extracellular region is composed of a scFv (single chain variable fragment) domain and a Fc domain (from an IgG1 antibody) which forms the hinge, the transmembrane region is composed of the transmembrane domain of the CD28 receptor (TM-CD28), and the intracellular region is composed of a fusion of the intracellular domains of three signalling receptors: CD28, OX-40 and CD3ζ. Upon expression, this type of design showed some cytotoxicity to the expressing cells due to high signalling in steady state, and the Fc hinge was bound by Fc Receptor-expressing cells (macrophages, NK cells) which inhibit the CAR effect. This design may be modified to a second generation CAR (as shown on the right) by modifying the signalling tail to contain only two signalling domains (here the intracellular domains of 4-1BB and CD3ζ) and the Fc domain may be replaced by either a CD8 hinge or a short "antibody" hinge carrying the dimerization cysteines. Due to its relative ease of detection, a 3rd generation CAR was used in preliminary experiments (Figs. 2 and 3)

Figure 2 shows 3rd generation CAR expression on the surface of T-cells following retroviral transfection. Expression of 3 CARs is shown: anti-CD37-(HH1)CAR (aCD37CAR graph), anti-CD19-(Æ-1)CAR (aCD19CAR graph) and anti-CD19-(FMC63)CAR. Expression was measured by identification of cells expressing the Fc hinge domain by flow cytometry. Mock transduced cells were used as a negative control.

Figure 3 shows the results of a functional assay of redirected effector cells. When the redirected effector cells are stimulated, cytotoxic granules are released and the cells become CD107a positive. Thus CD107a levels correspond to the level of effector cell activation.

Top: the anti-CD37-(HH1)CAR is active and specific. The anti-CD37-(HH1)CAR was compared to the clinical anti-CD19-(fmc63)CAR in a functional assay, where retrovirally redirected T-cells were incubated with double positive target cells (the Mantle cell lymphoma B-cell line "Mino"), double negative targets (HEK cells: human embryonic kidney cells), or CD37 positive HEK cells (HEK cells transfected with a CD37-encoding cDNA). The figure shows that the anti-CD37-(HH1)CAR is specific and as potent as a clinical CAR to stimulate redirected T-cells upon target recognition.
Bottom: NK-92 cells were transduced with the indicated 2nd generation constructs or not transduced (NT) and were incubated with target cells expressing the indicated antigens. Specific recognition of the respective targets of the indicated CARs was demonstrated.

Figure 4 shows expression of 2nd generation CARs using (A) retrovirus or (B) mRNA electroporation. Cells were stained 5 days (A) or 12 hours (B) after transduction/transfection using an anti-mouse Fab antibody. In (B), CAR constructs (as defined in Table 6) were analysed in T-cells obtained from two donors. Expression was only visible from constructs 756 and 757 (anti-CD19-(Æ-1)CAR and anti-CD37-(HH1)CAR, respectively, both with CD8 hinges). Importantly, expression from constructs 755 and 758 (anti-CD19-(fmc63)CAR with CD8 and Ab hinges, respectively) will not be detected by anti-mouse Fab antibody, since these CARs have been humanised.

Figure 5 shows the results of a functional assay (using CD107a as a degranulation marker) in which the redirected T-cells shown in Figure 4 were tested for activity against cells expressing their target proteins. The constructs carried by the T-cells are defined in Table 6. Redirection of CD4+ and CD8+ T-cells from the two donors were individually analysed according to CD107a cell surface expression. Target cells are K562 cells (CD37-/CD19-) and Mino cells (CD37+/CD19+). As shown, CD4+ T-cells might not react in all donors, and CD37CAR with the ab-hinge does not redirect either the CD4+ or CD8+ T-cells, suggesting that the hinge is important for proper CD37 recognition. This is not the case for CD19CAR, which recognises CD19 with both designs (i.e. with the Ab-hinge and the CD8 hinge). It should be noted that activity detection such as shown in this figure is more sensitive than direct protein labeling.

Figure 6 shows that CAR redirected effector cells kill their targets. (A) NK-92 cells expressing the indicated construct were incubated with Europium-labelled target cells (BL41 CD19+/CD37+) for two hours, and Europium release was detected as an indicator of target cell lysis. Non-transfected (NT) NK cells were used as a negative control. (B) The same experiment was run over 48 hours and the presence of remaining BL41 cells was monitored by flow cytometry.

Figure 7 shows that CAR redirected T-cells are fully functional. (A) A panel of B-cells was used to stimulate the redirected T-cells, and as targets for cell killing thereby. The target B-cells were CD37 and CD19 stained. (B) Cytokine release (IFNγ, TNFα and IL-2) from CAR redirected T-cells in response to U2932 cell exposure was monitored. (C) T-cells isolated from PBMC were electroporated with anti-CD37-(HH1)CAR or anti-CD19-(fmc63)CAR (indicated by triangles and squares, respectively) and tested for their killing ability against a panel of B-cells. The negative control (circles) was the same T-cells without CAR constructs. The experiment was run over 24 hours.

## EXAMPLES

### Example **1**

This shows the design and construction and testing of anti-CD37 CARs according to the present invention.

MATERIALS AND METHODS

CD37 CARs were designed to contain an antigen-binding part (scFv), in addition to a signaling part of the TCR (CD3ζ), and signaling parts of co-receptors (CD28, CD137/4-1BB and/or OX40). Importantly, the combination of signaling domains included in the CAR is critical for its performance. This is also true for the hinge domain connecting the scFv and the transmembrane region (CD8 hinge, antibody hinge or CH2CH3 hinge).

*5'-RACE and Antibody Sequence Determination:*

The hybridoma producing the HH1 antibody was described in (Smeland *et al,* 1985). The HH1 antibody is specific for the B-cell marker CD37. One million hybridoma cells were pelleted and deep frozen at -80°C. RNA was prepared using Stratagene RNA kit (Absolutely RNA Miniprep kit, 400800) with a starting volume of 350 μl lysis buffer. Final elution was done in 50 μl and the yield was less than 100 ng/μl.

cDNA was prepared using oligo dT primers in the following mix: 1 μl oligo dT 50 μM (Invitrogen, 18418-020), 1 μl dNTP 10 mM (Roche, 11969064001), 12 μl RNA (<1 μg), 1 μl DTT, 0.5 μl RNAsin® (Stratagene, N2511) and 1 μl SuperScript™ III Reverse Transcriptase (Invitrogen, 18080-044) and incubated at 50°C for 1 hr. RNAsin® was inactivated for 15' at 60°C. RNA was then removed by RNaseH treatment (21 μl cDNA+1 μl RNaseH (NEB, M0297S) at 37°C for 20 min). Quality of cDNA was then checked by actin PCR amplification (0.5 μl cDNA, 1.5 μl each primer (GCTCCG-GCATGTGCAA (SEQ ID NO. 41), AGGATCTTCATGAGGTAGT (SEQ ID NO. 42)), 1.5 μl dNTP, 1 μl Taq DNA polymerase, 5 μl 10x buffer, 38.5 μl $H_2O$) under the following conditions: 3' 94°C, 30x(30" 94°C, 30" 53°C and 1' 72°C), 7' 72°C and 4°C ∞.

cDNA was then cleaned and precipitated as follows: 22 μl cDNA, 0.5 μl Glycogen (Fermentas, R0561), 2.3 μl

NaAc 3M pH 5.6, 65 μl Et-OH 100 % and incubated at -20°C for 20 min, spun down (10' at >10'000 x g at 4°C). Pellet was washed with 200 μl EtOH 70% and dried. Finally, cDNA was resuspended in 11 μl $dH_2O$.

3' terminal dC tailing of cDNA was performed as follows: 10 μl of the purified cDNA from the previous step was incubated for 1 min at 95°C and chilled on ice before addition of other reagents: 10 μl template, 4 μl RB 5x, 4 μl $CoCl_2$, 1 μl dCTP (10 mM, Invitrogen, 10217-016) and 1 μl TdT (400U, Roche, 03333566001) and incubated for 15 min at 37°C.

dC-tailed cDNA was then precipitated as previously (22 μl cDNA, 0.5 μl Glycogen (Fermentas, R0561), 2.1 μl NaAc 3M pH 5.6, 65 μl Et-OH 100 % and incubated at -20°C for 20 min, spun down (10' at >10'000 x g at 4°C). Pellet was washed with 200 μl EtOH 70 % and dried. Finally cDNA was resuspended in 24 μl $dH_2O$.

Antibody amplification was performed in two steps by nested PCR using the following mix: 1 μl cDNA (dC-tailed for the $1^{st}$ PCR and $1^{st}$ amplification product for the second PCR), 1.5 μl of each primer (see Tables 1 and 2 below), 1.5 μl dNTP (Roche, 11969064001), 1 μl Titanium Taq DNA Polymerase (Clontech, 639208), 5 μl 10x buffer, 38.5 μl $H_2O$ in the following conditions (same conditions for both PCRs, only primers changed): 4' 94°C, 27x(1' 94°C, 1' 53°C and 1' 72°C), 7' 72°C and 4°C ∞.

| Table 1 | $1^{st}$ PCR | |
|---|---|---|
| PRIMERS | Forward | Reverse |
| Heavy chain amplification | CACCGGGIIGGGIIGGGII (SEQ ID NO. 22) | GGTCACTGTCACTGGCTCAG (G1, SEQ ID NO. 23)<br>TAGAGGTCAGACTGCAGGACA (G2a, SEQ ID NO. 24)<br>GAGTTCCAAGTCACAGTCACTG (G2b, SEQ ID NO. 25) |
| Light chain amplification | CACCGGGIIGGGIIGGGII (SEQ ID NO. 22) | GCCATCAATCTTCCACTTGACA (IgK, SEQ ID NO. 26) |

| Table 2 | $2^{nd}$ PCR | |
|---|---|---|
| PRIMERS | Forward | Reverse |
| Heavy chain amplification | CACCGGGIIGGGIIGGGII (SEQ ID NO. 22) | CTTGACCAGGCATCCTAGAGTCA (G2a, SEQ ID NO. 27)<br>ATACGCGTGTTTGCAGCAGATCCAGGG (G1, SEQ ID NO. 28)<br>ATACGCGTAGTTGTATCTCCACACCCAGG (G2b, SEQ ID NO. 29) |
| Light chain amplification | CACCGGGIIGGGIIGGGII (SEQ ID NO. 22) | CAAGAAGCACACGACTGAGGC (SEQ ID NO. 30) |

PCR fragments from the $2^{nd}$ PCR were gel-purified and cloned into pGEM vector (Stratagene, A1360). Bacteria were selected and blue/white screening was performed. Sequences were analyzed using the IMGT online antibody analysis tool (http://www.imgt.org/) and the anti-CD37 antibody composition was found to be the following:

$V_H$ (Musmus IGHV1S135*01, CDR1: GYSFTDYN (SEQ ID NO. 45); CDR2: IDPYNGDT (SEQ ID NO. 46); CDR3: ARSPYGHYAMDY (SEQ ID NO. 34)) and $V_L$ (Musmus IGKV6-25*01, CDR1: QDVSTA (SEQ ID NO. 43); CDR2: WAS (SEQ ID NO. 44); CDR3: RQHYSTPFT (SEQ ID NO. 35)).

Further alignment to the IMGT database identified the somatic mutations and the sequences of the V chains, as indicated below:

$V_H$ (SEQ ID NO. 36): 3 mutations: G57D/S59T/M811

MEWSWIFLFLLSGTTGVHSE$_{1}$IQLQQSGPELVKPGASVKVSCKASGYSFTDYNMYWV
KQSHGKSLEWIGYIDPYNGD$_{57}$TT$_{59}$YNQKFKGKATLTVDKSSSTAFI$_{81}$HLNSLTSEDSA
VYYCARSPYGHYAMDYWGQGTSVTVSSAKTTPPSVYPLAPGSAAQTRV

$V_L$ (SEQ ID NO. 37): 6 mutations: F10L/M11L/A34D/Y49N/V78M/Q89R

MAFVFVFLWLSGVDGD$_{1}$IVMTQSHKL$_{10}$L$_{11}$STSVGDRVSITCKASQDVSTAVD$_{34}$WYQQ
KPGQSPKLLIN$_{49}$WASTRHTGVPDRFTGSGSGTDYTLTISSM$_{78}$QAEDLALYYCR$_{89}$QHY
STPFTFGSGTKLEIKRADAAPTVSIFPPSSEQLTSGGASVVCFLNH

Subscript numbers identify the position of the previous residue. Number 1 identifies the first residue of the mature protein following leader peptide cleavage.

*CAR design and subcloning:*

Based on the cDNA sequence, a single chain antibody was designed following this scaffold: L-chain - $V_L$-chain - $(G4S)_4$ - $V_H$-chain. The amino acid sequence of this HH1 anti-CD37 antibody-derived scFv is shown in SEQ ID NO. 7. The leader sequence has the sequence of SEQ ID NO. 6, the $V_L$-chain section has the sequence of SEQ ID NO. 3, the $(G4S)_4$ linker has the sequence of SEQ ID NO. 5, and the $V_H$-chain section has the sequence of SEQ ID NO. 1.

Signal sequence-$V_L$-*linker*-$V_H$

A synthetic gene was generated having the nucleotide sequence of SEQ ID NO. 38. This scaffold was inserted into a third generation CAR retroviral expression vector (pSFG.aCD19fmc63-HCH2CH3-CD280XZ, Pulè et al. 2005, Mol. Ther. 12(5): p933-941) using compatible restriction sites from which aCD19fmc63 had been removed. For this, NcoI and BamHI restriction sites were used, which are present at the 5' and 3' ends respectively of SEQ ID NO. 38. The final product of this plasmid is a CD37-CAR-3rd generation, with a CH2CH3 hinge, a CD28 TM domain and a CD28 - OX40 - CD3ζ signaling domain, the 3rd generation CAR having the amino acid sequence of SEQ ID NO. 39.

The various domains of this CAR are as follows (from N-terminus to C-terminus):
scFv with L-chain leader sequence, as shown in SEQ ID NO. 7; CH2CH3 hinge domain as shown in SEQ ID NO. 40; CD28 TM domain as shown in SEQ ID NO. 17; CD28 intra-cellular domain as shown in SEQ ID NO. 18; OX-40 co-stimulatory domain as shown in SEQ ID NO. 19; and CD3ζ signalling domain as shown in SEQ ID NO. 8.

This construct was used to validate binding of the scFv to CD37 and the signalling from the CAR, but demonstrated some toxicity in expressing cells. An alternative 3rd generation CAR construct has the amino acid sequence of SEQ ID NO. 51, wherein the CH2CH3 hinge domain and the CD28 TM domain of the construct represented by SEQ ID NO. 39 are switched for the CD8 hinge and TM domains respectively. The CD8 hinge domain has the sequence shown in SEQ ID NO. 9 and the CD8 TM domain has the sequence shown in SEQ ID NO. 12. SEQ ID NO. 52 represents the DNA sequence for the construct of SEQ ID NO. 51. However, 2nd generation CAR constructs were designed and constructed for further studies.

In order to generate the new second generation CAR constructs the described CD37-binding scFv was fused to two different second generation signalling tails (see below). These tails were generated with compatible cloning ends so the CD37 antibody HH1 scFv could be extracted and subcloned in-frame into them using NcoI and BamH1. All the constructs were first subcloned into pENTR vector (Invitrogen) and then recombined using the Gateway technology into either a retroviral expression vector (MP71-Gateway) or an mRNA synthesis vector (pClpA102-Gateway). These expression vectors are described in Wälchli *et al,* 2011.

Complete CAR constructs:

scFv - CD8-hinge - CD8-TM - 4-1BB - CD3ζ, designated "8843". This construct comprises domains as follows (from N-terminus to C-terminus): the scFv of SEQ ID NO. 7; the CD8 hinge of SEQ ID NO. 9; the CD8 TM-domain of SEQ ID NO. 12; the 4-1BB co-stimulatory domain of SEQ ID NO. 13; and the CD3ζ signaling domain of SEQ ID NO. 8. The complete amino acid sequence of CAR 8843 is shown in SEQ ID NO. 32, and the complete DNA sequence encoding this CAR is shown in SEQ ID NO. 14.

scFv - ab-hinge-linker - CD8-TM - 4-1BB - CD3ζ, designated "ab843". This construct comprises domains as follows (from N-terminus to C-terminus): the scFv of SEQ ID NO. 7; the ab-hinge-linker of SEQ ID NO. 21; the CD8 TM-domain of SEQ ID NO. 12; the 4-1BB co-stimulatory domain of SEQ ID NO. 13; and the CD3ζ signaling domain of SEQ ID NO. 8. The complete amino acid sequence of CAR ab843 is shown in SEQ ID NO. 33, and the complete DNA sequence encoding this CAR is shown in SEQ ID NO. 15.

An overview of different CAR designs is presented in Figure 1.

*CAR expression:*

Retroviral particles of pSFG.aCD37HCH2CH3-CD280XZ (encoding the 3rd generation CAR) were prepared as follows: HEK-Phoenix (HEK-P, our collection) were grown in DMEM (PAA) supplemented with 10 % HyClone FCS (HyClone) and 1 % antibiotic-antimicotic (penicillin/streptomycin, P/S, PAA). Viral particles were produced using HEK-P cells transfected using Fugene-6 (Roche) with retroviral packaging vectors and the expression vector. After 24 hours of incubation at 37°C, medium was replaced with DMEM 1 % FCS and cells were incubated at 32°C. Supernatants were harvested after 24 and 48 hours.

PBMCs isolated from healthy donors were cultured and activated in X-VIVO™ 20 media supplemented with 5 % human serum and 100 U/ml IL2 (R&D Systems) for 48 hours in a 24-well plate pre-coated with anti-CD3 (OKT-3) and anti-CD28 antibodies (BD Biosciences). After two days of culture, PBMCs were harvested. Spinoculation of T-cells from PBMC was performed with 1 ml of retroviral supernatant in a 12-well culture non-treated plate (Nunc A/S) pre-coated with retronectin (20 mg/mL, Takara Bio.). Spinoculation was repeated once, 1 day after the 1st spinoculation. On day 7 post-transduction, PBMCs were used for experiments. The same protocol was followed to express the 2nd generation CAR, except that it was cloned into an MP71-gateway adapted vector (see Wälchli *et al,* 2011).
mRNA was prepared following the standard protocol:

Reagents:

| Table 3 | |
|---|---|
| Name | Product Reference |
| CutSmart Buffer | NEB, B7204S |
| Mfel-HF | NEB, R3589L |
| Nuclease Free Water (NFW) | Sigma, W4502 |
| Notl-HF | NEB, R3189L |
| Flash Gel DNA Cassette | Lonza, 57023 |
| Flash Gel Loading Dye | Lonza, 50463 |
| Flash Gel DNA Marker 100-4000bp | Lonza, 50473 |
| Wizard SV Gel and PCR Clean-Up System | Promega, A9281 |
| Molecular AccuGENE | Lonza, 51200 |
| RiboMAX Large Scale RNA Production System | Promega, P1300 |
| ARCA | Tri-Link, N-703 |
| MEGAclear | Ambion, AM1908 |
| Flash Gel RNA Cassette | Lonza, 57027 |
| Flash Gel RNA Marker | Lonza, 50577 |
| Formaldehyde Sample Buffer | Lonza, 50569 |
| Ethidium Bromide | Invitrogen, 15585-011 |
| Gel Loading Dye | NEB, B70245 |
| 1 kb DNA ladder | NEB, N3232L |

Reaction mix for linearization of template DNA:

| Table 4 | |
|---|---|
| Reagent | Volume |
| MQ water (to 500 μl) | X μl |
| Cut smart buffer (x10) | 50 μl |
| Mfel-HF | 15 μl |
| Plasmid DNA (100 μg) | Y μl |
| TOTAL | 500 μl |

100 μg Plasmid DNA was digested, enough for 500 μl mRNA synthesis (40 μg/200 μl synthesis) by incubation for 4 hrs at 37°C, followed by:

1. Inactivation of enzyme activity by placing tube in heating block, 65°C, 15 mins.

2. Proceeding with purification (or storage at -20°C).

Purification of linearised template DNA:

1. Wizard SV Gel and PCR Clean-Up System by Promega were used.

2. An equal volume of Membrane Binding Solution was added to the DNA and mixed well.

Binding of DNA

3. SV Minicolumn was inserted into Collection tube.
4. Dissolved mixture (1000 μl) was transferred to the Minicolumn assembly x2, then incubated at RT for 1 minute.
5. Minicolumn assembly was centrifuged at 16,000 x g for 1 min. Flow-through was discarded and the Minicolumn re-inserted into the Collection Tube.

Washing

6. 700 μl Membrane Wash Solution (with EtOH added) was added to the Minicolumn. The Minicolumn assembly was centrifuged at 16,000 x g for 1 min. Flow-through was discarded and the Minicolumn reinserted into the Collection Tube.

7. Step 6 was repeated with 500 μl Membrane Wash Solution. Centrifugation was performed at 16,000 x g for 1 min. Flow-through was discarded, and the column centrifuged for another 5 min at 16,000 x *g*.

8. The Minicolumn was carefully transferred to a clean 1.5 ml microcentrifuge tube.

9. 50 μl NFW was added to the Minicolumn. Minicolumn was then incubated at RT for 1 min, then centrifuged at 16,000 x g for 1 min.

10. Minicolumn was discarded.

11. DNA concentration was measured using NanoDrop ND-1000 Spectrophotometer.

*In Vitro* Transcription:

Reaction mix for mRNA Synthesis:

| Table 5 | |
|---|---|
| Reagent | Volume |
| Nuclease Free Water (to 100 µl) | X µl |
| rATP | 7.5 µl |
| rCTP | 7.5 µl |
| rUTP | 7.5 µl |
| rGTP | 2.3 µl |
| ARCA Cap | 9.0 µl |
| T7 Buffer* | 20.0 µl |
| Template DNA (50 ng/µl) | Y µl |
| T7 Enzyme Mix | 10.0 µl |
| Total | 100 (µl) |
| * Buffer was heated to 37°C to dissolve precipitated material and mixed regularly for complete dissolution. Buffer was kept at RT while setting up the reaction.<br>1. Mixture was mixed with a pipette and incubated at 37°C for 5hrs.<br>2. 5 µl RQ1 RNase-free DNase (1 U/µl) (Promega) was added per 100 µl reaction volume, mixed well and incubated for another 20 mins at 37°C.<br>3. Mixture was stored at -20°C overnight. | |

mRNA Isolation

mRNA was isolated using MEGAclear KIT from Ambion.

If sample volume was less than 100 µl, sample was brought to 100 µl with Elution Solution and mixed gently.

1. 350 µl of Binding Solution was added per 100 µl sample and mixed gently.
2. 250 µl 100 % ethanol was added per 100 µl sample and mixed gently.
3. Sample was applied to the filter:

    a) A Filter Cartridge was inserted into a Collection and Elution Tube.
    b) The RNA mixture was applied to the Filter Cartridge.
    c) The Filter Cartridge was centrifuged at 10,000-15,000 x g for 1 min.
    d) The flow-through was discarded.

4. The Filter Cartridge was washed with 3 x 500 µl Wash Solution.

    a. 500 µl Wash Solution was applied to the Filter Cartridge. This was then centrifuged at 15,000 x g for 1 min and the flow-through discarded.
    b. Step ’a’ was repeated twice.
    c. A final centrifugation step was performed to remove the last traces of Wash Solution (1 min for 15,000 x *g*).

5. RNA was eluted from the filter with 50 µl Elution Solution by centrifugation.

T-cell electroporation was carried out as in Wälchli *et al,* 2011.

RESULTS

Retroviral particles were prepared and the constructs were expressed in NK-92 cells (a commonly used cell line for CAR study) or in T-cells isolated from peripheral blood from a healthy donor. Expression was analysed by staining the cells with a specific antibody against a domain present in the three constructs (anti-Hinge) followed by flow cytometry. As shown in Figure 2, in both cell types, the CARs were well expressed, suggesting that our scFvs were well folded and could generate membrane proteins that were expressed in lymphocytes.

The ability of these CARs to redirect T-cells to specifically target NHL B-cell lines was further tested. Activation was monitored by cell surface expression of CD107a, a degranulation marker which is present only upon killer cell activation. When cells are activated, CD107a can be detected by flow cytometry, whereas in steady-state, its presence is not visible. Redirected T-cells were tested against the NHL cell line Mino (CD37/CD19 positive) and the non-B-cell line HEK (CD37/CD19 negative) either expressing CD37 cDNA or not. This control was performed to verify the specificity of the anti-CD37 CAR. As shown in Figure 3, only cells expressing CD37 endogenously (Mino) or artificially (transfected HEK cells) could stimulate anti-CD37 redirected T-cells, suggesting that stimulation was CD37-specific. When T-cells expressed anti-CD19 CAR, only Mino cells were detected, but not HEK cells. Taken together, the present data validate the use of our novel approach, using the antigen-binding parts of our in-house anti-CD37 antibody to construct efficient CARs. This is to our knowledge the first example of a functional anti-CD37 CAR.

## Example 2

mRNA Transduction of CD37 CAR:

Construct codes:

| Table 6 | |
|---|---|
| Construct | CAR |
| 755 | aCD19 FM63 (CD8 hinge) |
| 756 | aCD19 IKF (CD8 hinge) |
| 757 | aCD37 (CD8 hinge) |
| 758 | aCD19 FM63 (Ab hinge) |
| 759 | aCD19 IKF (Ab hinge) |
| 760 | aCD37 (Ab hinge) |

CAR Expression 12 hours After Electroporation:

Testing cells for Fab expression:

200 μl isolated T-cells were washed once, resuspended in 10 μl anti-Fab antibody (Goat F(ab')2 Anti-Mouse IgG (Fab')2 (Biotin), Abcam 98657) and incubated for 20 min at RT. They were then washed once more. Added 5 μl Streptavidin APC in Flow buffer, incubated for 10 min at RT. Cells were washed a final time, then resuspended in 180 μl Flow Buffer and expression analysed by flow cytometry. Results are shown in Figure 4B. Expression of 755 and 758 should not be visible because the FM63 is humanised. Only the CD8 hinge constructs (756 (CD19IKF CAR) and 757 (CD37 CAR)) were visible, indicating they are probably more stable.

Functional assay:

EFFECTOR: CAR transfected T-cells.

TARGETS: Mino (CD37 and CD19 positive);
K562 (CD37 and CD19 negative).

1. Cells were centrifuged and their supernatant removed.
2. 200 μl serum free CellGro® with 5 μl CD107a was added to each well, with 2 μl 1:10 dilution of GolgiPlug™ and GolgiStop™.
3. Cells were incubated for 5 hrs
4. Responses of both CD4+ and CD8+ T-cells were monitored.

Staining for Flow Cytometry

1. Plate was spun at 350 x g for 5 mins.
2. Supernatant was removed.
3. Cells were washed once with 150 μl Flow Buffer (2 % FCS, 1 mM EDTA in PBS).
4. Cells were resuspended in 10 μl Ab master mix: 4 μl anti-CD8 PE-Cy7 and 4 μl anti-CD4 PE + 2 μl Flow Buffer.
5. Cells were incubated at RT for 15 min.
6. Cells were washed once with 150 μl Flow Buffer.
7. Cells were incubated at RT for 10 min.
8. Plate was centrifuged at 350 x *g* for 5 min.
9. Supernatant was discarded and cells were resuspended in 180 μl Flow Buffer.

Proportions of CD107a+ cells are shown in Figure 5. T-cells obtained from 2 different donors were tested. Donor variation is apparent: CD4+ cells from Donor 1 were not redirected, whereas those from Donor 2 gave a decent response.

CD37 CARs (757 and 760, CD8 and Ab hinge, respectively) only work in the CD8 hinge format; this is not the case for CD19 CARs (both FM63 and IKF). This may be due to differences in the targets' positions. CD37 CAR with Ab hinge worked in the retroviral format, so this is probably a dose effect.

**Example 3**

Killing assay using Europium and monitoring of BL41 presence by flow cytometry:

The Europium killing assay is performed by loading target cells with BATDA (an acetoxymethyl ester of a fluorescence enhancing ligand: bis (acetoxymethyl) 2,2':6',2"-terpyridine-6,6"-dicarboxylate). Hydrophobic BATDA quickly penetrates the cell membrane. Within the cell, the ester bonds are hydrolysed by acetyl esterases to form the hydrophilic ligand TDA (2,2':6',2"-terpyridine-6,6"-dicarboxylic acid) which cannot pass through the membrane. After cytolysis the TDA ligand is released from the cells into a Europium solution. The Eu forms a highly fluorescent and stable chelate with the TDA ligand (EuTDA). The fluorescent signal is measured, its intensity correlating directly with the number of lysed cells.

MATERIALS AND METHODS

Reagents:

| Name | Product Nr. | Lot # |
|---|---|---|
| RPMI phenol red free | Life Technologies #11835-063 | 1723502 |
| BATDA | Perkin Elmer #C136-100 | 2093024 |
| Europium solution | Perkin Elmer #C135-100 | 642061 |
| Triton-x 100 | Sigma #T8787 | |

Protocol:

All reagents were stored at 4°C but brought to room temperature before use.

*Cell Loading*

- Target cells: BL41 (a CD19+/CD37+ B-cell line)
- Target cells per well: 7500

1. Target cells were washed once in phenol red-free RPMI medium.
2. The concentration of target cells was adjusted to 2 x $10^6$/ml.
3. 2 $\mu$l of BATDA reagent was added and the cells then incubated at 37°C for 15 mins.
4. Cells were washed twice with 5 ml PBS.
5. After the final wash, cells were resuspended to 7.5 x $10^4$/ml.

The loaded target cells must not be incubated or left standing at this point. It is necessary to proceed immediately to the next step in the assay.

*Preparation of Effector Cells*

- Effector cells: NK-92 stably expressing anti-CD19-(IKF)CAR or anti-CD37-(HH1)CAR. Untransduced NK-92 cells were used as a control
- Effector:Target (E:T) ratios used: 10:1 (75 000:7500), 1:1 (7500:7500)

1. NK-92 cells were harvested and counted.
2. NK-92 cells were resuspended to desired concentration: 7.5 x $10^5$/ml or 7.5 x $10^4$/ml.

*Assay*

1. 100 $\mu$l of loaded target cells (7500 cells) was pipetted into a round-bottomed sterile plate.
2. 100 $\mu$l effector cells (7500 or 75 000 cells) was added.
3. Control wells were set up for detection of background, spontaneous release and maximum release (see definitions below).
4. Plate was incubated at 37°C for 2 hours.
5. Plate was centrifuged for 5 min at 500 x *g*.
6. 50 $\mu$l of the supernatant was transferred to a flat-bottomed white plate.
7. 200 $\mu$l of the Europium Solution was added.
8. Mixture was shaken at 250 rpm for 15 min at RT.
9. Fluorescence was measured using a VICTOR microplate fluorometer with the Europium programme.
10. Specific release was calculated using the formula below:

$$\frac{\text{Experimental release (counts)} - \text{Spontaneous release (counts)}}{\text{Maximum release (counts)} - \text{Spontaneous release (counts)}} \times 100$$

Background = medium without cells: one aliquot of the loaded target cells was taken immediately after dilution in culture medium and not incubated. The cells were centrifuged and 100 $\mu$l of the supernatant was pipetted into the wells, and 100 $\mu$l of cell culture medium was added.

Spontaneous release = target cells without effector cells: 100 $\mu$l target cells were incubated with 100 $\mu$l cell culture medium instead of effector cells during the assay.

Maximum release = lysed target cells: 100 $\mu$l target cells was incubated with 100 $\mu$L of cell culture medium supplemented with 1 % Triton.

RESULTS

The killing assay was run as indicated using NK-92 cells transduced with the indicated CAR constructs. Non-transduced (NT) NK-92 cells were used as a control. As shown in Figure 6A, the killing of BL41 cells (a Burkitt's lymphoma β-cell line) was specific and dependent on the expression of the CAR constructs. Since the Europium assay is a short-term procedure (2 hours), the presence of target cells after 24 and 48 hours was also monitored by direct flow cytometry (detection of specific B-cell markers and exclusion of NK-92 by NK cell markers). As shown in Figure 6B, although NK-92 cells are naturally able to kill BL41 cells (as shown by the reduction in BL41 cell number when exposed to NT NK-92 cells), killing of BL41 cells is significantly enhanced by expression of the CARs.

Luciferase-Based Killing Assay:

MATERIALS AND METHODS

*mRNA Transfection of T-cells*

Before transfection, three T25 flasks were prepared with 15 ml complete CellGro DC medium (including 5 % Human Serum, Gentamycin, 0.01 M HEPES, 1.25 mg/ml N-acetyl-cysteine (Mucomyst)) plus 100 U/ml IL-2, and placed in a $CO_2$ incubator at 37°C. Transfected cells were transferred directly into these flasks immediately after electroporation.
1. One frozen vial of T-cells (previously expanded from PBMCs) was thawed.
2. T-cells were incubated for 10 min at RT in a 50 ml tube with 20 ml complete CellGro DC medium plus 100 U/ml IL-2 and 20 μg/ml DNase I.
3. T-cells were centrifuged at 300 x g for 10 min. The pellet was resuspended in 10 ml CellGro DC medium plus 100 U/ml IL-2 and transferred into a T25 flask
4. The T-cells were incubated for 2 hours in a $CO_2$ incubator at 37°C
5. The T-cells were transferred into a 50 ml tube and washed twice with CellGro DC medium (no supplements)
6. The viable cells were counted
7. The cell concentration was adjusted to 10 x $10^6$ cells in 144 μl CellGro DC medium (no supplements) for each transfection (leave the cells on ice)
8. 16 μg mRNA was pipetted into an Eppendorf tube and the total volume made up to 16 μl.

| RNA | RNA conc. | RNA Volume (16 μg total) | Water Added | Total Volume |
|---|---|---|---|---|
| Mock | - | - | 16 μl | 16 μl |
| Construct 755 (CD19) | 1.2 μg/μl | 13.3 μl | 2.7 μl | 16 μl |
| Construct 757 (CD37) | 2.2 μg/μl | 7.3 μl | 8.7 μl | 16 μl |

A 144 μl T-cell resuspension was added to each Eppendorf tube containing an RNA sample
9. Each cell/DNA mix was carefully transferred into an electroporation cuvette without introducing bubbles and making sure that the cells deposited across the bottom of the cuvette. Electroporation was performed using a BTX ECM 630 electroporator with the following conditions: 500 V and time constant of 2 milliseconds. One cuvette was electroporated at a time; each cuvette was washed with 800 μl medium after electroporation.
10. The electroporated cells were transferred with a pipette into the T25 flasks and incubated at 37°C for translation of the transfected mRNAs.

*Assay*

1. Target cells were washed once with assay medium.
2. The concentration of target cells was adjusted to about 3 x $10^5$ cells/ml with culture medium. 75 μg/ml D-firefly luciferin potassium salt was added and the cells immediately seeded into the 96-well plate (100 μl each well).
3. The effector cells were washed once with assay medium.
4. The concentration of effector cells was adjusted to about 3 x $10^6$ cells/ml with culture medium and 100 μl was added to each well.
5. Control wells were set up for detection of background, spontaneous release and maximum release (as defined above).
6. The cells were incubated for 2, 4, 6, 8, 10 or 24 hours in a humidified 5 % $CO_2$ atmosphere at 37°C.
7. Bioluminescence (as relative light units (RLU)) was measured with a luminator.

8. Specific lysis was calculated using the formula below:

$$\frac{\text{Experimental death RLU (average) – Spontaneous death RLU (average)}}{\text{Maximum death RLU (average) – Spontaneous death RLU (average)}} \times 100$$

RESULTS

The expression of CD19 and CD37 in different β-cell lines was monitored by flow cytometry (Figure 7A). U2932 cells were identified as most highly expressing CD37. T-cells were mock electroporated or electroporated with mRNA encoding anti-CD19-(fmc63)CAR or anti-CD37-(HH1)CAR and incubated for 5 hours with U2932 cells. Cytokine production was detected by flow cytometry (Figure 7B). All cell lines tested in Figure 7A were then used in the luciferase-based killing assay. To this end, target cells were retrovirally transduced to stably express luciferase gene and the killing assay performed as described above. The results are shown in Figure 7C. As can be seen, all β-cell lines were successfully killed by T-cells expressing the CARs. Significant killing by the control, non-transduced T-cells was seen only with the MINO cell line showing that CAR expression enables killing of the β-cell lines by the effector T-cells. It is notable that the greatest difference in killing efficacy between the T-cells expressing the anti-CD27 CAR and the anti-CD19 CAR was seen with the U2932 cells, which cell line demonstrated highest expression of CD37 and lowest expression of CD19 of all those tested.

## Claims

1. A nucleic acid molecule encoding a chimeric antigen receptor (CAR) directed against the antigen CD37, wherein said CAR when expressed on the surface of an immune effector cell is capable of binding to the antigen CD37 expressed on a target cell surface and comprises an antigen-binding domain comprising a $V_L$ sequence and a $V_H$ sequence each comprising three CDR sequences, wherein

   a) CDRs 1, 2 and 3 of the $V_L$ sequence have the sequences of SEQ ID NOs. 43, 44 and 35 respectively; and
   b) CDRs 1, 2 and 3 of the $V_H$ sequence have the sequences of SEQ ID NOs: 45, 46 and 34 respectively, and

   wherein one or more of said CDR sequences may optionally be modified by substitution, addition or deletion of 1 to 3 amino acids;
   optionally wherein the nucleic acid molecule is RNA.

2. The nucleic acid molecule of claim 1, wherein:

   a) CDRs 1, 2 and 3 of the $V_L$ sequence have the sequences of SEQ ID NOs. 43, 44 and 35 respectively; and
   b) CDRs 1, 2 and 3 of the $V_H$ sequence have the sequences of SEQ ID NOs: 45, 46 and 34 respectively.

3. The nucleic acid molecule of claim 1 or claim 2, wherein the $V_L$ sequence has an amino acid sequence as shown in SEQ ID NO. 3 or an amino acid sequence having at least 60 % sequence identity thereto; and/or
   the $V_H$ sequence has an amino acid sequence as shown in SEQ ID NO. 1 or an amino acid sequence having at least 60 % sequence identity thereto.

4. The nucleic acid molecule of any one of claims 1 to 3, wherein the framework regions of said $V_L$ and $V_H$ sequences are humanised;

optionally wherein the $V_L$ sequence has an amino acid sequence as shown in SEQ ID NO. 47 or an amino acid sequence having at least 95 % sequence identity thereto, and the $V_H$ sequence has an amino acid sequence as shown in SEQ ID NO. 48 or an amino acid sequence having at least 95% sequence identity thereto.

**5.** The nucleic acid molecule of any one of claims 1 to 4, wherein:

(i) the antigen-binding domain is a scFv comprising the $V_L$ and $V_H$ sequences joined by a linker, optionally wherein the linker sequence is $(G4S)_4$ (SEQ ID NO. 5); and/or
(ii) the CAR comprises a plasma membrane targeting sequence;

optionally wherein the plasma membrane targeting sequence is positioned upstream of the antigen-binding domain or scFv and/or is the L-chain having the sequence set out in SEQ ID NO. 6 or an amino acid sequence having at least 95 % sequence identity thereto.

**6.** The nucleic acid molecule of any one of claims 1 to 5, wherein the CAR comprises, downstream of an extracellular domain comprising the antigen-binding domain, a hinge domain, a transmembrane domain, an intracellular signalling domain, and optionally one or more co-stimulatory signalling domains;
optionally wherein the hinge domain is attached to the transmembrane domain by means of a linker, preferably wherein the linker has the sequence KDPK (SEQ ID NO. 11).

**7.** The nucleic acid molecule of claim 6, wherein:

(i) the intracellular signalling domain of the CAR is a CD3ζ or FcRγ intracellular signalling domain, preferably a human CD3ζ domain, more preferably a human CD3ζ domain having the amino acid sequence of SEQ ID NO.8 or an amino acid sequence having at least 95 % sequence identity thereto; and/or
(ii) the hinge domain is derived from CD8α, CD4, CD28, or CD7, preferably human CD8α, CD4, CD28, or CD7, or from the Fc of an immunoglobulin, for example IgG, preferably wherein the Fc-derived hinge domain does not comprise a CH3 domain, e.g. comprises or consists of the CH2 domain or a part thereof; and/or
(iii) the transmembrane domain of the CAR is a transmembrane domain derived from CD8α, CD3ζ, CD28, CD4, CD45, CD9, CD16, CD22, CD33, CD64, CD80, CD86, CD134, CD137, or CD154, preferably human CD8α, CD3ζ, CD28, or CD4; and/or
(iv) the co-stimulatory domain is derived from any one or more of 4-1BB (DAP10/CD137), CD28, OX-40 (CD134), CD278 (ICOS), CD27, CD30, CD40, PD-1, LFA-1, CD2, CD7, LIGHT, NKD2C, BH-H2 and a ligand which specifically binds CD83.

**8.** The nucleic acid molecule of claim 7, wherein:

(a) the hinge domain is:

(i) the hinge domain of CD8α having the amino acid sequence of SEQ ID NO. 9 or an amino acid sequence having at least 95 % sequence identity thereto; or
(ii) an IgG hinge domain having the amino acid sequence of SEQ ID NO. 10 or an amino acid sequence having at least 95 % sequence identity thereto; and/or

(b) the transmembrane domain is the CD8α transmembrane domain having the amino acid sequence of SEQ ID NO. 12 or an amino acid sequence having at least 95 % sequence identity thereto; and/or
(c) the co-stimulatory domain is the intracellular domain of 4-1BB having the amino acid sequence of SEQ ID NO. 13 or an amino acid sequence having at least 95 % sequence identity thereto.

**9.** The nucleic acid molecule of any one of claims 1 to 8, wherein the CAR comprises, preferably in the following order:

(i) a hinge domain being (a) the hinge domain of CD8α having the amino acid sequence of SEQ ID NO. 9 or an amino acid sequence having at least 95 % sequence identity thereto; or (b) an IgG hinge domain having the amino acid sequence of SEQ ID NO. 10 or an amino acid sequence having at least 95 % sequence identity thereto;
(ii) a CD8α transmembrane domain having the amino acid sequence of SEQ ID NO. 12 or an amino acid sequence having at least 95 % sequence identity thereto;
(iii) a co-stimulatory domain being the intracellular domain of 4-1BB having the amino acid sequence of SEQ ID NO. 13 or an amino acid sequence having at least 95 % sequence identity thereto; and

(iv) a human CD3ζ domain having the amino acid sequence of SEQ ID NO. 8 or an amino acid sequence having at least 95 % sequence identity thereto.

**10.** A vector comprising the nucleic acid molecule of any one of claims 1 to 9, optionally wherein said vector is:

(i) an expression vector, preferably an mRNA expression vector;
(ii) a cloning vector; and/or
(iii) a viral vector, preferably a retroviral or lentiviral vector.

**11.** An immune effector cell comprising the nucleic acid molecule of any one of claims 1 to 9 or the vector of claim 10, optionally wherein the cell is a T-cell or an NK cell.

**12.** A composition comprising the immune effector cell of claim 11 and at least one physiologically acceptable carrier or excipient.

**13.** An immune effector cell as defined in claim 11, or a composition as defined in claim 12, for use in therapy or for use in adoptive cell transfer therapy.

**14.** An immune effector cell as defined in claim 11 or a composition as defined in claim 12, for use in the treatment of cancer, optionally wherein the cancer is a B-cell malignancy.

**15.** A method of generating a CD37-specific immune effector cell, said method comprising introducing a nucleic acid molecule of any one of claims 1 to 9 or a vector of claim 10 into an immune effector cell, preferably a T-cell or an NK cell; optionally wherein the method further comprises stimulating the cells and inducing them to proliferate before and/or after introducing the nucleic acid molecule or vector.

Figure 1:

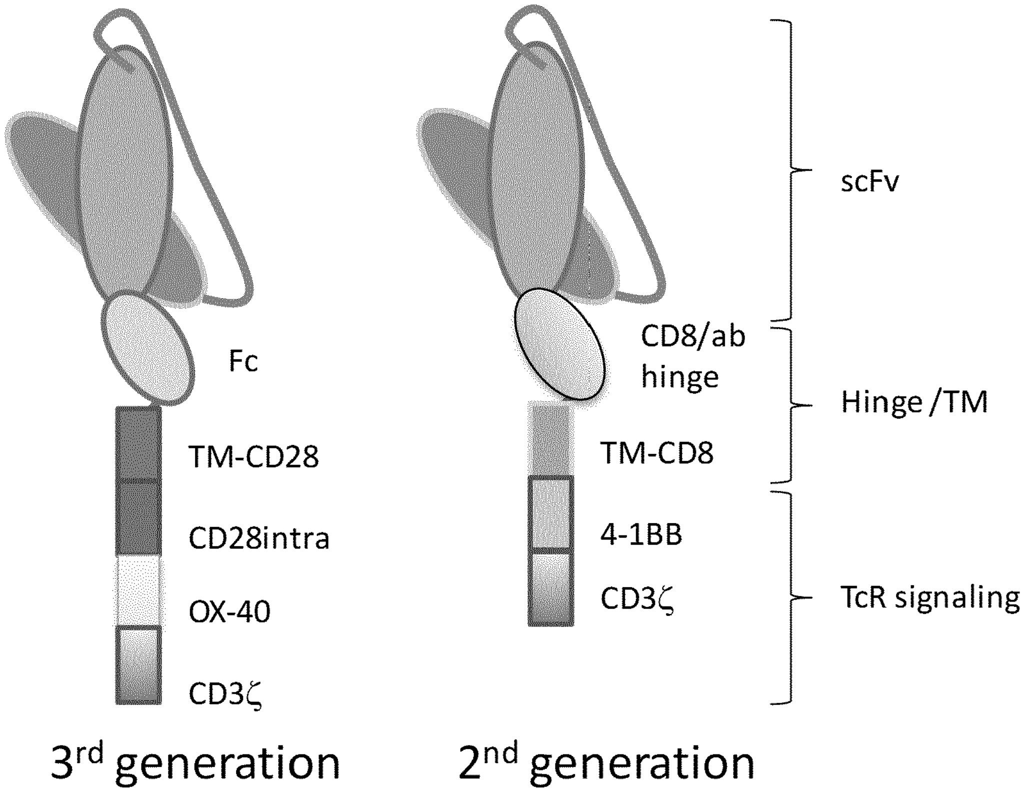


Figure 2:

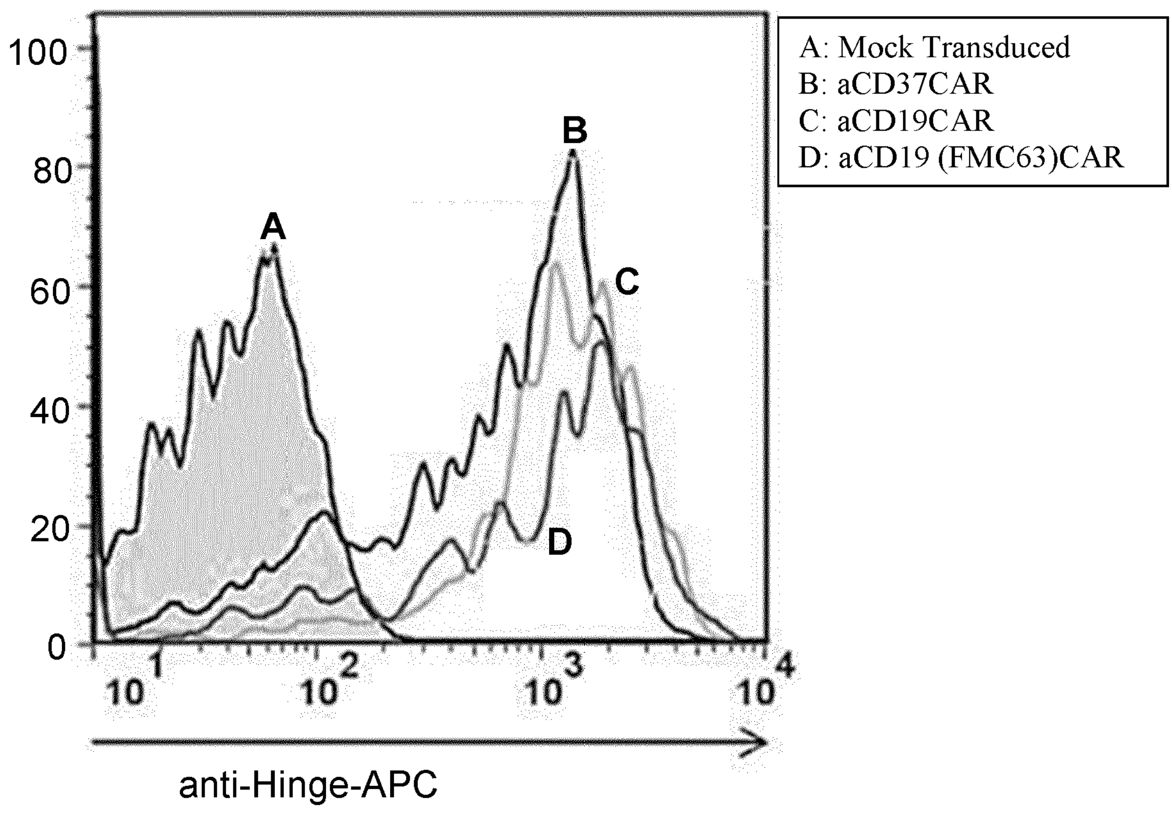

Figure 3:

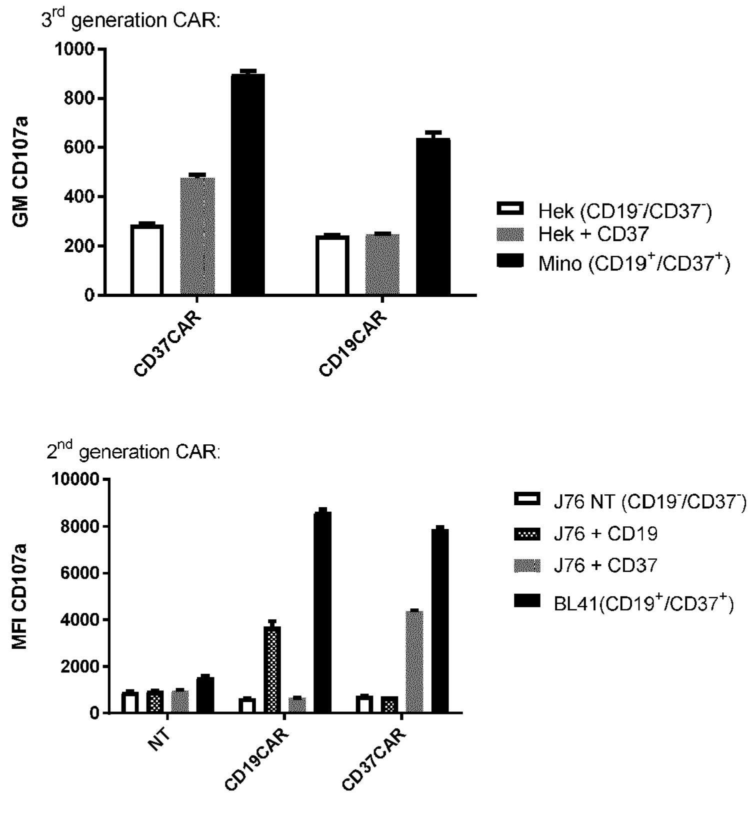
3rd generation CAR:
GM CD107a
1000
800
600
400
200
0
CD37CAR
CD19CAR
Hek (CD19-/CD37-)
Hek + CD37
Mino (CD19+/CD37+)
2nd generation CAR:
MFI CD107a
10000
8000
6000
4000
2000
0
NT
CD19CAR
CD37CAR
J76 NT (CD19-/CD37-)
J76 + CD19
J76 + CD37
BL41(CD19+/CD37+)

Figure 4:

A)

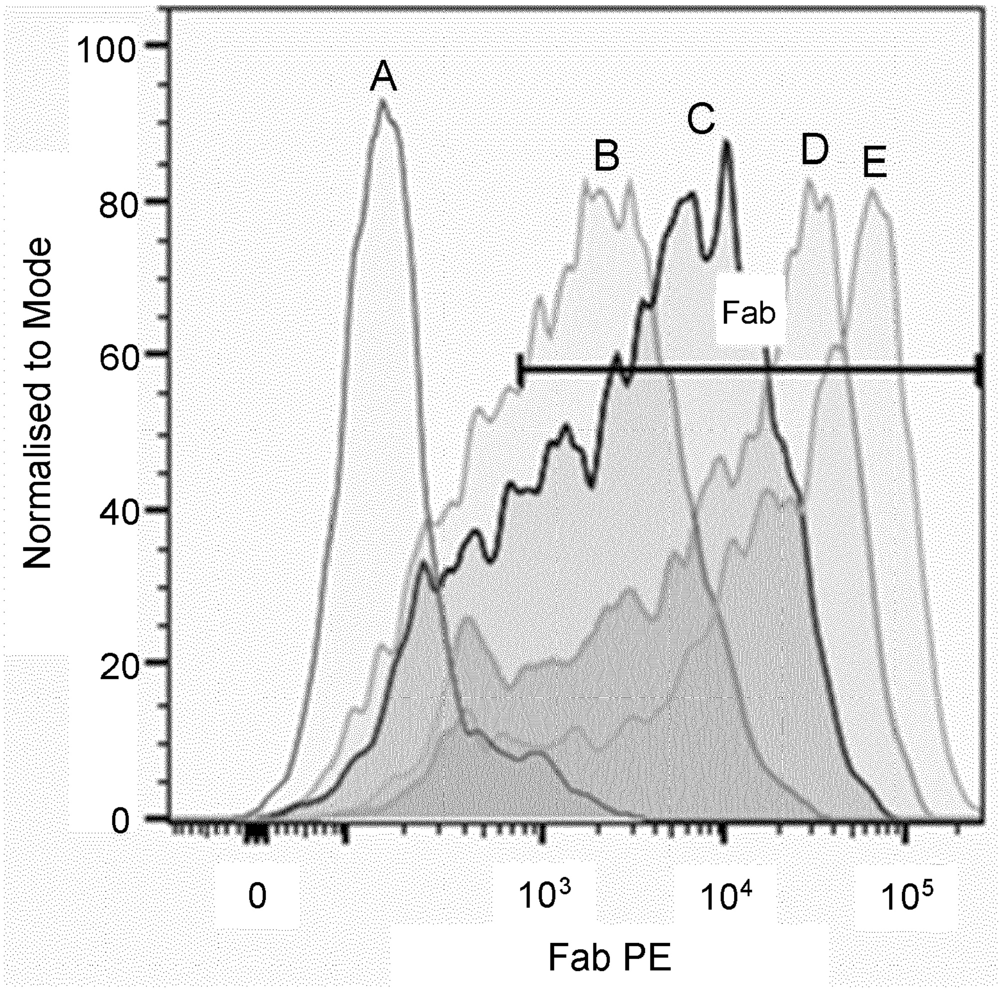


A: Not transduced
B: CD37 CAR Ab Hinge
C: CD19 CAR Ab Hinge
D: CD37CAR CD8 Hinge
E: CD37CAR 3rd Generation

Figure 4:

B)

Donor 1:

| | | | | | | |
|---|---|---|---|---|---|---|
| Mock/NT | 755 | 756 | 757 | 758 | 759 | 760 |

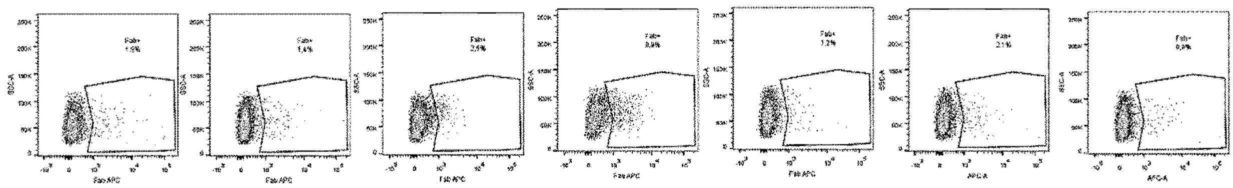

Donor 2

| | | | | | | |
|---|---|---|---|---|---|---|
| Mock/NT | 755 | 756 | 757 | 758 | 759 | 760 |

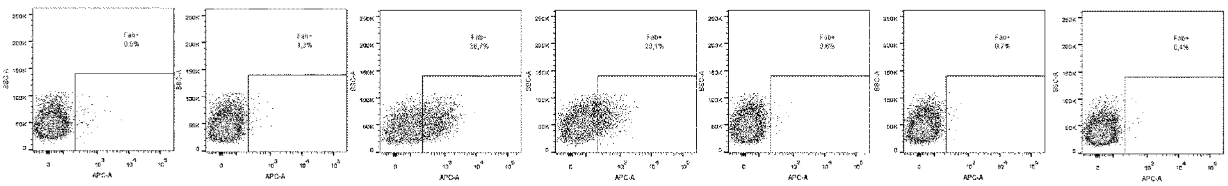

Figure 5:

Donor 1:

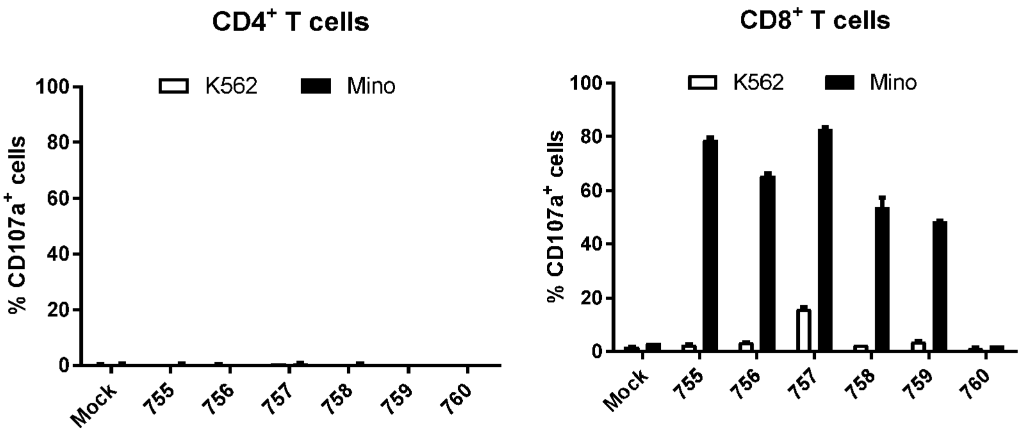


Donor 2:

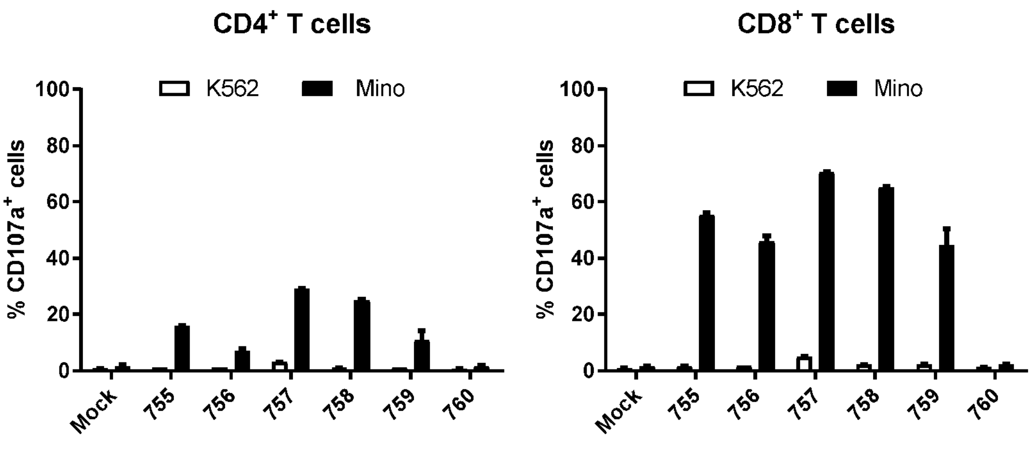

Figure 6
(A)

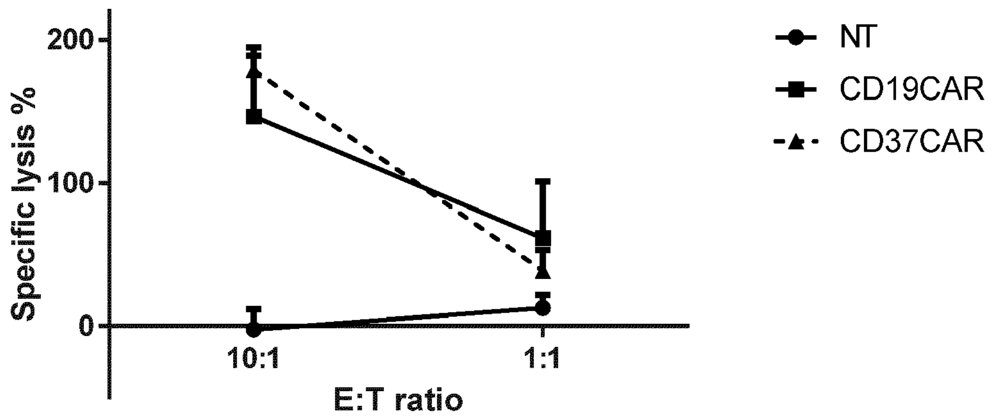
Specific lysis %
200
100
0
10:1
1:1
E:T ratio
NT
CD19CAR
CD37CAR

(B)

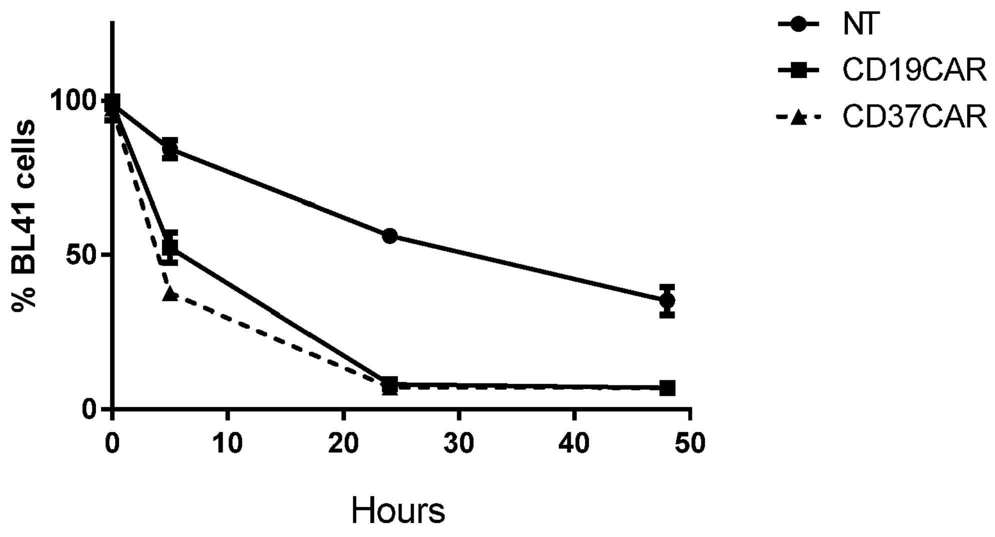
% BL41 cells
100
50
0
0
10
20
30
40
50
Hours
NT
CD19CAR
CD37CAR

Figure 7

(A)

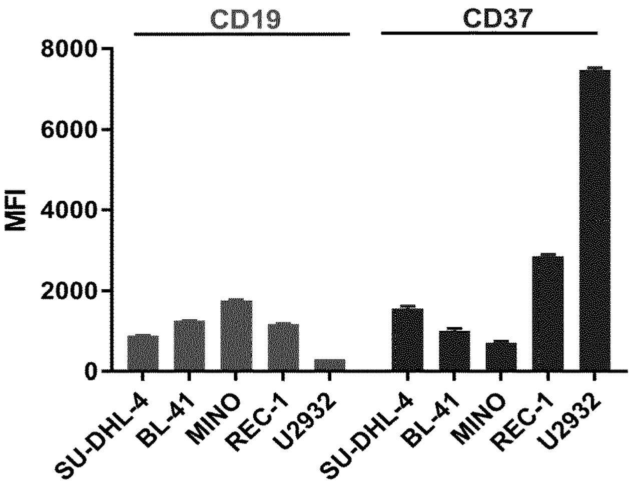
CD19
CD37
MFI
8000
6000
4000
2000
0
SU-DHL-4
BL-41
MINO
REC-1
U2932
SU-DHL-4
BL-41
MINO
REC-1
U2932

(B)

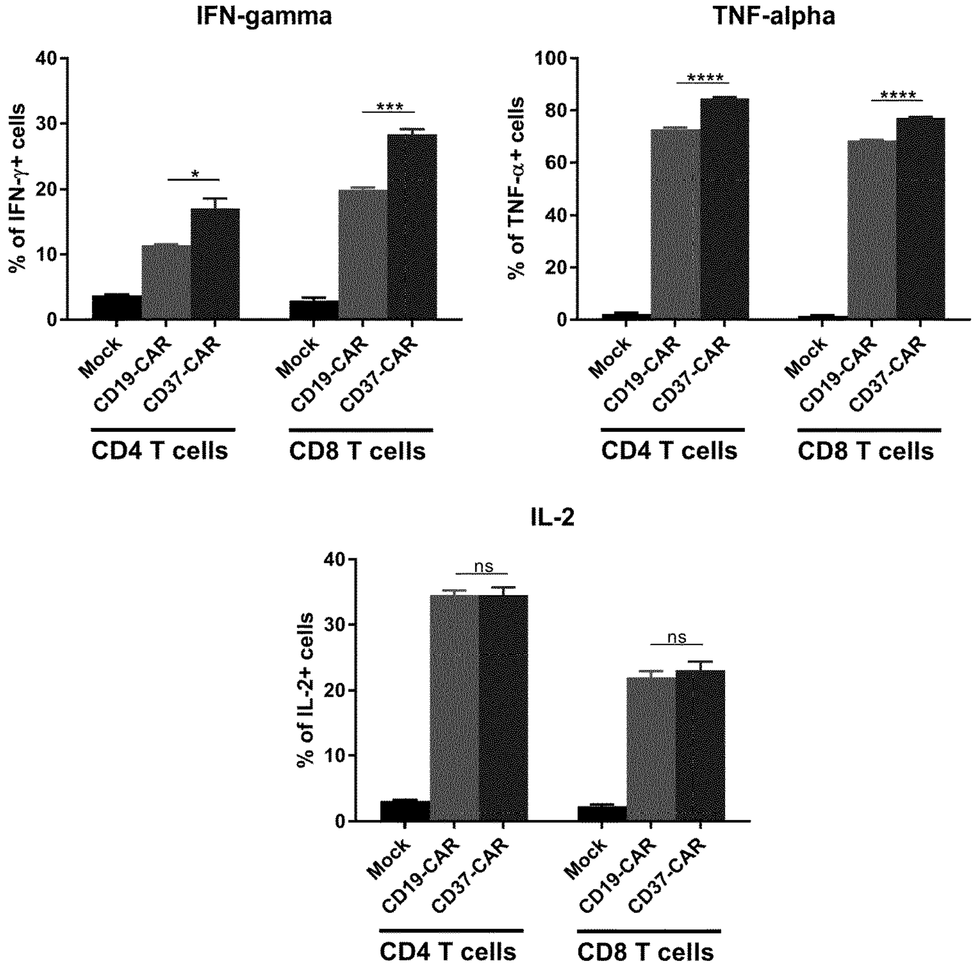
IFN-gamma
% of IFN-γ+ cells
40
30
20
10
0
*
***
Mock
CD19-CAR
CD37-CAR
CD4 T cells
CD8 T cells
TNF-alpha
% of TNF-α+ cells
100
80
60
40
20
0
****
****
IL-2
% of IL-2+ cells
ns
ns

Figure 7 C

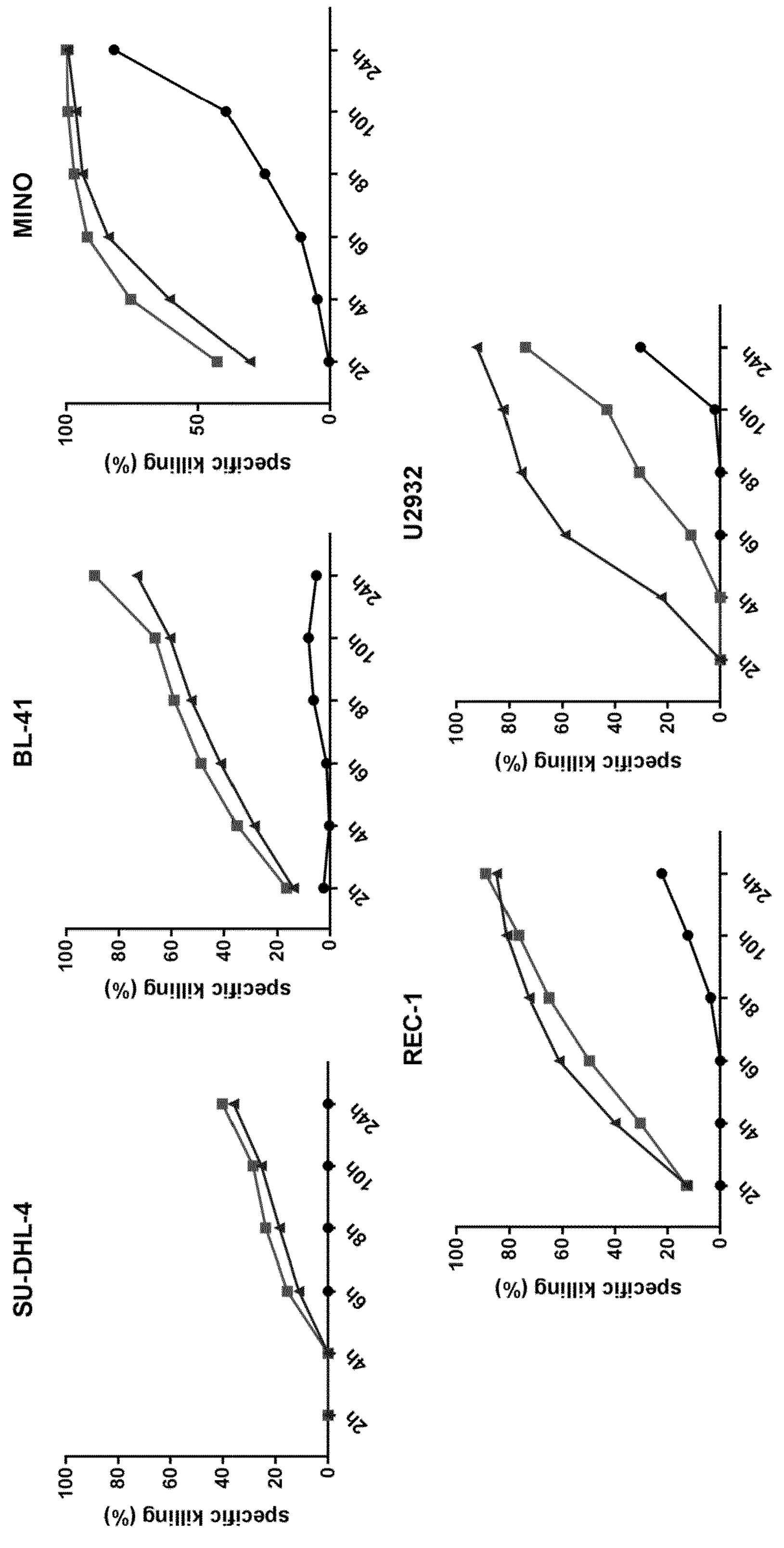
MINO
BL-41
SU-DHL-4
U2932
REC-1
specific killing (%)
2h
4h
6h
8h
10h
24h

Europäisches Patentamt
European Patent Office
Office européen des brevets

# EUROPEAN SEARCH REPORT

Application Number
EP 21 15 9557

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2014/099671 A1 (BLUEBIRD BIO INC [US]) 26 June 2014 (2014-06-26)<br>* tables 3-5 *<br>* figure 1 *<br>* examples 1, 2 *<br>* claim 26 *<br>----- | 1-15 | INV.<br>C07K16/28<br>C07K14/705<br>C07K14/725<br>A61K39/395<br>C12N5/22<br>C07K19/00<br>A61P35/00 |
| Y | WO 2011/092295 A2 (NORDIC NANOVECTOR AS [NO]; LARSEN ROY H [NO]; DAHLE JOSTEIN [NO]; BRUL) 4 August 2011 (2011-08-04)<br>* sequences 2, 4 *<br>----- | 1-15 | |
| Y | WO 2013/088363 A1 (NORDIC NANOVECTOR AS [NO]) 20 June 2013 (2013-06-20)<br>* sequences 1, 3 *<br>----- | 1-15 | |
| A | WO 2013/049666 A1 (SARCOTEIN DIAGNOSTICS LLC [US]) 4 April 2013 (2013-04-04)<br>* sequence 8 *<br>----- | 1-15 | TECHNICAL FIELDS SEARCHED (IPC) |
| A | A. HONEGGER ET AL: "The influence of the framework core residues on the biophysical properties of immunoglobulin heavy chain variable domains",<br>PROTEIN ENGINEERING DESIGN AND SELECTION, vol. 22, no. 3,<br>10 January 2009 (2009-01-10), pages 121-134, XP055000564,<br>ISSN: 1741-0126, DOI: 10.1093/protein/gzn077<br>* abstract *<br>-----<br>-/-- | 1-15 | C07K<br>A61K |

The present search report has been drawn up for all claims

1

| Place of search | Date of completion of the search | Examiner |
|---|---|---|
| The Hague | 17 June 2021 | Brouns, Gaby |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 15 9557

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MABRY ROBERT ET AL: "Therapeutic bispecific antibodies: The selection of stable single-chain fragments to overcome engineering obstacles", I DRUGS: THE INVESTIGATIONAL DRUGS JOURNAL, CURRENT DRUGS LTD, GB, vol. 13, no. 8, 1 August 2010 (2010-08-01), pages 543-549, XP009144921, ISSN: 2040-3410 [retrieved on 2010-07-27] ----- | 1-15 | |
| A | IGNAZIO CARUANA ET AL: "From Monoclonal Antibodies to Chimeric Antigen Receptors for the Treatment of Human Malignancies", SEMINARS IN ONCOLOGY, vol. 41, no. 5, 1 October 2014 (2014-10-01), pages 661-666, XP055362209, US ISSN: 0093-7754, DOI: 10.1053/j.seminoncol.2014.08.005 ----- | 1-15 | TECHNICAL FIELDS SEARCHED (IPC) |
| A | GILL SAAR ET AL: "Chimeric antigen receptor T cell therapy: 25years in the making", BLOOD REVIEWS, CHURCHILL LIVINGSTONE, AMSTERDAM, NL, vol. 30, no. 3, 6 November 2015 (2015-11-06), pages 157-167, XP029554913, ISSN: 0268-960X, DOI: 10.1016/J.BLRE.2015.10.003 ----- | 1-15 | |

The present search report has been drawn up for all claims

1

| Place of search | Date of completion of the search | Examiner |
|---|---|---|
| The Hague | 17 June 2021 | Brouns, Gaby |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 15 9557

This annex lists the patent family members relating to the patent documents cited in the above-mentioned European search report. The members are as contained in the European Patent Office EDP file on
The European Patent Office is in no way liable for these particulars which are merely given for the purpose of information.

17-06-2021

| Patent document cited in search report | Publication date | Patent family member(s) | Publication date |
|---|---|---|---|
| WO 2014099671 A1 | 26-06-2014 | US 2015329640 A1 | 19-11-2015 |
| | | WO 2014099671 A1 | 26-06-2014 |
| WO 2011092295 A2 | 04-08-2011 | AU 2011209441 A1 | 19-07-2012 |
| | | BR 112012018843 A2 | 17-10-2017 |
| | | CA 2786655 A1 | 04-08-2011 |
| | | CN 102762230 A | 31-10-2012 |
| | | CN 104338154 A | 11-02-2015 |
| | | DK 2528627 T3 | 12-05-2014 |
| | | DK 2705857 T3 | 29-08-2016 |
| | | EP 2528627 A2 | 05-12-2012 |
| | | EP 2705857 A2 | 12-03-2014 |
| | | ES 2469140 T3 | 17-06-2014 |
| | | ES 2592402 T3 | 30-11-2016 |
| | | HK 1179157 A1 | 27-09-2013 |
| | | HK 1195736 A1 | 21-11-2014 |
| | | HR P20140538 T1 | 18-07-2014 |
| | | IL 221091 A | 31-03-2015 |
| | | JP 5646652 B2 | 24-12-2014 |
| | | JP 5855209 B2 | 09-02-2016 |
| | | JP 2013518086 A | 20-05-2013 |
| | | JP 2015057415 A | 26-03-2015 |
| | | KR 20130028051 A | 18-03-2013 |
| | | KR 20170084349 A | 19-07-2017 |
| | | MX 342539 B | 04-10-2016 |
| | | NO 331080 B1 | 26-09-2011 |
| | | NZ 601055 A | 20-12-2013 |
| | | PH 12017501038 A1 | 11-12-2017 |
| | | PL 2528627 T3 | 29-08-2014 |
| | | PT 2528627 E | 12-06-2014 |
| | | RS 53346 B | 31-10-2014 |
| | | RU 2664475 C1 | 17-08-2018 |
| | | RU 2012135430 A | 10-03-2014 |
| | | SG 182685 A1 | 30-08-2012 |
| | | SI 2528627 T1 | 29-08-2014 |
| | | UA 108631 C2 | 25-05-2015 |
| | | US 2012301396 A1 | 29-11-2012 |
| | | US 2014147384 A1 | 29-05-2014 |
| | | WO 2011092295 A2 | 04-08-2011 |
| | | ZA 201205007 B | 25-09-2013 |
| WO 2013088363 A1 | 20-06-2013 | AU 2012354140 A1 | 03-07-2014 |
| | | BR 112014014258 A2 | 27-10-2020 |
| | | CA 2858964 A1 | 20-06-2013 |
| | | CN 104114192 A | 22-10-2014 |
| | | CN 109276713 A | 29-01-2019 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 21 15 9557

This annex lists the patent family members relating to the patent documents cited in the above-mentioned European search report. The members are as contained in the European Patent Office EDP file on
The European Patent Office is in no way liable for these particulars which are merely given for the purpose of information.

17-06-2021

| Patent document cited in search report | Publication date | Patent family member(s) | Publication date |
|---|---|---|---|
| | | EP 2790740 A1 | 22-10-2014 |
| | | EP 3272364 A1 | 24-01-2018 |
| | | ES 2827787 T3 | 24-05-2021 |
| | | HK 1203372 A1 | 30-10-2015 |
| | | IL 233084 A | 29-06-2017 |
| | | JP 2015501654 A | 19-01-2015 |
| | | KR 20140103140 A | 25-08-2014 |
| | | MX 358502 B | 23-08-2018 |
| | | NZ 626188 A | 23-12-2016 |
| | | PH 12014501338 A1 | 15-09-2014 |
| | | RU 2014125320 A | 10-02-2016 |
| | | SG 11201403134P A | 30-07-2014 |
| | | US 2014348745 A1 | 27-11-2014 |
| | | US 2018194852 A1 | 12-07-2018 |
| | | WO 2013088363 A1 | 20-06-2013 |
| WO 2013049666 A1 | 04-04-2013 | CA 2850178 A1 | 04-04-2013 |
| | | EP 2761298 A1 | 06-08-2014 |
| | | JP 6170925 B2 | 26-07-2017 |
| | | JP 2014531020 A | 20-11-2014 |
| | | US 2015011594 A1 | 08-01-2015 |
| | | US 2018172693 A1 | 21-06-2018 |
| | | WO 2013049666 A1 | 04-04-2013 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011092295 A
- WO 2013088363 A
- WO 2002087341 A
- WO 2002083080 A
- WO 2002082908 A
- WO 2004000220 A
- WO 2004054512 A
- US 6905874 B
- US 6867041 B
- US 6797514 B
- WO 2012079000 A
- US 6040177 A
- US 5827642 A
- WO 2012129514 A


### Non-patent literature cited in the description


- **SMELAND et al.** *Scand. J. Immunol.,* 1985, vol. 21 (3), 205-14
- **SMITH ; WATERMAN.** *Add. APL. Math,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad. Sci. USA,* 1988, vol. 85, 2444
- **ALTSCHUL et al.** *Nucl. Acids Res.,* 1977, vol. 25, 3389-3402
- **ALTSCHUL et al.** *J. Mol. Biol.,* 1990, vol. 215, 403-410
- **SÆBØE-LARSSEN et al.** *J. Immunol. Methods,* 2002, vol. 259, 191-203
- **WÄLCHLI et al.** *PLoS ONE,* 2011, vol. 6 (11), e27930
- **ALMASBAK et al.** *Cytotherapy,* 2011, vol. 13, 629-640
- **RABINOVICH et al.** *Hum. Gene Ther.,* 2009, vol. 20, 51-60
- **BEATTY et al.** *Cancer Immunol. Res.,* 2014, vol. 2, 112-120
- **WIGLER et al.** *Cell,* 1977, vol. 11 (1), 223-232
- **MULLEN et al.** *Proc. Natl. Acad. Sci. USA.,* 1992, vol. 89, 33-37
- **LUPTON S. D. et al.** *Mol. and Cell. Biology,* 1991, vol. 11, 3374-3378
- **VOGEL et al.** *Leukemia,* 2014, vol. 28, 192-195
- **GONG et al.** *Leukemia,* 1994, vol. 8, 652-658
- **PULÈ et al.** *Mol. Ther.,* 2005, vol. 12 (5), 933-941

SEQUENCE LISTING

<110>  Oslo universitetssykehus HF

<120>  Anti-CD37 Chimeric Antigen Receptors and Immune Cells Expressing Them

<130>  27.18.123933/05

<150>  GB 1600328.7
<151>  2016-01-08

<160>  54

<170>  PatentIn version 3.5

<210>  1
<211>  119
<212>  PRT
<213>  Mus musculus

<400>  1

```
Glu Ile Gln Leu Gln Gln Ser Gly Pro Glu Leu Val Lys Pro Gly Ala
1               5                   10                  15


Ser Val Lys Val Ser Cys Lys Ala Ser Gly Tyr Ser Phe Thr Asp Tyr
            20                  25                  30


Asn Met Tyr Trp Val Lys Gln Ser His Gly Lys Ser Leu Glu Trp Ile
        35                  40                  45


Gly Tyr Ile Asp Pro Tyr Asn Gly Asp Thr Thr Tyr Asn Gln Lys Phe
    50                  55                  60


Lys Gly Lys Ala Thr Leu Thr Val Asp Lys Ser Ser Ser Thr Ala Phe
65                  70                  75                  80


Ile His Leu Asn Ser Leu Thr Ser Glu Asp Ser Ala Val Tyr Tyr Cys
                85                  90                  95


Ala Arg Ser Pro Tyr Gly His Tyr Ala Met Asp Tyr Trp Gly Gln Gly
            100                 105                 110


Thr Ser Val Thr Val Ser Ser
        115
```

<210>  2
<211>  357
<212>  DNA
<213>  Mus musculus

<400>  2

```
gagattcagc tgcaacagtc tgggccagaa ctggtcaagc ctggtgcttc tgtgaaggtg       60
```

tcctgcaaag cgtctggata cagcttcact gactacaaca tgtattgggt caagcaatcc 120

cacggcaaat ccctggaatg gattgggtat atagacccct ataatgggga taccacctac 180

aaccagaagt tcaaagggaa agctacgctt actgtggaca aatccagcag cacagccttc 240

atccatctga atagcctgac tagcgaagat tcagccgtat actactgtgc ccgaagtcct 300

tacgggcact atgctatgga ctattggggt caaggtacgt cagtgactgt aagctcg 357

<210> 3
<211> 107
<212> PRT
<213> Mus musculus

<400> 3

Asp Ile Val Met Thr Gln Ser His Lys Leu Leu Ser Thr Ser Val Gly
1 5 10 15

Asp Arg Val Ser Ile Thr Cys Lys Ala Ser Gln Asp Val Ser Thr Ala
20 25 30

Val Asp Trp Tyr Gln Gln Lys Pro Gly Gln Ser Pro Lys Leu Leu Ile
35 40 45

Asn Trp Ala Ser Thr Arg His Thr Gly Val Pro Asp Arg Phe Thr Gly
50 55 60

Ser Gly Ser Gly Thr Asp Tyr Thr Leu Thr Ile Ser Ser Met Gln Ala
65 70 75 80

Glu Asp Leu Ala Leu Tyr Tyr Cys Arg Gln His Tyr Ser Thr Pro Phe
85 90 95

Thr Phe Gly Ser Gly Thr Lys Leu Glu Ile Lys
100 105

<210> 4
<211> 321
<212> DNA
<213> Mus musculus

<400> 4
gacatagtga tgacccagtc ccataagctg ctctccacct cagtcgggga tagggtgagc 60

atcacctgta aggcctctca ggacgttagc acagcagtcg attggtacca gcagaaacct 120

ggccagtctc ccaagctcct gatcaactgg gcaagtacac ggcataccgg agttcccgat 180

cgctttaccg gctctggctc tggcacagac tataccctca caatctcaag tatgcaggca 240

gaggatttgg ccctgtacta ctgcagacag cactattcca ctccgtttac atttgggtcc 300

gggacgaaac tggagattaa g 321

<210> 5
<211> 20
<212> PRT
<213> Artificial Sequence

<220>
<223> (G4S)4 Linker

<400> 5

Gly Gly Gly Gly Ser Gly Gly Gly Gly Ser Gly Gly Gly Gly Ser Gly
1 5 10 15

Gly Gly Gly Ser
20

<210> 6
<211> 20
<212> PRT
<213> Mus musculus

<400> 6

Met Glu Thr Asp Thr Leu Leu Leu Trp Val Leu Leu Leu Trp Val Pro
1 5 10 15

Gly Ser Thr Gly
20

<210> 7
<211> 266
<212> PRT
<213> Artificial Sequence

<220>
<223> HH1-Derived Anti-CD37 scFv

<400> 7

Met Glu Thr Asp Thr Leu Leu Leu Trp Val Leu Leu Leu Trp Val Pro
1 5 10 15

Gly Ser Thr Gly Asp Ile Val Met Thr Gln Ser His Lys Leu Leu Ser
20 25 30

Thr Ser Val Gly Asp Arg Val Ser Ile Thr Cys Lys Ala Ser Gln Asp
35 40 45

Val Ser Thr Ala Val Asp Trp Tyr Gln Gln Lys Pro Gly Gln Ser Pro
50 55 60

Lys Leu Leu Ile Asn Trp Ala Ser Thr Arg His Thr Gly Val Pro Asp
65 70 75 80

Arg Phe Thr Gly Ser Gly Ser Gly Thr Asp Tyr Thr Leu Thr Ile Ser
85 90 95

Ser Met Gln Ala Glu Asp Leu Ala Leu Tyr Tyr Cys Arg Gln His Tyr
100 105 110

Ser Thr Pro Phe Thr Phe Gly Ser Gly Thr Lys Leu Glu Ile Lys Gly
115 120 125

Gly Gly Gly Ser Gly Gly Gly Gly Ser Gly Gly Gly Gly Ser Gly Gly
130 135 140

Gly Gly Ser Glu Ile Gln Leu Gln Gln Ser Gly Pro Glu Leu Val Lys
145 150 155 160

Pro Gly Ala Ser Val Lys Val Ser Cys Lys Ala Ser Gly Tyr Ser Phe
165 170 175

Thr Asp Tyr Asn Met Tyr Trp Val Lys Gln Ser His Gly Lys Ser Leu
180 185 190

Glu Trp Ile Gly Tyr Ile Asp Pro Tyr Asn Gly Asp Thr Thr Tyr Asn
195 200 205

Gln Lys Phe Lys Gly Lys Ala Thr Leu Thr Val Asp Lys Ser Ser Ser
210 215 220

Thr Ala Phe Ile His Leu Asn Ser Leu Thr Ser Glu Asp Ser Ala Val
225 230 235 240

Tyr Tyr Cys Ala Arg Ser Pro Tyr Gly His Tyr Ala Met Asp Tyr Trp
245 250 255

Gly Gln Gly Thr Ser Val Thr Val Ser Ser
260 265

<210> 8
<211> 112
<212> PRT
<213> Homo sapiens

<400> 8

Arg Val Lys Phe Ser Arg Ser Ala Asp Ala Pro Ala Tyr Gln Gln Gly
1 5 10 15

Gln Asn Gln Leu Tyr Asn Glu Leu Asn Leu Gly Arg Arg Glu Glu Tyr
20 25 30

Asp Val Leu Asp Lys Arg Arg Gly Arg Asp Pro Glu Met Gly Gly Lys
35 40 45

Pro Arg Arg Lys Asn Pro Gln Glu Gly Leu Tyr Asn Glu Leu Gln Lys
50 55 60

Asp Lys Met Ala Glu Ala Tyr Ser Glu Ile Gly Met Lys Gly Glu Arg
65 70 75 80

Arg Arg Gly Lys Gly His Asp Gly Leu Tyr Gln Gly Leu Ser Thr Ala
85 90 95

Thr Lys Asp Thr Tyr Asp Ala Leu His Met Gln Ala Leu Pro Pro Arg
100 105 110

<210> 9
<211> 55
<212> PRT
<213> Homo sapiens

<400> 9

Phe Val Pro Val Phe Leu Pro Ala Lys Pro Thr Thr Thr Pro Ala Pro
1 5 10 15

Arg Pro Pro Thr Pro Ala Pro Thr Ile Ala Ser Gln Pro Leu Ser Leu
20 25 30

Arg Pro Glu Ala Cys Arg Pro Ala Ala Gly Gly Ala Val His Thr Arg
35 40 45

Gly Leu Asp Phe Ala Cys Asp
50 55

<210> 10
<211> 13
<212> PRT
<213> Homo sapiens

<400> 10

Ala Glu Pro Lys Ser Pro Asp Lys Thr His Thr Cys Pro
1 5 10

<210> 11
<211> 4
<212> PRT
<213> Artificial Sequence

<220>
<223> Hinge Domain/TM Domain Linker

<400> 11

Lys Asp Pro Lys
1

<210> 12
<211> 28
<212> PRT
<213> Homo sapiens

<400> 12

Ile Tyr Ile Trp Ala Pro Leu Ala Gly Thr Cys Gly Val Leu Leu Leu
1 5 10 15

Ser Leu Val Ile Thr Leu Tyr Cys Asn His Arg Asn
20 25

<210> 13
<211> 47
<212> PRT
<213> Homo sapiens

<400> 13

Arg Phe Ser Val Val Lys Arg Gly Arg Lys Lys Leu Leu Tyr Ile Phe
1 5 10 15

Lys Gln Pro Phe Met Arg Pro Val Gln Thr Thr Gln Glu Glu Asp Gly
20 25 30

Cys Ser Cys Arg Phe Pro Glu Glu Glu Glu Gly Gly Cys Glu Leu
35 40 45

<210> 14
<211> 1536
<212> DNA
<213> Artificial Sequence

<220>
<223> Anti-CD37 CAR 8843

<400> 14
atggagactg ataccctgtt gctttgggtg ctgcttctct gggttccagg cagcacaggc 60

gacatagtga tgacccagtc ccataagctg ctctccacct cagtcgggga tagggtgagc 120

atcacctgta aggcctctca ggacgttagc acagcagtcg attggtacca gcagaaacct 180

ggccagtctc ccaagctcct gatcaactgg gcaagtacac ggcataccgg agttcccgat 240

cgctttaccg gctctggctc tggcacagac tataccctca caatctcaag tatgcaggca 300

gaggatttgg ccctgtacta ctgcagacag cactattcca ctccgtttac atttgggtcc 360

gggacgaaac tggagattaa gggcggcgga ggaagtggtg gaggcggtag cggaggaggc 420

ggtagtggag gcggagggtc agagattcag ctgcaacagt ctgggccaga actggtcaag 480

cctggtgctt ctgtgaaggt gtcctgcaaa gcgtctggat acagcttcac tgactacaac 540

atgtattggg tcaagcaatc ccacggcaaa tccctggaat ggattgggta tatagacccc 600

tataatgggg ataccaccta caaccagaag ttcaaaggga aagctacgct tactgtggac 660

aaatccagca gcacagcctt catccatctg aatagcctga ctagcgaaga ttcagccgta 720

tactactgtg cccgaagtcc ttacgggcac tatgctatgg actattgggg tcaaggtacg 780

tcagtgactg taagctcgga tccctttgta cccgttttcc tgcctgcgaa acccaccacc 840

acaccagctc cgaggccacc cacaccagcc ccgaccattg cctctcagcc attgagcctt 900

aggcccgaag cttgtcggcc agctgcagga ggagcagtgc atacgagagg cctcgacttt 960

gcctgcgata tctacatctg ggcacctttg gccggaacat gtggggttct gctgctctca 1020

ctcgtgatta ccctgtactg caaccaccgt aacaggttca gcgtggtcaa gagaggacgc 1080

aagaaactgc tgtacatctt caagcaaccc ttcatgcgtc ctgtgcagac cactcaggag 1140

gaagatggct gcagttgccg gtttcctgag gaagaggaag gcggttgtga gctgagggtg 1200

aagttttccc gctctgcgga tgcacccgcc tatcagcagg gtcagaatca gctgtacaac 1260

gagctcaatc tgggtagacg cgaagagtac gacgtcttgg acaaacggcg agggagggac 1320

ccggagatgg gtgggaaacc ccgcagaaag aatcctcagg agggactgta taacgagctt 1380

cagaaggaca agatggccga agcctattcc gagataggca tgaaagggga acgacggaga 1440

gggaaaggcc atgatggcct gtatcaaggc ctgagcactg ccacaaagga cacttacgat 1500

gctctgcaca tgcaagctct tccacctcgg taatga 1536

<210> 15
<211> 1536
<212> DNA
<213> Artificial Sequence

<220>
<223> Anti-CD37 CAR Ab843

<400> 15
atggagactg ataccctgtt gctttgggtg ctgcttctct gggttccagg cagcacaggc 60

gacatagtga tgacccagtc ccataagctg ctctccacct cagtcgggga tagggtgagc 120

atcacctgta aggcctctca ggacgttagc acagcagtcg attggtacca gcagaaacct 180

ggccagtctc ccaagctcct gatcaactgg gcaagtacac ggcataccgg agttcccgat 240

cgctttaccg gctctggctc tggcacagac tataccctca caatctcaag tatgcaggca 300

gaggatttgg ccctgtacta ctgcagacag cactattcca ctccgtttac atttgggtcc 360

gggacgaaac tggagattaa gggcggcgga ggaagtggtg gaggcggtag cggaggaggc 420

ggtagtggag gcggagggtc agagattcag ctgcaacagt ctgggccaga actggtcaag 480

cctggtgctt ctgtgaaggt gtcctgcaaa gcgtctggat acagcttcac tgactacaac 540

atgtattggg tcaagcaatc ccacggcaaa tccctggaat ggattgggta tatagacccc 600

tataatgggg ataccaccta caaccagaag ttcaaaggga aagctacgct tactgtggac 660

aaatccagca gcacagcctt catccatctg aatagcctga ctagcgaaga ttcagccgta 720

tactactgtg cccgaagtcc ttacggcac tatgctatgg actattgggg tcaaggtacg 780

tcagtgactg taagctcgga tccctttgta cccgttttcc tgcctgcgaa acccaccacc 840

acaccagctc cgaggccacc cacaccagcc ccgaccattg cctctcagcc attgagcctt 900

aggcccgaag cttgtcggcc agctgcagga ggagcagtgc atacgagagg cctcgacttt 960

gcctgcgata tctacatctg ggcacctttg gccggaacat gtggggttct gctgctctca 1020

ctcgtgatta ccctgtactg caaccaccgt aacaggttca gcgtggtcaa gagaggacgc 1080

aagaaactgc tgtacatctt caagcaaccc ttcatgcgtc ctgtgcagac cactcaggag 1140

gaagatggct gcagttgccg gtttcctgag gaagaggaag gcggttgtga gctgagggtg 1200

aagttttccc gctctgcgga tgcacccgcc tatcagcagg gtcagaatca gctgtacaac 1260

gagctcaatc tgggtagacg cgaagagtac gacgtcttgg acaaacggcg agggagggac 1320

ccggagatgg gtgggaaacc ccgcagaaag aatcctcagg agggactgta taacgagctt 1380

cagaaggaca agatggccga agcctattcc gagataggca tgaaagggga acgacggaga 1440

gggaaaggcc atgatggcct gtatcaaggc ctgagcactg ccacaaagga cacttacgat 1500

gctctgcaca tgcaagctct tccacctcgg taatga 1536

<210> 16
<211> 246
<212> PRT
<213> Artificial Sequence

<220>
<223> HH1-Derived Anti-CD37 scFv Excluding Signal Sequence

<400> 16

Asp Ile Val Met Thr Gln Ser His Lys Leu Leu Ser Thr Ser Val Gly
1 5 10 15

Asp Arg Val Ser Ile Thr Cys Lys Ala Ser Gln Asp Val Ser Thr Ala
20 25 30

Val Asp Trp Tyr Gln Gln Lys Pro Gly Gln Ser Pro Lys Leu Leu Ile
35 40 45

Asn Trp Ala Ser Thr Arg His Thr Gly Val Pro Asp Arg Phe Thr Gly
50 55 60

Ser Gly Ser Gly Thr Asp Tyr Thr Leu Thr Ile Ser Ser Met Gln Ala
65 70 75 80

Glu Asp Leu Ala Leu Tyr Tyr Cys Arg Gln His Tyr Ser Thr Pro Phe
85 90 95

Thr Phe Gly Ser Gly Thr Lys Leu Glu Ile Lys Gly Gly Gly Gly Ser
100 105 110

Gly Gly Gly Gly Ser Gly Gly Gly Gly Ser Gly Gly Gly Gly Ser Glu
115 120 125

Ile Gln Leu Gln Gln Ser Gly Pro Glu Leu Val Lys Pro Gly Ala Ser
130 135 140

Val Lys Val Ser Cys Lys Ala Ser Gly Tyr Ser Phe Thr Asp Tyr Asn
145 150 155 160

Met Tyr Trp Val Lys Gln Ser His Gly Lys Ser Leu Glu Trp Ile Gly
165 170 175

Tyr Ile Asp Pro Tyr Asn Gly Asp Thr Thr Tyr Asn Gln Lys Phe Lys
180 185 190

Gly Lys Ala Thr Leu Thr Val Asp Lys Ser Ser Ser Thr Ala Phe Ile
195 200 205

His Leu Asn Ser Leu Thr Ser Glu Asp Ser Ala Val Tyr Tyr Cys Ala
210 215 220

Arg Ser Pro Tyr Gly His Tyr Ala Met Asp Tyr Trp Gly Gln Gly Thr
225 230 235 240

Ser Val Thr Val Ser Ser
245

<210> 17
<211> 27
<212> PRT
<213> Homo sapiens

<400> 17

Phe Trp Val Leu Val Val Val Gly Gly Val Leu Ala Cys Tyr Ser Leu
1 5 10 15

Leu Val Thr Val Ala Phe Ile Ile Phe Trp Val
20 25

<210> 18
<211> 41
<212> PRT
<213> Homo sapiens

<400> 18

Arg Ser Lys Arg Ser Arg Leu Leu His Ser Asp Tyr Met Asn Met Thr
1 5 10 15

Pro Arg Arg Pro Gly Pro Thr Arg Lys His Tyr Gln Pro Tyr Ala Pro
20 25 30

Pro Arg Asp Phe Ala Ala Tyr Arg Ser
35 40

<210> 19
<211> 36
<212> PRT
<213> Homo sapiens

<400> 19

Arg Asp Gln Arg Leu Pro Pro Asp Ala His Lys Pro Pro Gly Gly Gly
1 5 10 15

Ser Phe Arg Thr Pro Ile Gln Glu Glu Gln Ala Asp Ala His Ser Thr
20 25 30

Leu Ala Lys Ile
35

<210> 20
<211> 6
<212> DNA
<213> Homo sapiens

<400> 20
aataaa

<210> 21
<211> 17
<212> PRT
<213> Artificial Sequence

<220>
<223> Short IgG Hinge with Hinge-TM Linker

<400> 21

Ala Glu Pro Lys Ser Pro Asp Lys Thr His Thr Cys Pro Lys Asp Pro
1 5 10 15

Lys

<210> 22
<211> 19
<212> DNA
<213> Artificial Sequence

<220>
<223> Antibody Chain Forward Primer

<220>
<221> misc_feature
<222> (8)..(19)
<223> n = inosine

<400> 22
caccgggnng ggnngggnn 19

<210> 23
<211> 20
<212> DNA
<213> Artificial Sequence

<220>
<223> Heavy Chain Reverse Primer G1

<400> 23
ggtcactgtc actggctcag 20

<210> 24
<211> 21
<212> DNA
<213> Artificial Sequence

<220>
<223> Heavy Chain Reverse Primer G2a

<400> 24
tagaggtcag actgcaggac a 21

<210> 25
<211> 22
<212> DNA
<213> Artificial Sequence

<220>
<223> Heavy Chain Reverse Primer G2b

<400> 25
gagttccaag tcacagtcac tg 22

<210> 26
<211> 22
<212> DNA
<213> Artificial Sequence

<220>
<223>  Light Chain Reverse Primer IgK

<400>  26
gccatcaatc ttccacttga ca                                              22

<210>  27
<211>  23
<212>  DNA
<213>  Artificial Sequence

<220>
<223>  Heavy Chain Reverse Primer G2a

<400>  27
cttgaccagg catcctagag tca                                             23

<210>  28
<211>  27
<212>  DNA
<213>  Artificial Sequence

<220>
<223>  Heavy Chain Reverse Primer G1

<400>  28
atacgcgtgt ttgcagcaga tccaggg                                         27

<210>  29
<211>  29
<212>  DNA
<213>  Artificial Sequence

<220>
<223>  Heavy Chain Reverse Primer G2b

<400>  29
atacgcgtag ttgtatctcc acacccagg                                       29

<210>  30
<211>  21
<212>  DNA
<213>  Artificial Sequence

<220>
<223>  Light Chain Reverse Primer

<400>  30
caagaagcac acgactgagg c                                               21

<210>  31
<211>  798
<212>  DNA
<213>  Artificial Sequence

<220>
<223>  HH1-Derived Anti-CD37 scFv

<400> 31
atggagactg ataccctgtt gctttgggtg ctgcttctct gggttccagg cagcacaggc 60

gacatagtga tgacccagtc ccataagctg ctctccacct cagtcgggga tagggtgagc 120

atcacctgta aggcctctca ggacgttagc acagcagtcg attggtacca gcagaaacct 180

ggccagtctc ccaagctcct gatcaactgg gcaagtacac ggcataccgg agttcccgat 240

cgctttaccg gctctggctc tggcacagac tataccctca caatctcaag tatgcaggca 300

gaggatttgg ccctgtacta ctgcagacag cactattcca ctccgtttac atttgggtcc 360

gggacgaaac tggagattaa gggcggcgga ggaagtggtg gaggcggtag cggaggaggc 420

ggtagtggag gcggagggtc agagattcag ctgcaacagt ctgggccaga actggtcaag 480

cctggtgctt ctgtgaaggt gtcctgcaaa gcgtctggat acagcttcac tgactacaac 540

atgtattggg tcaagcaatc ccacggcaaa tccctggaat ggattgggta tatagacccc 600

tataatgggg ataccaccta caaccagaag ttcaaaggga aagctacgct tactgtggac 660

aaatccagca gcacagcctt catccatctg aatagcctga ctagcgaaga ttcagccgta 720

tactactgtg cccgaagtcc ttacgggcac tatgctatgg actattgggg tcaaggtacg 780

tcagtgactg taagctcg 798

<210> 32
<211> 508
<212> PRT
<213> Artificial Sequence

<220>
<223> Anti-CD37 CAR 8843

<400> 32

Met Glu Thr Asp Thr Leu Leu Leu Trp Val Leu Leu Leu Trp Val Pro
1               5                   10                  15

Gly Ser Thr Gly Asp Ile Val Met Thr Gln Ser His Lys Leu Leu Ser
            20                  25                  30

Thr Ser Val Gly Asp Arg Val Ser Ile Thr Cys Lys Ala Ser Gln Asp
        35                  40                  45

Val Ser Thr Ala Val Asp Trp Tyr Gln Gln Lys Pro Gly Gln Ser Pro
    50                  55                  60

Lys Leu Leu Ile Asn Trp Ala Ser Thr Arg His Thr Gly Val Pro Asp
65                  70                  75                  80

Arg Phe Thr Gly Ser Gly Ser Gly Thr Asp Tyr Thr Leu Thr Ile Ser
                85                  90                  95

Ser Met Gln Ala Glu Asp Leu Ala Leu Tyr Tyr Cys Arg Gln His Tyr
100 105 110

Ser Thr Pro Phe Thr Phe Gly Ser Gly Thr Lys Leu Glu Ile Lys Gly
115 120 125

Gly Gly Gly Ser Gly Gly Gly Gly Ser Gly Gly Gly Gly Ser Gly Gly
130 135 140

Gly Gly Ser Glu Ile Gln Leu Gln Gln Ser Gly Pro Glu Leu Val Lys
145 150 155 160

Pro Gly Ala Ser Val Lys Val Ser Cys Lys Ala Ser Gly Tyr Ser Phe
165 170 175

Thr Asp Tyr Asn Met Tyr Trp Val Lys Gln Ser His Gly Lys Ser Leu
180 185 190

Glu Trp Ile Gly Tyr Ile Asp Pro Tyr Asn Gly Asp Thr Thr Tyr Asn
195 200 205

Gln Lys Phe Lys Gly Lys Ala Thr Leu Thr Val Asp Lys Ser Ser Ser
210 215 220

Thr Ala Phe Ile His Leu Asn Ser Leu Thr Ser Glu Asp Ser Ala Val
225 230 235 240

Tyr Tyr Cys Ala Arg Ser Pro Tyr Gly His Tyr Ala Met Asp Tyr Trp
245 250 255

Gly Gln Gly Thr Ser Val Thr Val Ser Ser Phe Val Pro Val Phe Leu
260 265 270

Pro Ala Lys Pro Thr Thr Thr Pro Ala Pro Arg Pro Pro Thr Pro Ala
275 280 285

Pro Thr Ile Ala Ser Gln Pro Leu Ser Leu Arg Pro Glu Ala Cys Arg
290 295 300

Pro Ala Ala Gly Gly Ala Val His Thr Arg Gly Leu Asp Phe Ala Cys
305 310 315 320

Asp Ile Tyr Ile Trp Ala Pro Leu Ala Gly Thr Cys Gly Val Leu Leu
325 330 335

Leu Ser Leu Val Ile Thr Leu Tyr Cys Asn His Arg Asn Arg Phe Ser
340 345 350

Val Val Lys Arg Gly Arg Lys Lys Leu Leu Tyr Ile Phe Lys Gln Pro
355 360 365

Phe Met Arg Pro Val Gln Thr Thr Gln Glu Glu Asp Gly Cys Ser Cys
370 375 380

Arg Phe Pro Glu Glu Glu Glu Gly Gly Cys Glu Leu Arg Val Lys Phe
385 390 395 400

Ser Arg Ser Ala Asp Ala Pro Ala Tyr Gln Gln Gly Gln Asn Gln Leu
405 410 415

Tyr Asn Glu Leu Asn Leu Gly Arg Arg Glu Glu Tyr Asp Val Leu Asp
420 425 430

Lys Arg Arg Gly Arg Asp Pro Glu Met Gly Gly Lys Pro Arg Arg Lys
435 440 445

Asn Pro Gln Glu Gly Leu Tyr Asn Glu Leu Gln Lys Asp Lys Met Ala
450 455 460

Glu Ala Tyr Ser Glu Ile Gly Met Lys Gly Glu Arg Arg Arg Gly Lys
465 470 475 480

Gly His Asp Gly Leu Tyr Gln Gly Leu Ser Thr Ala Thr Lys Asp Thr
485 490 495

Tyr Asp Ala Leu His Met Gln Ala Leu Pro Pro Arg
500 505

<210> 33
<211> 470
<212> PRT
<213> Artificial Sequence

<220>
<223> Anti-CD37 CAR Ab843

<400> 33

Met Glu Thr Asp Thr Leu Leu Leu Trp Val Leu Leu Leu Trp Val Pro
1 5 10 15

Gly Ser Thr Gly Asp Ile Val Met Thr Gln Ser His Lys Leu Leu Ser
20 25 30

Thr Ser Val Gly Asp Arg Val Ser Ile Thr Cys Lys Ala Ser Gln Asp
35 40 45

Val Ser Thr Ala Val Asp Trp Tyr Gln Gln Lys Pro Gly Gln Ser Pro
50 55 60

Lys Leu Leu Ile Asn Trp Ala Ser Thr Arg His Thr Gly Val Pro Asp
65 70 75 80

Arg Phe Thr Gly Ser Gly Ser Gly Thr Asp Tyr Thr Leu Thr Ile Ser
85 90 95

Ser Met Gln Ala Glu Asp Leu Ala Leu Tyr Tyr Cys Arg Gln His Tyr
100 105 110

Ser Thr Pro Phe Thr Phe Gly Ser Gly Thr Lys Leu Glu Ile Lys Gly
115 120 125

Gly Gly Gly Ser Gly Gly Gly Gly Ser Gly Gly Gly Gly Ser Gly Gly
130 135 140

Gly Gly Ser Glu Ile Gln Leu Gln Gln Ser Gly Pro Glu Leu Val Lys
145 150 155 160

Pro Gly Ala Ser Val Lys Val Ser Cys Lys Ala Ser Gly Tyr Ser Phe
165 170 175

Thr Asp Tyr Asn Met Tyr Trp Val Lys Gln Ser His Gly Lys Ser Leu
180 185 190

Glu Trp Ile Gly Tyr Ile Asp Pro Tyr Asn Gly Asp Thr Thr Tyr Asn
195 200 205

Gln Lys Phe Lys Gly Lys Ala Thr Leu Thr Val Asp Lys Ser Ser Ser
210 215 220

Thr Ala Phe Ile His Leu Asn Ser Leu Thr Ser Glu Asp Ser Ala Val
225 230 235 240

Tyr Tyr Cys Ala Arg Ser Pro Tyr Gly His Tyr Ala Met Asp Tyr Trp
245 250 255

Gly Gln Gly Thr Ser Val Thr Val Ser Ser Ala Glu Pro Lys Ser Pro
260 265 270

Asp Lys Thr His Thr Cys Pro Lys Asp Pro Lys Ile Tyr Ile Trp Ala
275 280 285

Pro Leu Ala Gly Thr Cys Gly Val Leu Leu Leu Ser Leu Val Ile Thr
290 295 300

```
Leu Tyr Cys Asn His Arg Asn Arg Phe Ser Val Val Lys Arg Gly Arg
305                 310                 315                 320

Lys Lys Leu Leu Tyr Ile Phe Lys Gln Pro Phe Met Arg Pro Val Gln
                325                 330                 335

Thr Thr Gln Glu Glu Asp Gly Cys Ser Cys Arg Phe Pro Glu Glu Glu
            340                 345                 350

Glu Gly Gly Cys Glu Leu Arg Val Lys Phe Ser Arg Ser Ala Asp Ala
        355                 360                 365

Pro Ala Tyr Gln Gln Gly Gln Asn Gln Leu Tyr Asn Glu Leu Asn Leu
    370                 375                 380

Gly Arg Arg Glu Glu Tyr Asp Val Leu Asp Lys Arg Arg Gly Arg Asp
385                 390                 395                 400

Pro Glu Met Gly Gly Lys Pro Arg Arg Lys Asn Pro Gln Glu Gly Leu
                405                 410                 415

Tyr Asn Glu Leu Gln Lys Asp Lys Met Ala Glu Ala Tyr Ser Glu Ile
            420                 425                 430

Gly Met Lys Gly Glu Arg Arg Arg Gly Lys Gly His Asp Gly Leu Tyr
        435                 440                 445

Gln Gly Leu Ser Thr Ala Thr Lys Asp Thr Tyr Asp Ala Leu His Met
    450                 455                 460

Gln Ala Leu Pro Pro Arg
465                 470


<210>  34
<211>  12
<212>  PRT
<213>  Mus musculus

<400>  34

Ala Arg Ser Pro Tyr Gly His Tyr Ala Met Asp Tyr
1               5                   10


<210>  35
<211>  9
<212>  PRT
<213>  Mus musculus

<400>  35
```

Arg Gln His Tyr Ser Thr Pro Phe Thr
1 5

<210> 36
<211> 159
<212> PRT
<213> Mus musculus

<400> 36

Met Glu Trp Ser Trp Ile Phe Leu Phe Leu Leu Ser Gly Thr Thr Gly
1 5 10 15

Val His Ser Glu Ile Gln Leu Gln Gln Ser Gly Pro Glu Leu Val Lys
20 25 30

Pro Gly Ala Ser Val Lys Val Ser Cys Lys Ala Ser Gly Tyr Ser Phe
35 40 45

Thr Asp Tyr Asn Met Tyr Trp Val Lys Gln Ser His Gly Lys Ser Leu
50 55 60

Glu Trp Ile Gly Tyr Ile Asp Pro Tyr Asn Gly Asp Thr Thr Tyr Asn
65 70 75 80

Gln Lys Phe Lys Gly Lys Ala Thr Leu Thr Val Asp Lys Ser Ser Ser
85 90 95

Thr Ala Phe Ile His Leu Asn Ser Leu Thr Ser Glu Asp Ser Ala Val
100 105 110

Tyr Tyr Cys Ala Arg Ser Pro Tyr Gly His Tyr Ala Met Asp Tyr Trp
115 120 125

Gly Gln Gly Thr Ser Val Thr Val Ser Ser Ala Lys Thr Thr Pro Pro
130 135 140

Ser Val Tyr Pro Leu Ala Pro Gly Ser Ala Ala Gln Thr Arg Val
145 150 155

<210> 37
<211> 153
<212> PRT
<213> Mus musculus

<400> 37

Met Ala Phe Val Phe Val Phe Leu Trp Leu Ser Gly Val Asp Gly Asp
1 5 10 15

Ile Val Met Thr Gln Ser His Lys Leu Leu Ser Thr Ser Val Gly Asp
20 25 30

Arg Val Ser Ile Thr Cys Lys Ala Ser Gln Asp Val Ser Thr Ala Val
35 40 45

Asp Trp Tyr Gln Gln Lys Pro Gly Gln Ser Pro Lys Leu Leu Ile Asn
50 55 60

Trp Ala Ser Thr Arg His Thr Gly Val Pro Asp Arg Phe Thr Gly Ser
65 70 75 80

Gly Ser Gly Thr Asp Tyr Thr Leu Thr Ile Ser Ser Met Gln Ala Glu
85 90 95

Asp Leu Ala Leu Tyr Tyr Cys Arg Gln His Tyr Ser Thr Pro Phe Thr
100 105 110

Phe Gly Ser Gly Thr Lys Leu Glu Ile Lys Arg Ala Asp Ala Ala Pro
115 120 125

Thr Val Ser Ile Phe Pro Pro Ser Ser Glu Gln Leu Thr Ser Gly Gly
130 135 140

Ala Ser Val Val Cys Phe Leu Asn His
145 150

<210> 38
<211> 805
<212> DNA
<213> Artificial Sequence

<220>
<223> Anti-CD37 scFv Sequence with Restriction Sites at Both Ends

<400> 38
ccatggagac tgataccctg ttgctttggg tgctgcttct ctgggttcca ggcagcacag 60

gcgacatagt gatgacccag tcccataagc tgctctccac ctcagtcggg gatagggtga 120

gcatcacctg taaggcctct caggacgtta gcacagcagt cgattggtac cagcagaaac 180

ctggccagtc tcccaagctc ctgatcaact gggcaagtac acggcatacc ggagttcccg 240

atcgctttac cggctctggc tctggcacag actataccct cacaatctca agtatgcagg 300

cagaggattt ggccctgtac tactgcagac agcactattc cactccgttt acatttgggt 360

ccgggacgaa actggagatt aagggcggcg gaggaagtgg tggaggcggt agcggaggag 420

gcggtagtgg aggcggaggg tcagagattc agctgcaaca gtctgggcca gaactggtca 480

agcctggtgc ttctgtgaag gtgtcctgca aagcgtctgg atacagcttc actgactaca 540

acatgtattg ggtcaagcaa tcccacggca aatccctgga atggattggg tatatagacc 600

cctataatgg ggataccacc tacaaccaga agttcaaagg gaaagctacg cttactgtgg 660

acaaatccag cagcacagcc ttcatccatc tgaatagcct gactagcgaa gattcagccg 720

tatactactg tgcccgaagt ccttacgggc actatgctat ggactattgg ggtcaaggta 780

cgtcagtgac tgtaagctcg gatcc 805

<210> 39
<211> 721
<212> PRT
<213> Artificial Sequence

<220>
<223> 3rd Generation Anti-CD37 CAR

<400> 39

Met Glu Thr Asp Thr Leu Leu Leu Trp Val Leu Leu Leu Trp Val Pro
1 5 10 15

Gly Ser Thr Gly Asp Ile Val Met Thr Gln Ser His Lys Leu Leu Ser
20 25 30

Thr Ser Val Gly Asp Arg Val Ser Ile Thr Cys Lys Ala Ser Gln Asp
35 40 45

Val Ser Thr Ala Val Asp Trp Tyr Gln Gln Lys Pro Gly Gln Ser Pro
50 55 60

Lys Leu Leu Ile Asn Trp Ala Ser Thr Arg His Thr Gly Val Pro Asp
65 70 75 80

Arg Phe Thr Gly Ser Gly Ser Gly Thr Asp Tyr Thr Leu Thr Ile Ser
85 90 95

Ser Met Gln Ala Glu Asp Leu Ala Leu Tyr Tyr Cys Arg Gln His Tyr
100 105 110

Ser Thr Pro Phe Thr Phe Gly Ser Gly Thr Lys Leu Glu Ile Lys Gly
115 120 125

Gly Gly Gly Ser Gly Gly Gly Gly Ser Gly Gly Gly Gly Ser Gly Gly
130 135 140

Gly Gly Ser Glu Ile Gln Leu Gln Gln Ser Gly Pro Glu Leu Val Lys
145 150 155 160

Pro Gly Ala Ser Val Lys Val Ser Cys Lys Ala Ser Gly Tyr Ser Phe
165 170 175

```
Thr Asp Tyr Asn Met Tyr Trp Val Lys Gln Ser His Gly Lys Ser Leu
            180                 185                 190

Glu Trp Ile Gly Tyr Ile Asp Pro Tyr Asn Gly Asp Thr Thr Tyr Asn
        195                 200                 205

Gln Lys Phe Lys Gly Lys Ala Thr Leu Thr Val Asp Lys Ser Ser Ser
    210                 215                 220

Thr Ala Phe Ile His Leu Asn Ser Leu Thr Ser Glu Asp Ser Ala Val
225                 230                 235                 240

Tyr Tyr Cys Ala Arg Ser Pro Tyr Gly His Tyr Ala Met Asp Tyr Trp
                245                 250                 255

Gly Gln Gly Thr Ser Val Thr Val Ser Ser Asp Pro Ala Glu Pro Lys
            260                 265                 270

Ser Pro Asp Lys Thr His Thr Cys Pro Pro Cys Pro Ala Pro Glu Leu
        275                 280                 285

Leu Gly Gly Pro Ser Val Phe Leu Phe Pro Pro Lys Pro Lys Asp Thr
    290                 295                 300

Leu Met Ile Ser Arg Thr Pro Glu Val Thr Cys Val Val Val Asp Val
305                 310                 315                 320

Ser His Glu Asp Pro Glu Val Lys Phe Asn Trp Tyr Val Asp Gly Val
                325                 330                 335

Glu Val His Asn Ala Lys Thr Lys Pro Arg Glu Glu Gln Tyr Asn Ser
            340                 345                 350

Thr Tyr Arg Val Val Ser Val Leu Thr Val Leu His Gln Asp Trp Leu
        355                 360                 365

Asn Gly Lys Glu Tyr Lys Cys Lys Val Ser Asn Lys Ala Leu Pro Ala
    370                 375                 380

Pro Ile Glu Lys Thr Ile Ser Lys Ala Lys Gly Gln Pro Arg Glu Pro
385                 390                 395                 400

Gln Val Tyr Thr Leu Pro Pro Ser Arg Asp Glu Leu Thr Lys Asn Gln
                405                 410                 415

Val Ser Leu Thr Cys Leu Val Lys Gly Phe Tyr Pro Ser Asp Ile Ala
```

420 425 430

Val Glu Trp Glu Ser Asn Gly Gln Pro Glu Asn Asn Tyr Lys Thr Thr
435 440 445

Pro Pro Val Leu Asp Ser Asp Gly Ser Phe Phe Leu Tyr Ser Lys Leu
450 455 460

Thr Val Asp Lys Ser Arg Trp Gln Gln Gly Asn Val Phe Ser Cys Ser
465 470 475 480

Val Met His Glu Ala Leu His Asn His Tyr Thr Gln Lys Ser Leu Ser
485 490 495

Leu Ser Pro Gly Lys Lys Asp Pro Lys Phe Trp Val Leu Val Val Val
500 505 510

Gly Gly Val Leu Ala Cys Tyr Ser Leu Leu Val Thr Val Ala Phe Ile
515 520 525

Ile Phe Trp Val Arg Ser Lys Arg Ser Arg Leu Leu His Ser Asp Tyr
530 535 540

Met Asn Met Thr Pro Arg Arg Pro Gly Pro Thr Arg Lys His Tyr Gln
545 550 555 560

Pro Tyr Ala Pro Pro Arg Asp Phe Ala Ala Tyr Arg Ser Arg Asp Gln
565 570 575

Arg Leu Pro Pro Asp Ala His Lys Pro Pro Gly Gly Gly Ser Phe Arg
580 585 590

Thr Pro Ile Gln Glu Glu Gln Ala Asp Ala His Ser Thr Leu Ala Lys
595 600 605

Ile Arg Val Lys Phe Ser Arg Ser Ala Asp Ala Pro Ala Tyr Gln Gln
610 615 620

Gly Gln Asn Gln Leu Tyr Asn Glu Leu Asn Leu Gly Arg Arg Glu Glu
625 630 635 640

Tyr Asp Val Leu Asp Lys Arg Arg Gly Arg Asp Pro Glu Met Gly Gly
645 650 655

Lys Pro Arg Arg Lys Asn Pro Gln Glu Gly Leu Tyr Asn Glu Leu Gln
660 665 670

Lys Asp Lys Met Ala Glu Ala Tyr Ser Glu Ile Gly Met Lys Gly Glu
675 680 685

Arg Arg Arg Gly Lys Gly His Asp Gly Leu Tyr Gln Gly Leu Ser Thr
690 695 700

Ala Thr Lys Asp Thr Tyr Asp Ala Leu His Met Gln Ala Leu Pro Pro
705 710 715 720

Arg

<210> 40
<211> 239
<212> PRT
<213> Homo sapiens

<400> 40

Asp Pro Ala Glu Pro Lys Ser Pro Asp Lys Thr His Thr Cys Pro Pro
1 5 10 15

Cys Pro Ala Pro Glu Leu Leu Gly Gly Pro Ser Val Phe Leu Phe Pro
20 25 30

Pro Lys Pro Lys Asp Thr Leu Met Ile Ser Arg Thr Pro Glu Val Thr
35 40 45

Cys Val Val Val Asp Val Ser His Glu Asp Pro Glu Val Lys Phe Asn
50 55 60

Trp Tyr Val Asp Gly Val Glu Val His Asn Ala Lys Thr Lys Pro Arg
65 70 75 80

Glu Glu Gln Tyr Asn Ser Thr Tyr Arg Val Val Ser Val Leu Thr Val
85 90 95

Leu His Gln Asp Trp Leu Asn Gly Lys Glu Tyr Lys Cys Lys Val Ser
100 105 110

Asn Lys Ala Leu Pro Ala Pro Ile Glu Lys Thr Ile Ser Lys Ala Lys
115 120 125

Gly Gln Pro Arg Glu Pro Gln Val Tyr Thr Leu Pro Pro Ser Arg Asp
130 135 140

Glu Leu Thr Lys Asn Gln Val Ser Leu Thr Cys Leu Val Lys Gly Phe
145 150 155 160

Tyr Pro Ser Asp Ile Ala Val Glu Trp Glu Ser Asn Gly Gln Pro Glu
165 170 175

Asn Asn Tyr Lys Thr Thr Pro Pro Val Leu Asp Ser Asp Gly Ser Phe
180 185 190

Phe Leu Tyr Ser Lys Leu Thr Val Asp Lys Ser Arg Trp Gln Gln Gly
195 200 205

Asn Val Phe Ser Cys Ser Val Met His Glu Ala Leu His Asn His Tyr
210 215 220

Thr Gln Lys Ser Leu Ser Leu Ser Pro Gly Lys Lys Asp Pro Lys
225 230 235

<210> 41
<211> 16
<212> DNA
<213> Artificial Sequence

<220>
<223> Actin Forward Primer

<400> 41
gctccggcat gtgcaa 16

<210> 42
<211> 19
<212> DNA
<213> Artificial Sequence

<220>
<223> Actin Reverse Primer

<400> 42
aggatcttca tgaggtagt 19

<210> 43
<211> 6
<212> PRT
<213> Mus musculus

<400> 43

Gln Asp Val Ser Thr Ala
1 5

<210> 44
<211> 3
<212> PRT
<213> Mus musculus

<400> 44

Trp Ala Ser

1

<210> 45
<211> 8
<212> PRT
<213> Mus musculus

<400> 45

Gly Tyr Ser Phe Thr Asp Tyr Asn
1 5

<210> 46
<211> 8
<212> PRT
<213> Mus musculus

<400> 46

Ile Asp Pro Tyr Asn Gly Asp Thr
1 5

<210> 47
<211> 107
<212> PRT
<213> Artificial Sequence

<220>
<223> Modified VL Sequence with Humanised FR regions and Murine CDRs

<400> 47

Asp Ile Val Met Thr Gln Ser Pro Asp Ser Leu Ala Val Ser Leu Gly
1 5 10 15

Glu Arg Ala Thr Ile Asn Cys Lys Ser Ser Gln Asp Val Ser Thr Ala
20 25 30

Leu Ala Trp Tyr Gln Gln Lys Pro Gly Gln Pro Pro Lys Leu Leu Ile
35 40 45

Tyr Trp Ala Ser Thr Arg Glu Ser Gly Val Pro Asp Arg Phe Ser Gly
50 55 60

Ser Gly Ser Gly Thr Asp Phe Thr Leu Thr Ile Ser Ser Leu Gln Ala
65 70 75 80

Glu Asp Val Ala Val Tyr Tyr Cys Arg Gln His Tyr Ser Thr Pro Phe
85 90 95

Thr Phe Gly Gln Gly Thr Lys Leu Glu Ile Lys
100 105

<210> 48
<211> 119
<212> PRT
<213> Artificial Sequence

<220>
<223> Modified VH Sequence with Humanised FR Regions and Murine CDRs

<400> 48

Glu Val Gln Leu Val Gln Ser Gly Ala Glu Val Lys Lys Pro Gly Ala
1               5                   10                  15

Thr Val Lys Ile Ser Cys Lys Val Ser Gly Tyr Ser Phe Thr Asp Tyr
            20                  25                  30

Asn Met His Trp Val Gln Gln Ala Pro Gly Lys Gly Leu Glu Trp Met
        35                  40                  45

Gly Leu Ile Asp Pro Tyr Asn Gly Asp Thr Ile Tyr Ala Glu Lys Phe
    50                  55                  60

Gln Gly Arg Val Thr Ile Thr Ala Asp Thr Ser Thr Asp Thr Ala Tyr
65                  70                  75                  80

Met Glu Leu Ser Ser Leu Arg Ser Glu Asp Thr Ala Val Tyr Tyr Cys
                85                  90                  95

Ala Arg Ser Pro Tyr Gly His Tyr Ala Met Asp Tyr Trp Gly Gln Gly
            100                 105                 110

Thr Thr Val Thr Val Ser Ser
        115

<210> 49
<211> 321
<212> DNA
<213> Artificial Sequence

<220>
<223> Modified VL Sequence with Humanised FR Regions and Murine CDRs

<400> 49
gacatcgtga tgacccagag ccccgacagc ctggccgtga gcctgggcga gagagccacc 60

atcaactgca agagcagcca ggacgtgagc accgccctgg cctggtacca gcagaagccc 120

ggccagcccc ccaagctgct gatctactgg gccagcacca gagagagcgg cgtgcccgac 180

agattcagcg gcagcggcag cggcaccgac ttcaccctga ccatcagcag cctgcaggcc 240

gaggacgtgg ccgtgtacta ctgcagacag cactacagca ccccttcac cttcggccag 300

ggcaccaagc tggagatcaa g 321

<210> 50
<211> 357
<212> DNA
<213> Artificial Sequence

<220>
<223> Modified VH Sequence with Humanised FR Regions and Murine CDRs

<400> 50
gaggtgcagc tggtgcagag cggcgccgag gtgaagaagc ccggcgccac cgtgaagatc 60

agctgcaagg tgagcggcta cagcttcacc gactacaaca tgcactgggt gcagcaggcc 120

cccggcaagg gcctggagtg gatgggcctg atcgacccct acaacggcga caccatctac 180

gccgagaagt tccagggcag agtgaccatc accgccgaca ccagcaccga caccgcctac 240

atggagctga gcagcctgag aagcgaggac accgccgtgt actactgcgc cagaagcccc 300

tacggccact acgccatgga ctactggggc cagggcacca ccgtgaccgt gagcagc 357

<210> 51
<211> 538
<212> PRT
<213> Artificial Sequence

<220>
<223> 3rd Generation CAR with CD8 Hinge and TM Domains

<400> 51

Met Glu Thr Asp Thr Leu Leu Leu Trp Val Leu Leu Leu Trp Val Pro
1 5 10 15

Gly Ser Thr Gly Asp Ile Val Met Thr Gln Ser His Lys Leu Leu Ser
20 25 30

Thr Ser Val Gly Asp Arg Val Ser Ile Thr Cys Lys Ala Ser Gln Asp
35 40 45

Val Ser Thr Ala Val Asp Trp Tyr Gln Gln Lys Pro Gly Gln Ser Pro
50 55 60

Lys Leu Leu Ile Asn Trp Ala Ser Thr Arg His Thr Gly Val Pro Asp
65 70 75 80

Arg Phe Thr Gly Ser Gly Ser Gly Thr Asp Tyr Thr Leu Thr Ile Ser
85 90 95

Ser Met Gln Ala Glu Asp Leu Ala Leu Tyr Tyr Cys Arg Gln His Tyr
100 105 110

Ser Thr Pro Phe Thr Phe Gly Ser Gly Thr Lys Leu Glu Ile Lys Gly
115 120 125

```
Gly Gly Gly Ser Gly Gly Gly Gly Ser Gly Gly Gly Gly Ser Gly Gly
    130                 135                 140

Gly Gly Ser Glu Ile Gln Leu Gln Gln Ser Gly Pro Glu Leu Val Lys
145                 150                 155                 160

Pro Gly Ala Ser Val Lys Val Ser Cys Lys Ala Ser Gly Tyr Ser Phe
                165                 170                 175

Thr Asp Tyr Asn Met Tyr Trp Val Lys Gln Ser His Gly Lys Ser Leu
            180                 185                 190

Glu Trp Ile Gly Tyr Ile Asp Pro Tyr Asn Gly Asp Thr Thr Tyr Asn
        195                 200                 205

Gln Lys Phe Lys Gly Lys Ala Thr Leu Thr Val Asp Lys Ser Ser Ser
    210                 215                 220

Thr Ala Phe Ile His Leu Asn Ser Leu Thr Ser Glu Asp Ser Ala Val
225                 230                 235                 240

Tyr Tyr Cys Ala Arg Ser Pro Tyr Gly His Tyr Ala Met Asp Tyr Trp
                245                 250                 255

Gly Gln Gly Thr Ser Val Thr Val Ser Ser Phe Val Pro Val Phe Leu
            260                 265                 270

Pro Ala Lys Pro Thr Thr Thr Pro Ala Pro Arg Pro Pro Thr Pro Ala
        275                 280                 285

Pro Thr Ile Ala Ser Gln Pro Leu Ser Leu Arg Pro Glu Ala Cys Arg
    290                 295                 300

Pro Ala Ala Gly Gly Ala Val His Thr Arg Gly Leu Asp Phe Ala Cys
305                 310                 315                 320

Asp Ile Tyr Ile Trp Ala Pro Leu Ala Gly Thr Cys Gly Val Leu Leu
                325                 330                 335

Leu Ser Leu Val Ile Thr Leu Tyr Cys Asn His Arg Asn Arg Ser Lys
            340                 345                 350

Arg Ser Arg Leu Leu His Ser Asp Tyr Met Asn Met Thr Pro Arg Arg
        355                 360                 365

Pro Gly Pro Thr Arg Lys His Tyr Gln Pro Tyr Ala Pro Pro Arg Asp
```

370 375 380

Phe Ala Ala Tyr Arg Ser Arg Asp Gln Arg Leu Pro Pro Asp Ala His
385 390 395 400

Lys Pro Pro Gly Gly Gly Ser Phe Arg Thr Pro Ile Gln Glu Glu Gln
405 410 415

Ala Asp Ala His Ser Thr Leu Ala Lys Ile Arg Val Lys Phe Ser Arg
420 425 430

Ser Ala Asp Ala Pro Ala Tyr Gln Gln Gly Gln Asn Gln Leu Tyr Asn
435 440 445

Glu Leu Asn Leu Gly Arg Arg Glu Glu Tyr Asp Val Leu Asp Lys Arg
450 455 460

Arg Gly Arg Asp Pro Glu Met Gly Gly Lys Pro Arg Arg Lys Asn Pro
465 470 475 480

Gln Glu Gly Leu Tyr Asn Glu Leu Gln Lys Asp Lys Met Ala Glu Ala
485 490 495

Tyr Ser Glu Ile Gly Met Lys Gly Glu Arg Arg Arg Gly Lys Gly His
500 505 510

Asp Gly Leu Tyr Gln Gly Leu Ser Thr Ala Thr Lys Asp Thr Tyr Asp
515 520 525

Ala Leu His Met Gln Ala Leu Pro Pro Arg
530 535

<210> 52
<211> 1617
<212> DNA
<213> Artificial Sequence

<220>
<223> DNA Sequence Encoding CAR of SEQ ID NO. 51

<400> 52
atggagaccg acaccctgct gctgtgggtg ctgctgctgt gggtgcccgg cagcaccggc 60

gacatcgtga tgacccagag ccacaagctg ctgagcacca gcgtgggcga cagagtgagc 120

atcacctgca aggccagcca ggacgtgagc accgccgtgg actggtacca gcagaagccc 180

ggccagagcc ccaagctgct gatcaactgg gccagcacca gacacaccgg cgtgcccgac 240

agattcaccg gcagcggcag cggcaccgac tacaccctga ccatcagcag catgcaggcc 300

gaggacctgg ccctgtacta ctgcagacag cactacagca ccccccttcac cttcggcagc 360

```
ggcaccaagc tggagatcaa gggcggcggc ggcagcggcg gcggcggcag cggcggcggc      420
ggcagcggcg gcggcggcag cgagatccag ctgcagcaga gcggccccga gctggtgaag      480
cccggcgcca gcgtgaaggt gagctgcaag gccagcggct acagcttcac cgactacaac      540
atgtactggg tgaagcagag ccacggcaag agcctggagt ggatcggcta catcgacccc      600
tacaacggcg acaccaccta caaccagaag ttcaagggca aggccaccct gaccgtggac      660
aagagcagca gcaccgcctt catccacctg aacagcctga ccagcgagga cagcgccgtg      720
tactactgcg ccagaagccc ctacggccac tacgccatgg actactgggg ccagggcacc      780
agcgtgaccg tgagcagctt cgtgcccgtg ttcctgcccg ccaagcccac caccaccccc      840
gcccccagac cccccacccc cgcccccacc atcgccagcc agcccctgag cctgagaccc      900
gaggcctgca gacccgccgc cggcggcgcc gtgcacacca gaggcctgga cttcgcctgc      960
gacatctaca tctgggcccc cctggccggc acctgcggcg tgctgctgct gagcctggtg     1020
atcaccctgt actgcaacca cagaaacaga agcaagagaa gcagactgct gcacagcgac     1080
tacatgaaca tgacccccag aagacccggc cccaccagaa agcactacca gccctacgcc     1140
ccccccagag acttcgccgc ctacagaagc agagaccaga gactgccccc cgacgcccac     1200
aagccccccg gcggcggcag cttcagaacc cccatccagg aggagcaggc cgacgcccac     1260
agcaccctgg ccaagatcag agtgaagttc agcagaagcg ccgacgcccc cgcctaccag     1320
cagggccaga accagctgta caacgagctg aacctgggca gaagagagga gtacgacgtg     1380
ctggacaaga gaagaggcag agaccccgag atgggcggca agcccagaag aaagaacccc     1440
caggagggcc tgtacaacga gctgcagaag gacaagatgg ccgaggccta cagcgagatc     1500
ggcatgaagg gcgagagaag aagaggcaag ggccacgacg gcctgtacca gggcctgagc     1560
accgccacca aggacaccta cgacgccctg cacatgcagg ccctgccccc cagatga        1617
```

```
<210>  53
<211>  266
<212>  PRT
<213>  Artificial Sequence

<220>
<223>  HH1-Derived scFv Domain of Anti-CD37 CAR with Humanised FR Regions

<400>  53

Met Glu Thr Asp Thr Leu Leu Leu Trp Val Leu Leu Leu Trp Val Pro
1               5                   10                  15

Gly Ser Thr Gly Asp Ile Val Met Thr Gln Ser Pro Asp Ser Leu Ala
            20                  25                  30

Val Ser Leu Gly Glu Arg Ala Thr Ile Asn Cys Lys Ser Ser Gln Asp
```

35 40 45

Val Ser Thr Ala Leu Ala Trp Tyr Gln Gln Lys Pro Gly Gln Pro Pro
50 55 60

Lys Leu Leu Ile Tyr Trp Ala Ser Thr Arg Glu Ser Gly Val Pro Asp
65 70 75 80

Arg Phe Ser Gly Ser Gly Ser Gly Thr Asp Phe Thr Leu Thr Ile Ser
85 90 95

Ser Leu Gln Ala Glu Asp Val Ala Val Tyr Tyr Cys Arg Gln His Tyr
100 105 110

Ser Thr Pro Phe Thr Phe Gly Gln Gly Thr Lys Leu Glu Ile Lys Gly
115 120 125

Gly Gly Gly Ser Gly Gly Gly Gly Ser Gly Gly Gly Gly Ser Gly Gly
130 135 140

Gly Gly Ser Glu Val Gln Leu Val Gln Ser Gly Ala Glu Val Lys Lys
145 150 155 160

Pro Gly Ala Thr Val Lys Ile Ser Cys Lys Val Ser Gly Tyr Ser Phe
165 170 175

Thr Asp Tyr Asn Met His Trp Val Gln Gln Ala Pro Gly Lys Gly Leu
180 185 190

Glu Trp Met Gly Leu Ile Asp Pro Tyr Asn Gly Asp Thr Ile Tyr Ala
195 200 205

Glu Lys Phe Gln Gly Arg Val Thr Ile Thr Ala Asp Thr Ser Thr Asp
210 215 220

Thr Ala Tyr Met Glu Leu Ser Ser Leu Arg Ser Glu Asp Thr Ala Val
225 230 235 240

Tyr Tyr Cys Ala Arg Ser Pro Tyr Gly His Tyr Ala Met Asp Tyr Trp
245 250 255

Gly Gln Gly Thr Thr Val Thr Val Ser Ser
260 265

<210> 54
<211> 798
<212> DNA
<213> Artificial Sequence

```
<220>
<223>  HH1-Derived scFv Domain of Anti-CD37 CAR with Humanised FR Regions

<400>  54
atggagaccg acaccctgct gctgtgggtg ctgctgctgt gggtgcccgg cagcaccggc       60

gacatcgtga tgacccagag ccccgacagc ctggccgtga gcctgggcga gagagccacc      120

atcaactgca agagcagcca ggacgtgagc accgccctgg cctggtacca gcagaagccc      180

ggccagcccc ccaagctgct gatctactgg gccagcacca gagagagcgg cgtgcccgac      240

agattcagcg gcagcggcag cggcaccgac ttcaccctga ccatcagcag cctgcaggcc      300

gaggacgtgg ccgtgtacta ctgcagacag cactacagca ccccccttcac cttcggccag      360

ggcaccaagc tggagatcaa gggcggcggc ggcagcggcg gcggcggcag cggcggcggc      420

ggcagcggcg gcggcggcag cgaggtgcag ctggtgcaga gcggcgccga ggtgaagaag      480

cccggcgcca ccgtgaagat cagctgcaag gtgagcggct acagcttcac cgactacaac      540

atgcactggg tgcagcaggc ccccggcaag ggcctggagt ggatgggcct gatcgacccc      600

tacaacggcg acaccatcta cgccgagaag ttccagggca gagtgaccat caccgccgac      660

accagcaccg acaccgccta catggagctg agcagcctga gaagcgagga caccgccgtg      720

tactactgcg ccagaagccc ctacggccac tacgccatgg actactgggg ccagggcacc      780

accgtgaccg tgagcagc                                                    798
```